# NEW EUROPEAN PATENT SPECIFICATION

(11) **EP 0 449 958 B2**
(45) Date of publication and mention of the opposition decision: **13.11.2002**
(45) Mention of the grant of the patent: 22.03.1995
(21) Application number: 90901397.1
(22) Date of filing: 19.12.1989
(51) Int. Cl.: A61K 39/095, C07K 14/22, C07K 7/04, A61K 39/39, A61K 39/385, C12N 15/31, C12N 15/62

(54) **MENINGOCOCCAL CLASS 1 OUTER-MEMBRANE PROTEIN VACCINE**
MENINGOCOCCALES KLASSE I-AUSSENMEMBRANPROTEIN-VAKZIN
VACCIN MENINGOCOQUE DE LA PROTEINE DE LA MEMBRANE EXTERNE DE LA CLASSE 1

(30) Priority: 19.12.1988 NL 8803111; 06.01.1989 NL 8900036; 26.06.1989 NL 8901612
(43) Date of publication of application: 09.10.1991
(73) Proprietor: AMERICAN CYANAMID COMPANY, Portland, Maine 04101 (US); De Staat der Nederlanden, represented by the Deputy Director-General of the RIVM of Bilthoven, NL-3720 BA Bilthoven (NL)
(72) Inventor: SEID, Robert, C., Jr., San Francisco, CA 94121 (US); PARADISO, Peter, R., Pittsford, NY 14534 (US); POOLMAN, Jan, T., NL-1151 AK Broek in Waterland (NL); HOOGERHOUT, Peter, NL-2805 SZ Gouda (NL); WIERTZ, Emmanuel, J., H., J., NL-3583 HW Utrecht (NL); VAN DER LEY, Peter, NL-3583 AM Utrecht (NL); HECKELS, John, Edward 6 Arun Way West Wellow, Hampshire SO51 6GT (GB); CLARKE, Ian, Nicholas 15 Fernyhurst Avenue, Hampshire SO1 8DR (GB)
(74) Representative: Roques, Sarah Elizabeth
(86) International application number: US8905678
(87) International publication number: WO90006696

(56) References cited:
- EP-A- 0 090 660
- EP-A- 0 145 359
- EP-A- 0 301 992
- WO-A-89/10967
- US-A- 4 601 903
- US-A- 4 673 574
- Infection and Immunity, vol. 40, no. 1, April 1983 American Society for Microbiology, E.C. Beuvery et al. pp. 369-380
- Infection and Immunity, vol. 54, no. 2, Nov. 1986, American Society for Microbiology, W. Jiskoot et al. pp. 333-338
- Infection and Immunity, vol. 46, no. 2, Nov. 1984 American Society for Microbiology pp. 408-414
- Vaccine, vol. 5, no. 4, 5 Dec. 1987, Butterworth & Co. (Publishers) Ltd, (London, GB) T. Teerlink et al. pp. 307-314
- Vaccine, vol. 5, no. 2, 5 June 1987, Butterworth & Co., (Publishers) Ltd, (Guildford, Surrey, GB), G. Gregoriadis et al. pp. 145-151
- Journal of Bacteriology, vol. 141, no. 1, January 1980, Chao-Ming Tsai et al. pp. 169-176
- Chemical Abstracts, vol. 94, no. 25, 22 June 1981, American Chemical Society, (Columbus, Ohio, US), C.-M. Tsai et al. s. p. 281, abstract 205111z, & J. Bacteriol. 1981, 146(1), 69-78
- Infection and Immunity, vol. 55, no. 11, November 1987, American Society for Microbiology, A.K. Barlow et al. pp. 2734-2740
- Journal of Immunological Methods, vol. 113, 1988, Elsevier Science Publishers B.V. (Biomedical Division) E. Wedege et al. pp. 51-59
- Biological Abstracts, vol. 87, 1989 H. Abdillahi et al. "Microbiol. Definition of meningococcal class 1 OMP subtyping antigens by monoclonal antibodies, s. abstr. no. 115445
- Biological Abstracts/RRM, BR36:126147, AN 89:258923 E.J.H.J. Wiertz et al. (University of California-Los Angeles) Symposium on immunogenicity, steamboat Springs, Colorado, USA, Jan. 21-28, 1989 & J Cell Biochem suppl 0 (13 PART A). 1989, 326
- Infection and Immunity, vol. 57, no. 3, March 1989, American Society for Microbiology, J.T. Poolman et al. pp. 1005-1007

## Description

### Background of the Invention

Bacterial meningitis is an inflammatory disease of the central nervous system caused by the growth of bacteria in and adjacent to the leptomeninges. Meningitis is an acute infectious disease which affects children and young adults and is caused by the Neisseria meningitidis, amongst other agents including other bacterial and viral pathogens.

Meningococci are subdivided into serological groups depending on the presence of either capsular or cell wall antigens. Currently recognized serogroups include A, B, C, D, W-135, X, Y, Z, and 29E as segregated by seroagglutination. The polysaccharides responsible for the serogroup specificity of the group A, B, C, X, W-135 and Y have been purified.

The carrier rate for meningococci is much higher than the incidence of the disease. Some persons are temporary carriers, while others are chronic carriers, discharging meningococci either more or less continuously or in a sporadic fashion. The meningococcal carrier state is an immunizing process, and within two weeks of colonization, production of antibodies to meningococci can be identified. It appears that bactericidal antibodies are directed against both the capsular polysaccharide and other cell wall antigens.

Studies have shown that meningococcal outer membranes have three to five major proteins, with the predominant 41,000Mr or 38,000Mr proteins carrying the serotype specific determinants. There is a considerable degree of interstrain heterogeneity in the profiles of the outer membrane proteins on sodium dodecyl sulfate-polyacrylamide electrophoretic gels (SDS-PAGE). As defined by peptide mapping studies, the proteins comprise five classes, designated 1 through 5, based upon common peptide structures. Bactericidal monoclonal antibodies have been produced against the 46,000 Mr Class 1 proteins which are shared to some extent among strains of different serotypes. (Frasch, C.E. et al., (1985) pg. 633,"New Developments in Meningococcal Vaccines", in G.K. Schoolnik et al. (ed.) The Pathogenic Nisseriae, American Society for Microbiology, Washington, D.C.).

The capsular polysaccharide of groups A, C, W-135 and Y meningococci have been used to develop vaccines against the organism. Although these vaccines have been effective in the short term, they do not induce immunological memory and subjects must be revaccinated within approximately 3 years to maintain their resistance. The group B polysaccharide is poorly immunogenic and successful vaccines have not been produced. A possible explanation for the low activity may be due to tolerance to the group B polysaccharide induced by crossreactive thus the Class I OMP, fragments and oligopeptides of this invention allow for the development of vaccines for other gram negative pathogens.

### Brief Description of the Figures

Figure 1. Scheme for amplification of genes encoding meningococcal Class I outer membrane protein by PCR (Polymerase Chain Reaction).

Figure 2. 5' gene sequences encoding VR1 (first variable region) of Class I outer membrane proteins of several N. meningitidis subtypes.

Figure 3. 3' gene sequences encoding VR2 (second variable region) of Class I outer membrane proteins of several N. meningitidis subtypes.

Figure 4. Epitope scanning by reaction of monoclonal antibodies with solid phase decapeptides spanning the predicted amino acid sequences of Class I proteins from strains P1.7,16, P1.16 and P1.15. Adjacent decapeptides differ by five amino residues. Annotations show the strain from which the sequence was derived, the mAb used and its subtype specificity.

Figure 5. Reaction of the monoclonal antibodies with series of overlapping decapeptides corresponding to variable regions VR1 and VR2, with adjacent peptides differing by a single amino acid residue. Annotations show the strain from which the sequence was derived, the mAb used and its subtype specificity.

Figure 6. Construction of recombinant flagellins expressing variable region epitopes of N. meningitidis Class I OMP subtype P1.6,16.

Figure 7. Structure of recombinant flagellins expressing variable region epitopes of N. meningitidis Class I OMP subtype P1.6,16.

Figure 8. Representative chromotogram of high performance liquid chromatography of a recombinant flagellin.

Figure 9. Representative analysis by SDS-PAGE of recombinant flagellin.

Figure 10. Regresentative Western blot analyses of a conjugate comprising an epitope of N. meningitidis Class I OMP conjugated to CRM ₁₉₇.

Figure 11. Putative conformation of N. meningitidis Class I OMP subtype P1.16. antigens found in human tissues such as the brain. Furthermore, studies show that most of the bactericidal antibodies in the convalescent sera of patients who have had group B meningococcal disease are directed against outer membrane proteins.

Vaccines for protecting against group B meningococcal disease have been developed in which non-covalent complexes of outer membrane proteins (OMP) and group B polysaccharide were administered. Beuvery, et al (1983) Infect. Immun. 40:369-380. However, the B polysaccharide is known to induce a transient IgM antibody response, which does not confer immunoprotection. Furthermore, there is great antigenic diversity and variability in the meningocococci outer membrane proteins from strain to strain. Additionally, lipopolysaccharides are present in the OMP and exhibit antigenic variability as well.

There is a need for safe and effective vaccines against meningococcal disease which provide immunity from infection, particularly in infants and the elderly.

### Summary of the Invention

In a first aspect of the invention, we provide a vaccine effective against meningococcal disease, comprising an effective amount of an outer-membrane vessicle isolated from genetically engineered or mutant meningococcal strain, the vesicle comprising: at least one meningococcal Class I outer-membrane protein, fragment thereof or oligopeptide containing an epitope thereof, wherein said protein, fragment thereof or oligopeptide is capable of eliciting a bactericidal immune response in a host; and not comprising a meningococcal Class 2/3 outer-membrane protein; the meningococcal Class I outer-membrane protein originating from a mutant meningococcal strain which is negative for Class 2/3 outer-membrane protein.

In an alternative aspect of the invention, there is provided substantially purified fragment of Class I outer-membrane protein of Neisseria meningitidis, the fragment having a molecular weight of about 25 kD or less and containing continuous or discontinuous epitopes reactive with bactericidal antibodies against N. meningitidis, wherein the epitopes are located in surface loops of meningococcal Class I outer-membrane proteins in the area of amino acids 24-34 and 176-187.

There is provided, according to another, alternative, aspect of the invention isolated nucleic acid encoding full length Class I OMP protein having the sequence P1.15 or the sequence P1.7,16, as depicted in the Figure accompanying Example 4.

According to another, alternative, aspect of the invention, we provide isolated oligonucleotide encoding an epitope located in surface loops of meningococcal Class I outer-membrane proteins in the area of amino acids 24-34 and 176-187.

We provide, in accordance with a further alternative aspect of the invention a composition for eliciting a protective immune response against Neisseria meningitidis, comprising a) liposome comprising one or more meningococcal Class I outer-membrane proteins, but not comprising a meningococcal Class 2/3 outer-membrane protein or fragment thereof; and, optionally, an adjuvant in a pharmaceutically acceptable vehicle, or b) a liposome comprising at least one oligopeptide containing a continuous or discontinuous epitope of a Class I outer-membrane protein reactive with bactericidal antibodies against N. meningitidis but not comprising an epitope of a Class 2/3 outer membrane protein; and optionally, an adjuvant in a pharmaceutically acceptable vehicle; said Class I outer-membrane protein(s) having the sequence p1.15 the sequence or the sequence p1.7,16, as depicted in the figure accompanying Example 4.

According to further alternative aspects of the invention, we provide an antigenic conjugate, comprising a carrier protein or epitope thereof, to which is conjugated a fragment of intact Class I outer-membrane protein of Neisseria meningitidies, genetic fusion protein thereof or a continuous or discontinuous epitope reactive with Class I OMP specific antibodies against N. meningitidis.

There is further provided, according to another alternative aspect of the invention, a genetically engineered microorganism consisting of a Neisseria strain capable of expressing more than one Class I outer-membrane protein of Neisseria meningitidis, each protein individually comprising at least one continuous or discontinuous epitope reactive with Class I OMP specific antibodies against N. meningitidis.

We provide, in accordance with a further alternative aspect of the invention a method of expressing at least one meningococcal Class I outer-membrane protein that is capable of eliciting a bactericidal immune response in a host, comprising inserting at least one gene encoding meningococcal Class I outer-membrane protein into a recombinant microorganism selected from a) a mutant meningococcal strain which is negative for Class 2/3 outer-membrane protein or b) a group A, B, C, W-135 or Y meningococcus; wherein the microorganism is capable of expressing said gene as intact protein that is capable of eliciting a bactericidal immune response in a host.

According to another alternative aspect of the invention, we provide a genetically engineered Neisseria strain capable of expressing a non-native, intact Class I outer-membrane protein of Neisseria meningitidis, genetic fusion protein thereof or a continuous or discontinuous epitope reactive with Class I OMP specific antibodies against N. meningitidis.

This invention also pertains to fragments of the Class I outer-membrane protein (OMP) of Neisseria meningitidis and to oligopeptides derived from the Class I OMP which contain continuous or discontinuous, immunogenic and,

### Detailed Description of the Invention

This invention pertains to vaccines comprising isolated OMV's, meningococcal Class 1 OMP, fragments of the OMP (e.g., prepared by the application of cyanogen bromide) and oligopeptides bearing epitopes of the OMP; the preparation of isolated OMV's, pure Class 1 outer-membrane proteins, using mutant strains which do not express the Class 2/3 outer-membrane protein; the preparation of isolated OMV's pure Class 1 outer-membrane proteins with the aid of cloned DNA in recombinant DNA expression vectors. This invention also comprises the application of genetic engineering with the object of producing isolated OMV, Class I OMP or portions thereof, genetic fusions of Class I OMP, portions or epitopes therof; and the preparation of multivalent Class 1 outer-membrane vaccine through peptide synthesis, as the epitopes with a short peptide chain can be synthetically prepared.

It has emerged that meningococcal Class 1 outer-membrane proteins induce a strong bactericidal immune response to the strains containing the appropriate subtype epitopes, irrespective of whether these are from group A,B,C,W-135,and Y strains. The polysaccharide vaccine can be enhanced or replaced by a vaccine according to the invention as a vaccine with broad, extensive action against most serotypes. The protective bactericidal monoclonal antibodies specific for the Class 1 outer--membrane protein react strongly with fragments that have been split off and short synthetic peptides which have been prepared using the amino acid sequence of Class 1 outer-membrane proteins. Since meningococcal disease is currently caused chiefly by group B meningococci and because the Class 1 outer--membrane proteins occurring in group B meningococci also occur in group A,C,W-135,Y meningococci, vaccines of this invention which comprise one or more Class 1 OMP epitopes derived from N. meningitidis group B should be effective in preventing disease caused by group A, C, W-135 and Y. Preferably, the preparation of such a vaccine starts from at least two different immunogenic and protective epitopes which have been selected on epidemiological grounds. Vaccines according to the invention comprise, for example, at least one protein which is obtained either in OMV formulation or by purification from mutant strains producing one or more Class I OMP or at least two fragments prepared through a cyanogen bromide fragmentation or at least two synthetic peptides, chosen from about 10 major epitopes, or products obtained by gene expression via recombinant DNA technology, which contain the desired epitopes. To maximize efficacy to a broad range of meningococcal strains, the greater number of different protective epitopes in the vaccine the better. In addition, the vaccines according to the invention may advantageously contain meningococci A and C or optionally W-135 and Y polysaccharides and/or detergents. Preferably, the A and C polysaccharides are convalently coupled to a protein or polypeptide carrier. These carriers include, for example, isolated OMV, the Class I OMP protein, T-helper epitopes, bacterial toxins, toxoids, nontoxic mutants (CRM's), recombinant Salmonella flagellin and viral particles such as rotavirus VP6 protein, Hepatitis B surface antigen or parvovirus VP1 and VP2 proteins. Both Zwitterionogenic, cationogenic, anionogenic and nonionogenic detergents can be used. Examples of such detergents are Zwittergent 3-10, Zwittergent 3-14 (N-tetradecyl-N, N-dimethyl-3-ammonia-1-propane sulphonate), Tween-20, sodium deoxycholate, sodium cholate and octylglucoside. The vaccines according to the invention may also contain an adsorbent such as aluminium hydroxide, calcium phosphate; or advantageously, aluminium phosphate. The fragments, proteins, peptides can also be processed in immunostimulating complexes (ISCOMS), liposomes or microspheres for delivering and/or use as an adjuvant or in connection with other adjuvants so that greater immunogenicity is obtained.

This invention encompasses isolated OMV, substantially pure meningococcal class 1 outer membrane proteins (of any subtype) and fragments of the proteins containing epitopes thereof. The fragments can be any portions of the molecular weight of 25kD or less which contain epitopes which are bound by protective bactericidal antibodies against N. meningitidis. These include proteolytic fragments and synthetic oligopeptides which are comprised of amino acid sequences which correspond, at least in part, to epitopes of a Class I OMP.

The isolated OMV's, Class I OMP, fragments or epitope-containing oligopeptides derived therefrom can be comprised of amino acid sequences which are different, but essentially biologically equivalent to the natural sequences. These sequences can include sequences in which functionally equivalent amino acid residues are substituted for residues within the sequence resulting in a silent change. For example, one or more amino acid residues within the sequence can be substituted by another amino acid of a similar polarity which acts as a functional equivalent, resulting in a silent alteration. Substitutes for an amino acid within the sequence may be selected from other members of the class to which the amino acid belongs. For example, the nonpolar (hydrophobic) amino acids include glycine, alanine, leucine, isoleucine, valine, proline, phenylalanine, tryptophan and methionine. The polar neutral amino acids include serine, threonine, cysteine, tyrosine, asparagine, and glutamine. The charged (basic) amino acids include arginine, lysine and histidine. The negatively charged (acidic) amino acids include aspartic and glutamic acids.

Additionally, isolated OMV's, the class I OMP, fragments or the oligopeptides can be modified for conjugation to other molecules (e.g., by the attachment of coupling groups such as the amino acids cysteine and/or lysine or other linking groups and/or spacer groups) including other class 1 OMP of a different subtype, T cell epitopes, B cell epitopes, carrier peptides or proteins or adjuvanting molecules.

As described in detail below, the Class I OMP. fragments or oligopeptides can be used in many different forms (e.g., alone, in mixtures, or as conjugates and genetic fusions produced from recombinant DNA vectors) in vaccines. For these purposes, the materials can be produced by isolation from N. meningitidis, by proteolytic digestion, by chemical synthesis, or by expression as recombinant molecules. The methods of production and use of the isolated OMV's, the class I OMP and the fragments and the oligopeptides of class 1 OMP are described below.

Protein modeling and structure analysis of the Class I OMPs were performed using the principles for several E. coli outer membrane proteins. (Vogel, H. et al., J. Mol. Bio., 190:191 (1986); Ference, T. et al., J. Mol. Bio., 201:493 (1988) and Tommassen, J. in "Membrane Biogenesis", NATO ASI Series H16, pp351, Springer-Verlag, NY (1988)). The derived amino acid sequence of the Class I OMPs were used for the modeling studies and comparison. The amino acid sequence homology was compared to other gram negative bacterial porin proteins and similarity was established for the protein structure. Exposed surface loops and transmembrane structure were very similar for these porin proteins. With the information revealed concerning variable and constant region protective epitopes of N. meningitidis and their structure, one can predict based upon the amino acid sequence where protective epitopes may reside for other pathogenic gram negative bacteria to be evaluated and included in vaccines for the same.

### Production of isolated OMV's

OMV's can be produced either from the culture supernatant or from the bacterial cells after fragmentation as described by Beuvery et al. (1983) Infect. Immun. 40:369-380. OMV's carrying proteins from more than one meningococcus can be isolated from strains manipulated to express heterologous proteins.

### Production and Purification of Class I OMP and CNBr fragments thereof

Class 1 and Class 3 outer membrane proteins can be isolated as described by Beuvery, E.C. et al., Antonie wan Leeuvenhoek J. Microbiol. 52:232 (1986). The production of substantially pure Class I OMP free of Class 2 or 3 OMP's is achieved by this method using mutant meningococcal strains which do not express Class 2/3 OMP. A preferred strain for production of Class I OMP is the HIII5 strain, deposited as CBS 636.89.

Fragments can be produced by cyanogen bromide cleavage as described by Teerlink T. et al., J. Exp.Med. 166:63 (1987) for a gonococcal protein. The N-terminal fragment is referred to as CB-1 and the C-terminal fragment is referred to as CB-2. These CNBr fragments can be purified via reverse phase HPLC on a Vydax™ C4 or an Aquapor™ R-300 column using a water/acetonitrile gradient. Alternatively, the fragment can be purified by multiple cold trichloroacetic acid precipitations. These procedures remove greater than 95% of interferring contaminants (e.g., buffer salts, detergents and fragment contaminants).

### Preparation of fragments and oligopeptides containing epitopes of class I OMP

### A. Preparation by proteolytic digestion

Oligopeptides containing epitopes reactive with bactericidal antibodies against N. meningitidis can be produced by digestion of the class I OMP, CB-1 or CB-2 fragments with proteinases such as endoLys-C, endoArg-C, endoGlu-C and staphylococcins V8-protease. The digested fragments can be purified by, for example, high performance liquid chromatograghic (HPLC) techniques.

### B. Preparation by Chemical synthesis

Oligopeptides of this invention can be synthesized by standard solid peptide synthesis (Barany, G. and Merrifield, R.B., The Peptides 2:1-284, Gross, E. and Meienhofer, J., Eds., Academic Press, New York) using tert-butyloxycarbonyl amino acids and phenylacetamidomethyl resins (Mitchell, A. R. et al.., J. Org. Chem. 43:2845-2852 (1978)) or 9-fluorenylmethyloxycarbonyl amino acids on a polyamide support (Dryland, A. and Sheppard, R.C., J. Chem. So. Perkin Trans. I, 125-137 (1986)). Alternatively, synthetic peptides can be prepared by pepscan synthesis (Geysen, H.M. et al., J. Immunol Methods 03:259 (1987); Proc. Natl. Acad. Sci. USA 81:3998 (1984)), Cambridge Research Biochemicals, Cambridge, U.K. or by standard liquid phase peptide synthesis. The deletion or substitution of amino acids (and including extensions and additions to amino acids) in other ways which do not substantially detract from the immunological properties of the oligopeptide.

### C. Preparation by recombinant DNA techniques

The Class I OMP, fragments and oligopeptides which exhibit epitopes of the Class I OMP can be produced by recombinant DNA techniques. In general, these entail obtaining DNA sequences which encode the desired OMP, [Barlow et al., (1989) Mol. Micro., 3:131) fragment or oligopeptide sequences and introducing into an appropriate vector/host expression system one or more similar or different DNA sequences of Class I OMP's where it is expressed. The DNA can consist of the gene encoding the Class I OMP or any segment of the gene which encodes a functional epitope of the OMP. The DNA can be fused to DNA encoding other antigens of N. meningitidis (such as other outer membrane proteins either of the same or different class) or antigens of other bacteria, viruses, parasites or fungi to create genetically fused (sharing a common peptide backbone), multivalent antigens. For example, Class I OMP fragments can be fused to another class 1 outer membrane protein of a different subtype (or fragments or epitopes thereof) of N. meningitidis to yield fusion proteins comprising multiple class 1 outer membrane protein subtype determinants.

Genetic engineering techniques can also be used to characterize, modify and/or adapt the encoded peptides or proteins. For example, site directed mutagenesis to modify an OMP fragment in regions outside the protective domains, for example, to increase the solubility of the subfragment to allow easier purification. DNA can also be manipulated to effect superproduction of OMP fragments or combinations thereof in various organisms.

DNA encoding a Class I OMP, fragments or oligopeptides can be synthesized or isolated and sequenced as described by Barlow, A.K. et al. Infect. Immune 55:2734-40 (1987) and Barlow, A.K. et al., Mol. Micro. 3:131 (1989). Class I OMP genes can be amplified from bacterial DNA by the methods of Mullis and Faloona, (1987) Method. Enzym. 155:335-350, using the primer sequences disclosed herein. Related DNA sequences for class 1 OMP of different subtypes can be obtained by the procedures described and the amino acid sequences deduced.

A variety of host-vector systems can be used to express the oligopeptides of this invention. Primarily the vector system must be compatible with the host cell used. Host-vector systems include but are not limited to the following: bacteria transformed with bacteriophage DNA, plasmid DNA or cosmid DNA; microorganisms such as yeast containing yeast vectors; mammalian cell systems infected with virus (e.g., vaccinia virus, adenovirus, etc.); insect cell systems infected with virus (e.g., baculovirus) . The expression elements of these vectors vary in their strength and specificities. Depending upon the host-vector system utilized, any one of a number of suitable transcription and translation elements can be used.

In order to obtain efficient expression of the cloned DNA, a promoter must be present in the expression vector. RNA polymerase normally binds to the promoter and initiates transcription of a gene or a group of linked genes and regulatory elements (called an operon). Promoters vary in their "strength", i.e., their ability to promote transcription. It is desirable to use strong promoters in order to obtain a high level of transcription and, hence, a high level of DNA expression. Depending upon the host cell system any one of a number of suitable promoters can be used. For instance, for E. coli, its bacteriophages or plasmids, promoters such as the lac promoter, trp promoter, recA promoter, ribosomal RNA promoter, and P_{R} or P_{L} promoters of coliphage lambda and others including but not limited to lacUV5, ompF, bla, lpp and the like, may be used to direct high levels of transcription of adjacent DNA segments. Additionally, a hybrid trp-lacUV5 (tac) promoter or other E. coli promoters produced by recombinant DNA or other synthetic DNA techniques may be used to provide for transcription of the inserted DNA.

Bacterial host cells and expression vectors may be chosen which inhibit the action of the promoter unless specifically induced. In certain operons the addition of specific inducers is necessary for efficient transcription of the inserted DNA; for example, the lac operon is induced by the addition of lactose or IPTG (isopropylthio-beta-D-galactoside). A variety of other operons, such as trp, etc., are under different controls. The trp operon is induced when tryptophan is absent in the growth media; and the P_{L} promoter of lambda can be induced by an increase in temperature in host cells containing a temperature sensitive lambda repressor, e.g., cl857. In this way, greater than 95% of the promoter--directed transcription may be inhibited in uninduced cells. Thus, expression of the recombinant peptide or protein can be controlled. This is important if the expression product of the DNA is lethal or detrimental to the host cells. In such cases, transformants may be cultured under conditions such that the promoter is not induced; then, when the cells reach a suitable density in the growth medium, the promoter can be induced for production of the protein.

One such promoter/operator system is the "tac" or trp-lac promoter/operator system (Russell and Bennett, 1982, Gene 20:2312-243; DeBoer, European Patent Application, 67, 540 filed May 18, 1982). This hybrid promoter is constructed by combining the -35 b.p. (-35 region) of the trp promoter and the -10 b.p. (-10 region or Pribnow box) of the lac promoter (the sequences of DNA which are the RNA polymerase binding site). In addition to maintaining the strong promoter characteristics of the tryptophan promoter, tac is also controlled by the lac repressor.

When cloning in a eukaryotic host cell, enhancer sequences (e.g., the 72 bp tandem repeat of SV40 DNA or the retroviral long terminal repeats or LTRs, etc.) may be inserted to increase transcriptional efficiency. Enhancer sequences are a set of eucaryotic DNA elements that appear to increase transcriptional efficiency in a manner relatively independent of their position and orientation with respect to a nearby gene. Unlike the classic promoter elements (e.g., the polymerase binding site and the Goldberg-Hogness "TATA" box) which must be located immediately 5' to the gene, enhancer sequences have a remarkable ability to function upstream from, within, or downstream from eucaryotic genes; therefore, the position of the enhancer sequence with respect to the inserted DNA is less critical.

Specific initiation signals are also required for efficient gene transcription and translation in procaryotic cells. These transcription and translation initiation signals may vary in "strength" as measured by the quantitiy of gene specific messenger RNA and protein synthesized, respectively. The DNA expression vector, which contains a promoter, may also contain any combination of various "strong" transcription and/or translation initiation signals. For instance, efficient translation in E. coli requires a Shine-Dalgarno (SD) sequence about 7-9 bases 5' to the initiation codon (ATG) to provide a ribosome binding site. Thus, any SD-ATG combination that can be utilized by host cell ribosomes may be employed. Such combinations include but are not limited to the SD-ATG combination from the cro gene or the N gene of coliphage lambda, or from the E. coli tryptophan E, D, C, B or A genes.
Additionally, any SD-ATG combination produced by recombinant DNA or other techniques involving incorporation of synthetic nucleotides may be used.

Any of the methods described for the insertion of DNA into an expression vector can be used to ligate a promoter and other genetic control elements into specific sites within the vector. N. meningitidis sequences for expression can be ligated into an expression vector at a specific site in relation to the vector promoter and control elements so that when the recombinant DNA molecule is introduced into a host cell the foreign genetic sequence can be expressed (i.e., transcribed and translated) by the host cell.

The recombinant DNA vector can be introduced into appropriate host cells (bacteria, virus, yeast, mammalian cells or the like) by transformation, transduction or transfection (depending upon the vector/host cell system). Host cells containing the vector are selected based upon the expression of one or more appropriate gene markers normally present in the vector, such as ampicillin resistance or tetracycline resistance in pBR322, or thymidine kinase activity in eucaryotic host systems. Expression vectors may be derived from cloning vectors, which usually contain a marker function. Such cloning vectors may include, but are not limited to the following: SV40 and adenovirus, vaccinia virus vectors, insect viruses such as baculoviruses, yeast vector, bacteriophage vectors such as lambda gt-WES-lambda B, Charon 28, Charon 4A, lambda gt-1-lambda BC, lambda gt-1-lambda B, M13mp7, M13mp8, M13mp9, or plasmid DNA vectors such as pBR322, pAC105, pVA51, pACYC177, pKH47, pACYC184, pUB110, pMB9, pBR325, Col E1, pSC101, pBR313, pML21, RSF2124, pCR1, RP4, pBR328 and the like.

Expression vectors containing the DNA inserts can be identified by three general approaches: (1) DNA-DNA hybridization using probes comprising sequences that are homologous to the inserted gene; (2) presence or absence of "marker" gene functions ( e.g., resistance to antibiotics, transformation phenotype, thymidine kinase activity, etc.); and (3) expression of inserted sequences based on the physical immunological or functional properties of the gene product.

Once a putative recombinant clone which expresses a desired Class I OMP amino acid sequence is identified, the gene product can be analyzed as follows. Immunological analysis is especially important because the ultimate goal is to use the gene products in vaccine formulations and/or as antigens in diagnostic immunoassays. The expressed peptide or protein should be immunoreactive with bactericidal antibodies against N. meningitidis. This reactivity may be demonstrated by standard immunological techniques, such as radioimmunoprecipitation, radioimmune competition, ELISA or immunoblots.

Once the gene product is identified as a Class I OMP fragment or an oligopeptide contain a functional epitope thereof, it can be isolated and purified by standard methods including chromatography (e.g., ion exchange, affinity, and sizing column chromatography), centrifugation, differential solubility, or by any other standard techniques for the purification or proteins. Several techniques exist for purification of heterologous protein from prokaryotic cells. See e.g., Olson, U.S. Patent No. 4,518,526, Wetzel, U.S. Patent No. 4,599,197 and Hung et al., U.S. Patent No. 4,734,362. The purified preparation however produced should be substantially free of host toxins which might be harmful to humans. In particular, when expressed in gram negative bacterial host cells such as E. coli or Salmonella, the purified peptide or protein should be substantially free of endotoxin contamination.

Class I OMP, fragments and oligopeptides of this invention can be formulated as univalent and multivalent vaccines. These materials can be used as produced or isolated by the methods described above. They can be mixed, conjugated or fused with other antigens, including B or T cell epitopes of other antigens. In addition, they can be conjugated to a carrier protein as described below for oligopeptides.

When a haptenic oligopeptide is used (i.e., a peptide which reacts with cognate antibodies, but cannot itself elicit an immune response), it can be conjugated to an immunogenic carrier molecule. Conjugation to an immunogenic carrier can render the oligopeptide immunogenic. The conjugation can be performed by standard procedures. Preferred carrier proteins for the haptenic oligopeptides are toxins, toxoids or any mutant crossreactive material (CRM) of the toxin from tetanus, diphtheria, pertussis, Pseudomonas, E. coli, Staphylcoccus, and Streptococcus. A particularly preferred carrier is CRM₁₉₇ of diphtheria toxin, derived from P. diphtheriae strain C7(β 197) which produces CRM₁₉₇ protein. This strain has ATCC accession no. 53281. Alternatively, a fragment or epitope of the carrier protein or other immunogenic protein can be used. For example, the hapten can be coupled to a T cell epitope of a bacterial toxin, toxoid or CRM. See U.S.Patent Application Serial No. 150,688, filed February 1, 1988, entitled "Synthetic Peptides Representing a T-Cell Epitope as a Carrier Molecule For Conjugate Vaccines", the teachings of which are incorporated herein. Other carriers include viral particles composed of Rotavirus VP6, Hepatitis B surface antigen or parvovirus VP1 and VP2.

The peptides or proteins of this invention can be administered as multivalent subunit vaccines in combination with antigens of N. meningitidis or antigens of other organisms. Some of the other organisms include the pathogenic bacteria H. influenzae, N. meningitidis, B. catarrhalis, N. gonorrhoeae, E. coli, S. pneumoniae, etc. For example, they may be administered in conjunction with oligo- or polysaccharide capsular components of N. meningitidis. The capsular components can be derived from any of the serological groups, including A, B, C, D, X, Y, Z, 29E and W135.

Class 1 outer membrane proteins of different subtypes can be used. These may be used in combination to evoke bactericidal antibodies against N. meningitidis. For example, a fragment derived from class 1 outer membrane protein of the P1.7.16 subtype can be used together with outer membrane proteins or fragments of outer membrane proteins of other subtypes, such as P1.1, P1.1,16; P1.2; P1.6; P1.9; P1.15; P1.16; or P1.4 (Abdillahi, H. et al. 1988 Micro. Pathog. 4:27) or with meningococcal polysaccharides in mixtures or as chemical conjugates. For combined administration with epitopes of other outer membrane proteins, they can be administered separately, as a mixture or as a conjugate or genetic fusion peptide or protein. The conjugates can be formed by standard techniques for coupling proteinaceous materials or techniques for coupling saccharide polymers to proteins. Fusions can be expressed from fused gene constructs prepared by recombinant DNA techniques as described.

As mentioned, Class I OMP, fragment or any oligopeptides derived therefrom can be used in conjunction with antigens (e.g., polymer capsules or saccharide units, envelope or surface proteins) of other pathogenic organisms (e.g. bacteria (encapsulated or nonencapsulated), viruses, fungi and parasites). Additional examples of other organisms include respiratory syncytial virus, rotavirus, malaria parasites, and Cryptococcus neoformans.

In formulating the vaccine compositions with the peptide or protein, alone or in the various combinations described, the immunogen is adjusted to an appropriate concentration and formulated with any suitable vaccine adjuvant. Suitable adjuvants include, but are not limited to: surface active substances, e.g., hexadecylamine, octadecylamine, octadecyl amino acid esters, lysolecithin, dimethyldioctadecylammonium bromide), methoxyhexadecylgylcerol, and pluronic polyols; polyamines, e.g., pyran, dextransulfate, poly IC, carbopol; peptides, e.g., muramyl dipeptide and derivatives, dimethylglycine, tuftsin; oil emulsions; and mineral gels, e.g., aluminum hydroxide, aluminum phosphate, etc., lymphokines and immune stimulating complexes (ISCOMS). The immunogen may also be incorporated into liposomes, microspheres, or conjugated to polysaccharides and/or other polymers for use in a vaccine formulation.

The vaccines can be administered to a human or animal in a variety of ways. These include intradermal, intramuscular, intraperitoneal, intravenous, subcutaneous, oral and intranasal routes of administration.

### Live vaccines

The peptide and proteins of this invention can be administered as live vaccines. To this end, recombinant microorganisms are prepared that express the peptides or proteins. The vaccine recipient is inoculated with the recombinant microorganism which multiplies in the recipient, expresses the Class I OMP, fragment or oligopeptide thereof and evokes a immune response to N. meningitidis. Live vaccine vectors include: adenovirus, cytomegalovirus and preferably, pox viruses such as vaccinia (Paoletti and Panicali, U.S. Patent No. 4,603,112) and attenuated Salmonella strains (Stocker, U.S. Patent No. 4,550,081 and Curtiss et al., Vaccine 6:155-160 (1988)). In addition, class I OMP epitopes can be incorporated into the flagella of attenuated bacterial strains.

Live vaccines are particularly advantageous because they lead to a prolonged stimulus which can confer substantially long-lasting immunity. When the immune response is protective against subsequent N. meningitidis infection, the live vaccine itself may be used in a preventative vaccine against N. meningitidis.

Multivalent live vaccines can be prepared from a single or a few recombinant microorganisms that express different epitopes of N. meningitidis (e.g., other outer membrane proteins from other subtypes or epitopes thereof). In addition, epitopes of other pathogenic microorganisms can be incorporated into the vaccine. For example, a vaccinia virus can be engineered to contain coding sequences for other epitopes in addition to those of N. meningitidis. Such a recombinant virus itself can be used as the immunogen in a mulivalent vaccine. Alternatively, a mixture of vaccinia or other viruses, each expressing a different gene encoding for different epitopes of outer membrane proteins of N. meningitidis and/or epitopes of other disease causing organisms can be formulated as a multivalent vaccine.

An inactivated virus vaccine may be prepared. Inactivated vaccines are "killed", i.e., infectivity has been destroyed, usually by chemical treatment (e.g., formaldehyde treatment). Ideally, the infectivity of the virus is destroyed without affecting the proteins which carry the immunogenicity of the virus. In order to prepare inactivated vaccines, large quanitites of the recombinant virus expressing the desired epitopes are grown in culture to provide the necessary quantity of relevant antigens. A mixture of inactivated viruses express different epitopes may be used for the formulation of "multivalent" vaccines. In certain instances, these "multivalent" inactivated vaccines may be preferable to live vaccine formulation because of potential difficulties arising from mutual interference of live viruses administered together. In either case, the inactivated virus or mixture of viruses may be formulated in a suitable adjuvant in order to enhance the immunological response to the antigens. Suitable adjuvants include: surface active substances, e.g., hexadecylamine, octadecyl amino acid esters, octadecylamine, lysolecithin, dimethyl-dioctadecylammonium bromide, N, N-dicoctadecyl-N', N'bis (2-hydroxyethyl-propane diamine), methoxyhexadecylglycerol, and pluronic polyols; polyamines, e.g., pyran, dextransulfate, poly IC, carbopol; peptides,e.g., muramyl dipeptide and derivatives thereof, dimethylglycine, tuftsin; oil emulsions; and mineral gels, e.g., aluminum hydroxide, aluminum phopshate, and lymphokines.

### EXEMPLIFICATION

### EXAMPLE 1: Monoclonal Antibodies Against Class I OMP's and Their Biological Activity

Type specific monoclonal antibodies were prepared against various meningococci Class 1 outer-membrane proteins. These monoclonal antibodies recognize the following subtypes: P1,1; P1,2; P1,6; P1,7; P1,9; P1.10; P1,15; P1,16; and P1,17 (now called P1,14). The monoclonal antibodies are available as "Monoclonal Kit Serotyping Meningococci" from the RIVM, Bilthoven, The Netherlands. All these monoclonal antibodies react with the SDS (sodium dodecyl sulfate) denatured protein when tested by Western blotting. It also emerged that a number of these monoclonal antibodies reacted with a 25Kd CNBr fragment of the 42Kd Class 1 outer-membrane protein (see below). This result implied that the Class 1 outer-membrane protein epitopes are mainly of linear type and can therefore be copied with synthetic peptides. The epidemiological results of tests carried out by the Applicants show that the described monoclonal antibodies can subtype most of the group A,B,C meningococci which suggests a limited heterogenity. Each Class 1 outer membrane protein also appears to contain two individual type specific epitopes (Abdillahi and Poolman, Microb. Pathogen, 1988, 4: pages 27-32; idem FEMS Microbiol. Immunol. 47: pages 139-144).

The purified Class 1 outer-membrane protein (see below), subtype P1,7.16, originating from the culture of the Class 2/3 free mutant (HIII5) appeared to induce a bactericidal antibody response of 1:64 serum dilution in a dose of 2.5 µg in mice. The monoclonal antibodies against meningococci Class 1 outer-membrane proteins, Class 2/3 outer-membrane proteins and lipopolysaccharides were compared as to bacteridical effect. The monoclonal antibodies against the Class 1 outer-membrane proteins appeared to possess the strongest bactericidal activity (see Table 1). The bacterial response was determined as per Poolman, J.T. (1985), in Schoolnick, G.J. et al. Eds. 'The Pathogenic Neisseriae' ASM Publications, Washington, D.C., page 562.

**TABLE 1**

| Bactericidal activity of a collection of monoclonal antibodies, directed against the Class 1 (cl 1), Class 2/3 (cl 2/3) and lipopolysaccharide (LPS) of meningococci. (ND - not determined). | | | |
|---|---|---|---|
| Test strain | Bactericidal activity of antibody pool (titre) | | |
| strain (Gp:serotype: | Cl 2/3 pool | Cl 1 pool | LPS pool |
| subtype:LPS type) | | | |
| 3006 (B:26:P1.2:L2) | 1000 | 8000 | ND |
| M981 (B:4P1.-:L5) | 10 | ND | 2000 |
| M990 (B:6:P1.6:L/) | 10 | 2000 | ND |
| M978 (B:8:P1.1:L1.8) | ND | 8000 | 1000 |
| M982 (B:9:P!.9:L3.7) | 500 | 2000 | 1000 |
| H355 (B:15:P1.15:L1.8) | 1000 | 8000 | 1000 |
| H44/76 (B:15:P1.7.16:L3.7) | 1000 | 8000 | 4000 |

The bactericidal activity of these monoclonal antibodies appears to correlate well with the in vivo protective activity as measured in the rat meningitis model of Saukkonen et al., 1987, Microbial Pathogen 3:261.

### EXAMPLE 1A: Construction of meningococcal strains carrying multiple Class 1 genes

Replacement of chromosomal genes by clones, slightly different versions has been described for Neisseria gonorrhoeae. (Stein, D.C., Clin. Microbiol Rev. 2 (Suppl.), S146-S149 (1989).) We have found that this method can be applied to the Class 1 gene in Neisseria meningitidis. This was done in the following way:
(i) The Class 1 gene of strain 2996 (subtype P1.2) was cloned into the vector pTZ19R. (Mead, D.A. et al., Protein Engineering 1, 67 (1986).) The complete gene is located on a 2.2 kb Xbal fragment that was ligated to Xbal digested vector DNA.
(ii) The resulting plasmid was used for transformation of strain H44/76 (subtype P1.7,16). Cells of the acceptor strain were incubated with plasmid DNA in the presence of Mg²⁺ and normal meningococcal medium; they were subsequently diluted and plated, and the resulting colonies were tested for their ability to bind P1.2-specific monoclonal antibody. Such transformants were found with a frequency of approximately 10⁻³. Further characterization showed that replacement of the H44/76 Class 1 gene had indeed occurred. An essential feature of the method is the presence of the donor gene on a circular plasmid DNA molecule that is not able to replicate in N. meningitidis, since the use of linearized DNA yielded no transformants at all.

Construction of a strain with two Class 1 genes was done by a modification of the method described above. For this purpose, the P1.2 Class 1 gene was inserted into a clones Class 5 gene. The Class 5 gene family has two features which make it particularly suitable for this construction. (Meyer, T.F. and Van Putten, J.P.M., Clin. Microbiol. Rev., 2 (Suppl.) S139-S145 (1989): (i) there are four or five Class 5 genes present in the meningococcal genome, and (ii) expression of these genes is not necessary for growth under laboratory conditions. A Class 5 gene was cloned from strain H44/76 and the P1.2 gene was inserted into an Sphl site located in or very close to the Class 5 gene. The resulting hybrid plasmid, pMC22, was used for transformation of strain HIII5, a Class 3-deficient mutant of H44.76. Colonies reacting with the P1.2-specific monoclonal antibody were isolated and characterized. Out of 10 such transformants, nine were found to have lost the P1.16 epitope of the acceptor strain. This indicates that in all these cases recombination has only occurred between the Class 1 genes, resulting in subtype replacement. However, one transformant was found which made both Class 1 subtypes, i.e., P1.7,16 and P1.2, suggesting that recombination between the Class 5 gene sequences on plasmid and chromosome must have occurred. This was confirmed by Western blotting, which revealed the presence of both types of Class 1 protein and by Southern blotting, which demonstrated the acquisition of a second Class 1 gene. By continuing this construction with other Class 1 subtypes, it is possible to make a strain with four or five different Class 1 genes. The same Class 5 gene can be used in each subsequent transformation step, the different Class 5 genes can be clones and used separately. These recombinant strains can be used to prepare mixtures of different purified Class I OMPs.

### EXAMPLE 1B:Purification of isolated OMV's from bacteriological culture

The purification is carried out according to Beuvery et al. (1983) Infect. immun. 40:369-380.

This culture can be done with the desired wild type strains, mutant meningococci strains without Class 2/3 outer-membrane proteins and/or homologeous and heterologeous recombinant microorganisms which express one or more of the desired meningococci Class 1 outer-membrane protein and/or epitopes by overproducing vectors either through or not through existing open reading frames and/or manipulated reading frames so that fusion proteins or proteins with exchanged epitopes can be prepared.

Readily available of wild strains are: H44/76 (B:15:P1,7.16) (Holten E., Norway, deposited as CBS 635-89); 187 (B:4:P1,7) (Etienne J., France); M1080 (B:1:P1,1.7) (Frasch C., USA); Swiss4 (B:4:P1,15) (Hirschel B., Switzerland); B2106l (B:4:P1,2) (Berger U., West-Germany); 395 (B:NT:P1,9) (Jonsdottir K., Iceland): M990 (B:6:P1,6) (Frasch C., USA); 2996 (B:2b:P1,2) RIVM, The Netherlands; M982 (B:9:P1,9) (Frasch C., USA); S3446 (B:14:P1,6) (Frasch C., USA); H355 (B:15:P1,15) (Holten E., Norway); 6557 (B:17:P1,17) (Zollinger W., USA) sand B40 (A:4:P1,10) (Achtman M., West-Germany). An example of a Class 3 negative mutant is HIII5 (B:-:P1.16) deposit # CBS 636.89.

These strains were inoculated from precultures at -70°C into shake flasks and transferred from these into 40, 150 or 350 litre fermenter cultures. The semisynthetic medium had the following composition: L-glutamic acid 1.3 g/l, L-cysteine.HCl 0.02 g/l, Na₂HPO₄.2H₂O 10 g/l, KCl 0.09 g/l, NaCI 6 g/l, NH₄Cl 1.25 g/l, MgSO₄.7H₂O 0.6 g/l, glucose 5 g/l, Fe(NO₃)₃ 100 µM, yeast dialysate.

During culturing in the fermenter, the pH and PO₂ were monitored and automatically regulated to a pH of 7.0-7.2 and an air saturation of 10%. The cells were grown to early stationary phase harvested by means of centrifuging and washing with sterile 0.1 M NaCI and stored at -20 ° C or freeze-dried.

### EXAMPLE 2: Purification of Class 1 outer-membrane proteins from bacteriological culture

This culture can be done with the desired wild type strains, mutant meningococci strains without Class 2/3 outer-membrane proteins and/or homologeous and heterologeous recombinant microorganisms which express one or more of the desired meningococci Class 1 outer-membrane protein and/or epitopes by overproducing vectors either through or not through existing open reading frames and/or manipulated reading frames so that fusion proteins or proteins with exchanged epitopes can be prepared.

Readily available of wild strains are: H44/76 (B:15:P1,7.16) (Holten E., Norway, deposited as CBS 635-89); 187 (B:4:P1,7) (Etienne J., France); M1080 (B:1:P1,1.7) (Frasch C., USA); Swiss4 (B:4:P1,15) (Hirschel B., Switzerland); B2106l (B:4:P1,2) (Berger U., West-Germany); 395 (B:NT:P1,9) (Jonsdottir K., Iceland): M990 (B:6:P1,6) (Frasch C., USA); 2996 (B:2b:P1,2) RIVM, The Netherlands; M982 (B:9:P1,9) (Frasch C., USA); S3446 (B:14:P1,6) (Frasch C., USA); H355 (B:15:P1,15) (Holten E., Norway); 6557 (B:17:P1,17) (Zollinger W., USA) and B40 (A:4:P1,10) (Achtman M., West-Germany). An example of a Class 3 negative mutant is HIII5 (B:-:P1.16) deposit # CBS 636.89.

These strains were inoculated from precultures at -70°C into shake flasks and transferred from these into 40, 150 or 350 litre fermenter cultures. The semisynthetic medium had the following composition: L-glutamic acid 1.3 g/l, L-cysteine.HCl 0.02 g/l, Na₂HPO₄.2H₂O 10 g/l, KCl 0.09 g/l, NaCI 6 g/l, NH₄Cl 1.25 g/l, MgSO₄.7H₂O 0.6 g/l, glucose 5 g/l, Fe(NO₃)₃ 100 µM, yeast dialysate.

During culturing in the fermenter, the pH and PO₂ were monitored and automatically regulated to a pH of 7.0-7.2 and an air saturation of 10%. The cells were grown to early stationary phase harvested by means of centrifuging and washing with sterile 0.1 M NaCl₂ and stored at -20°C or freeze-dried.

The bacterial mass was for example extracted with the aid of 0.5 M CaCl₂, 1% (w/v) Zwittergent 3-14 (Zw 3-14) and 0.14 M NaCI, pH 4.0, using 100 ml per gram of freeze-dried bacterial mass. The suspension was stirred for 1 hour at room temperature and then centrifuged (1 hour, 3000 x g), after which the supernatant was collected in a sterile manner. 20% ethanol (v/v) was added to the supernatant and after stirring for 30 min. the product was centrifuged (30 min., 10,000 x g), after which the supernatant was collected aseptically. The supernatant was then concentrated by means of diafiltration in an Amicon Hollow Fiber System (HID x 50, cut off 50,000) and CaCl₂ and ethanol were removed. The concentrate was diluted with 0.1 M sodium acetate, 25 mm EDTA, 0.05% Zw 3-14 having a pH of 6.0 to the original volume and then concentrated again by means of diafiltration. This procedure was repeated five times. The pH of the final concentrate was adjusted to a value of 4.0. 20% (v/v) ethanol was added to the concentrate and, after stirring for 30 min., the product was centrifuged (30 min., 10,000 x g). The whole proteins are purified with the aid of column chromatography in the presence of detergent, for example Zw 3-14. Often gel filtration over Sephacryl S-300 as well as the ion exchange over DEAE Sepharose is applied (Beuvery et al. (1986) supra). The used extraction method, detergents, column chromatography are not the only applicable method yet only serve as examples and must not be regarded as restrictive.

### EXAMPLE 3: Preparaton and Characterization of Class I OMP Peptide Fragments

Cyanogen bromide was used to prepare fragments of meningococci Class 1 outer-membrane proteins. The purified Class 1 or mixtures of Class 1 or 3 outer-membrane proteins were taken up in 70% (v/v) formic acid and treated with a 10-fold excess of CNBr for 16 hours at room temperature. The CNBr and the formic acid were removed by means of evaporation and replaced by 0.2 M Tris.HCl, 6 M urea solution, pH 7.2. The supernatant was prepurified by means of gel filtration over Sepharyl S-200 and subsequently purified with the aid of TSK-2000 gel filtration via HPLC. Beuvery et al., (1986) supra.

### Enzymatic digestion of CB2 fragments

To further delineate the epitopes, the meningococcal CB2 fragment was subjected to digestion with EndoArg-C, EndoGlu-C or V-8 and the resulting fragments isolated by HPLC. Briefly, 20 nMoles of CB2 fragment in 1 ml of 25 mM phosphate/0.1 mM tris buffer (pH 8.0) containing 3M urea was digested at 37°C with 0.2 nMoles of EndoArg-C (1mg/ml in distilled water) or 0.22 nMoles of EndoGlu-C or V-8 (1 mg/ml in distilled water) for 14-18 hours. The resulting digested fragments were separated by reverse phase HPLC using a Vydac-C4 column and a trifluoroacetic acid-acetonitrile gradient. The main peak eluted from the EndoArg-C digestion had an apparent molecular weight of 7-9 Kdal while the main peak observed following EndoGlu-C or V-8 had an apparent molecular weight of 4-6 Kdals. The isolated peaks were subsequently shown by Western blot to react to a pool of monoclonal antibodies (Adam I, 62-D12-8, MN5-C11G and MN14-C116).

The P1.16 epitope appears to be present on the C-terminal CNBr fragment of the Class 1 outer-membrane protein of strain H44/76 (B:15: P1,7.16). Further characterisation of the P1,16 epitope was carried out through amino acid sequence determination of the 17Kd (N-terminal) and 25Kd (C-terminal) CNBr fragments. The C-terminal 25Kd is further fragmented with V8 protease, endoLysC, endoGlu-C and endoArg-C. Fragments which were positive with the P1,16 monoclonal antibody were sequenced as far as possible. The sequences which were obtained are as follows:
N-terminus of whole protein: 
DVSLYGEIKACVEDRNYQLQLTEAQUAAGN...

N-terminus of 25Kd C-terminal CNBr
fragment : (M) PVSVRYDSPEFSGFSGSVQFVPIONS-
KSAYTPAYYTKDTNNN...
 Fragments which react with P1,16 monoclonal antibodies were isolated using V8 protease and endoArg-C fragmentation with a molecular weight of 7-9Kd and 4-6Kd respectively. The N-terminal sequences hereof are as follows:

V8 7-9Kd fragment: FSGFSGSVQFVPIQNSKSAYTPAYYTKDTN...

Arg-C 4-6Kd fragment: PVSVRYDSPEFSGFSGSVQFVPIQNSKSAYTPAYYTK...

### EXAMPLE 4: DNA Sequences of Class I OMP Genes

Amino acid sequences of Class I OMP were deduced from the nucleotide sequence of the structural genes of four meningococci Class 1 OMP's with various subtypes. Comparison with four amino acid sequences enabled a prediction of the composition and the location of these epitopes. Further, the P1,7 and P1,16 epitopes were confirmed with the aid of peptide synthesis and the demonstration of binding of the respective monoclonal antibodies.

Class I OMP genes were cloned into lambda gt11 (as described for P1, 16 in Barlow et al., (1987) Infect. Immun. 55: 2743-2740)and subcloned in M13 sequencing vectors and the DNA sequence was determined by standard chain termination dideoxynucleotide techniques.

The complete derived amino acid sequence for P1,16; P1,15, P1,7.16; and P1,2 proteins are as follows:

10          20          30          40          50
P1.16:    DVSLYGEIKAGVEGRNIQAQLTEQPQVTNGVQGNQV--KVTKAKSRIRTKIS
          **************** * ****              *************

P1.15:    DVSLYGEIKAGVEGRNFQLQLTEPP-SKSQP---QV--KVTKAKSRIRTKIS
          **************** * ****              *************

P1.7.16:  DVSLYGEIKAGVEGRNYQLQLTEAQAANGGASGQVKVTKVTKAKSRIRTKIS
          **************** * ****              *************

P1.2:     DVSLYGEIKAGVEGRNIQLQLTEPLQNIQQPQ-------VTKAKSRIRTKIS
          **************** * ****              *************

60        70        80        90        100       110
DFGSFIGFKGSEDLGEGLKAVWQLEQDVSVAGGGASQWGNRESFIGLAGEFGTLRAGRVA
************** ********************  ******* ***************

DFGSFIGFKGSEDLGEGLKAVWQLEQDVSVAGGGATQWGNRESFVGLAGEFGTLRAGRVA
************** ********************  ******* ***************

DFGSFIGFKGSEDLGDGLKAVWQLEQDVSVAGGGATQWGNRESFIGLAGEFGTLRAGRVA
************** ********************  ******* ***************

DFGSFIGFKGSEDLGEGLKAVWQLEQDVSVAGGGATRWGNRESFVGLAGEFGTLRAGRVA
************** ********************  ******* ***************

120       130       140       150       160       170
NQFDDASQAINPWDSNNDVASQLGIFKRHDDMPVSVRYDSPEFSGFSGSVQFVPAQNSKS
******* ** ****************************** ************ *****

NQFDDASQAIDPWDSNNDVASQLGIFKRHDDMPVSVRYDSPDFSGFSGSVQFVPIQNSKS
******* ** ****************************** ************ *****

NQFDDASQAIDPWDSNNDVASQLGIFKRHDDMPVSVRYDSPEFSGFSGSVQFVPIQNSKS
******* ** ****************************** ************ *****

NQFDDASKAIDPWDSNNVVASQLGIFKRMDDMPVSVRYDSPEFSGFSGSVQFVPAQNSKS
******* ** ****************************** ************ *****

180       190       200       210       220       230
AYKPAYYTKDTNNNLTLVPAVVGKPGSDVYYAGLNYKNGGFAGNYAFKYARHANVGRNAF
** **            ************************** ************* **

AYTPAHYTRQNNTDV-FVPAVVGKPGSDVYYAGLNYKNGGFAGSYAFKYARHANVGRDAF
** **            ************************** ************* **

AYTPAYYTKNTNNNLTLVPAVVGKPGSDVYYAGLNYKNGGFAGNYAFKYARHANVGRNAF
** **            ************************** ************* **

AYTPAHFVQQTPQQPTLVPAVVGKPGSDVYYAGLNYKNGGFAGNYAFKYAKHANVGRDAF
            S +
** **            ************************** ************* **

240 250 260 270 280 290
ELFLIGSATSDEAKGTDPLKNHQVHRLTGGYEEGGLNLALAAQLDLSENGDKAKTKNSTT
**** ** ** ************************************ * *******

ELFLLGS-TSDEAKGTDPLKNHQVHRLTGGYEEGGLNLALAAQLDLSENGDKAKTKNSTT
**** ** ** ************************************ * *******

ELFLIGS-GSDQAKGTDPLKNHQVHRLTGGYEEGGLNLALAAQLDLSENGD--KTKNSTT
**** ** ** ************************************ * *******

ELFLLGS-GSDEAKGTDPLKNHQVHRLTGGYEEGGLNLALAAQLDLSENAD--KTKNSTT
**** ** ** ************************************ * *******

300 310 320 330 340 350
EIAATASYRFGNAVPRISYAHGFDLIERGKKGENTSYDQIIAGVDYDFSKRTSAIVSGAW
************************* ***********************************

EIAATASYRFGNAVPRISYAHGFDLIERGKKGENTSYDQIIAGVDYDFSKRTSAIVSGAW
************************* ***********************************

EIAATASYRFGNAVPRISYAHGFDFIERGKKGENTSYDQIIAGVDYDFSKRTSAIVSGAW
************************* ***********************************

EIAATASYRFGNAVPRISYAHGFDFIERGKKGENTSYDQIIAGVDYDFSKRTSAIVSGAW
+************************ ***********************************

360 370
LKRNTGIGNYTQINAASVGLRHKF
************************

LKRNTGIGNYTQINAASVGLRHKF
************************

LKRNTGIGNYTQINAASVGLRHKF
************************

LKRNTGIGNYTQINAASVGLRHKF
************************

+ Note this amino acid 15 is located between
A.A.S.184 and 185 of this sequence

### EXAMPLE 5: DNA Sequencing of Class I OMP Genes from different N. meningitidis Serosubtypes

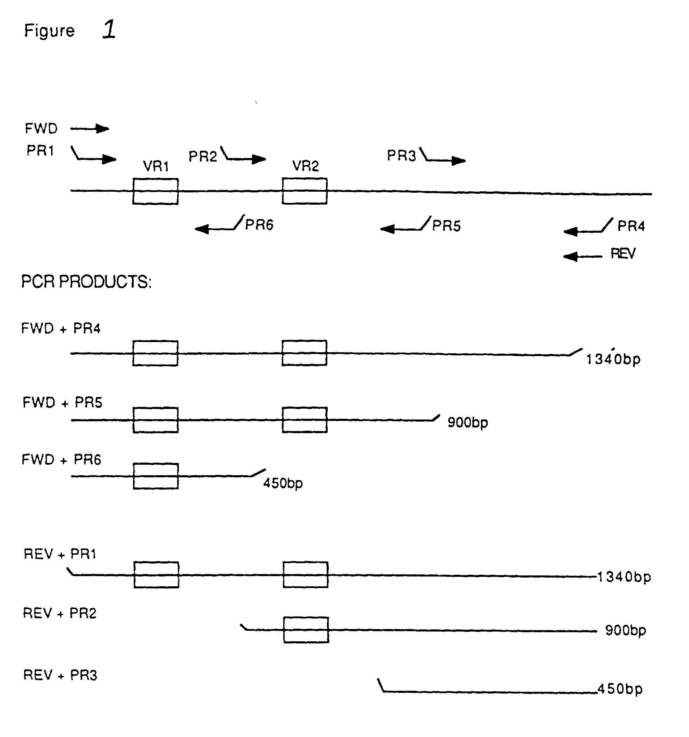

The Polymerase Chain Reaction (PCR) technique of Mullis and Faloona (Methods in Enzymol. 155:335--50, 1987) was used to amplify the entire Class I OMP gene and specific fragments according to the scheme shown in figure 1.

Primers were synthesized on an Applied Biosystems 380B DNA synthesizer and used in standard PCR 30 cycle amplification reactions using Taq polymerase in a Thermal Cycler (Perkin-Elmer Cetus, Norwalk, CT) according to the recommendations of the Supplier. Amplified fragments of about 1300, 900 and 450bp were generated from each serosubtype genomic DNA preparation from the primer combinations shown in Figure 1. The primers used had the following sequences:

PR1: (41 bases with universal primer extension)
TGT AAA ACG ACG GCC AGT TTG AAG ACG TAT CGG GRG TTT GC

PR2: (42 bases with universal primer extension)
TGT AAA ACG ACG GCC AGT GGC GAA TTC GGT ACG CTG CGC GCC

PR3: (42 bases with universal primer extension)
TGT AAA ACG ACG GCC AGT CAT CAG GTA CAC CGC CTG ACG GGC
[?]

PR4: (40 bases with universal primer extension)
TGT AAA ACG ACG GCC AGT GCA GAT TGG CAG TCA GAT TGC A

PR5: (40 bases with universal primer extension)
TGT AAA ACG ACG GCC AGT GGG ATC GGT ACC TTT GGC TTG A

PR6: (40 bases with universal primer extension)
TGT AAA ACG ACG GCC AGT AAC TGA TTC GCA ACG CGA CCG G

FWD: (24 bases)
TTG AAG GAC GTA TCG GGT GTT TCG

REV: (23 bases)
GCA GAT TGG CAG TCA GAT TGC TT

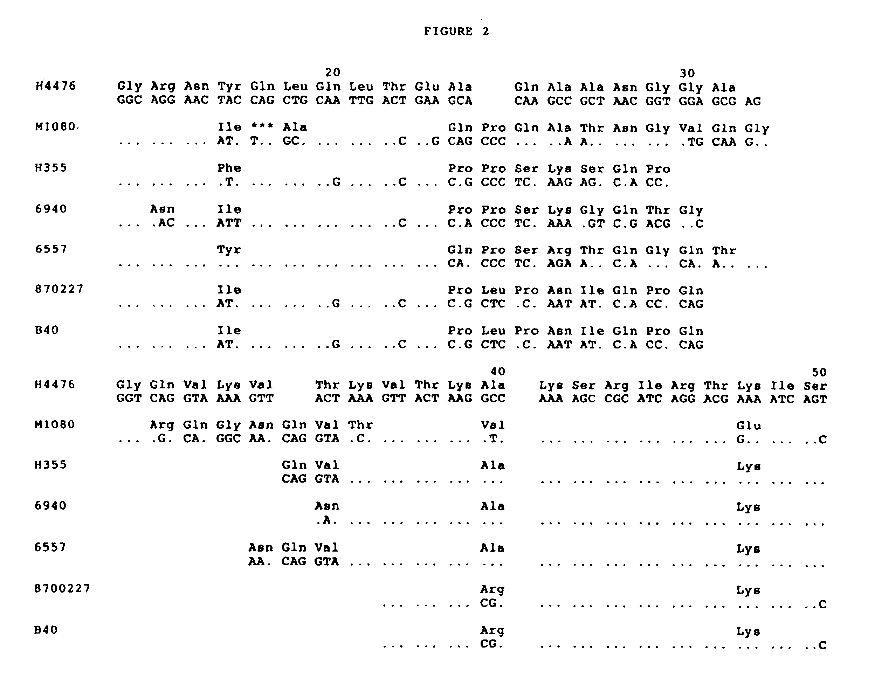

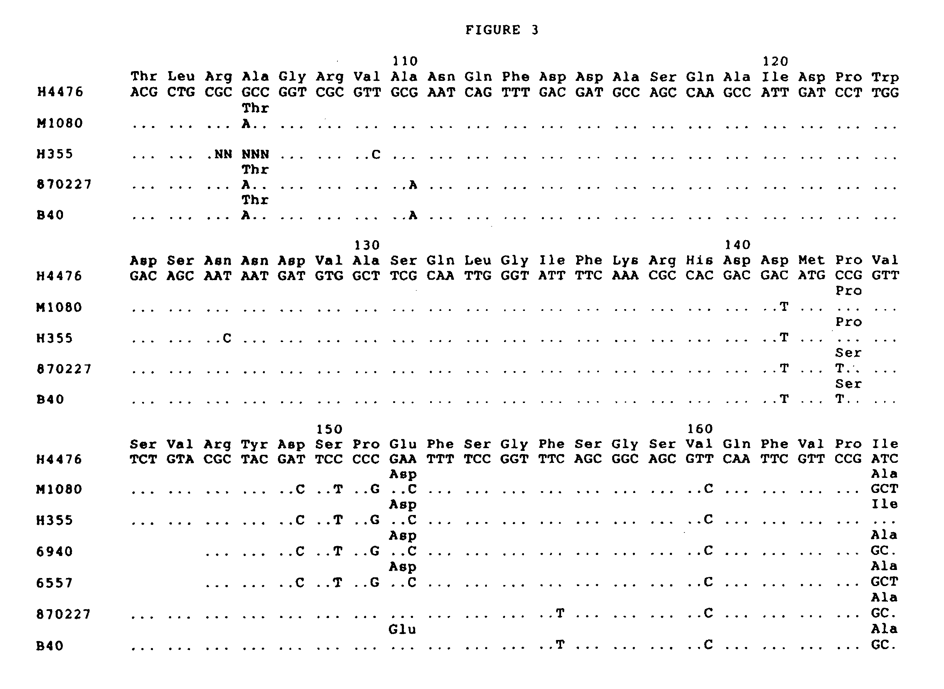

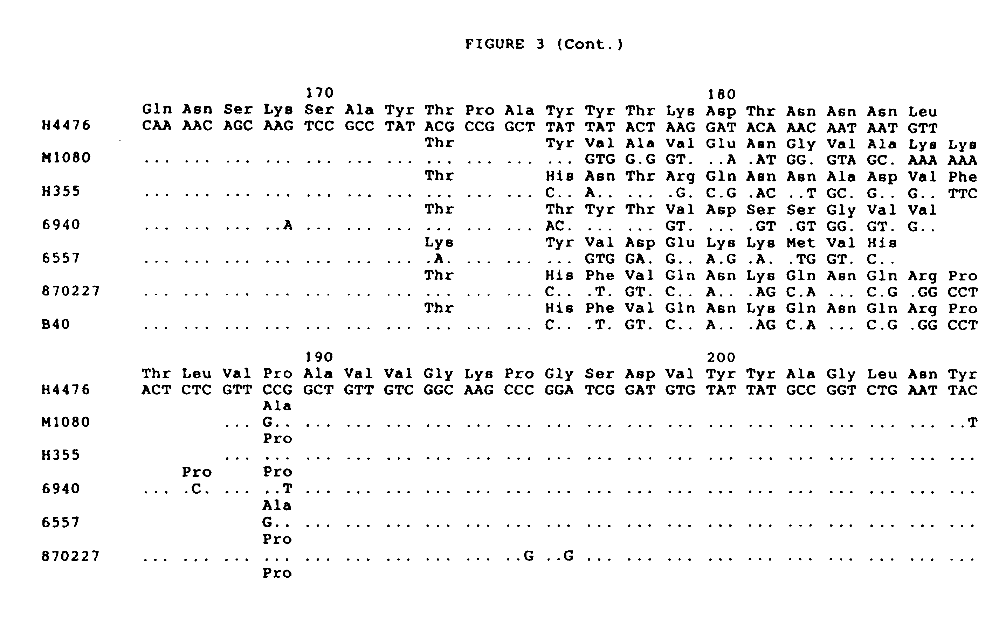

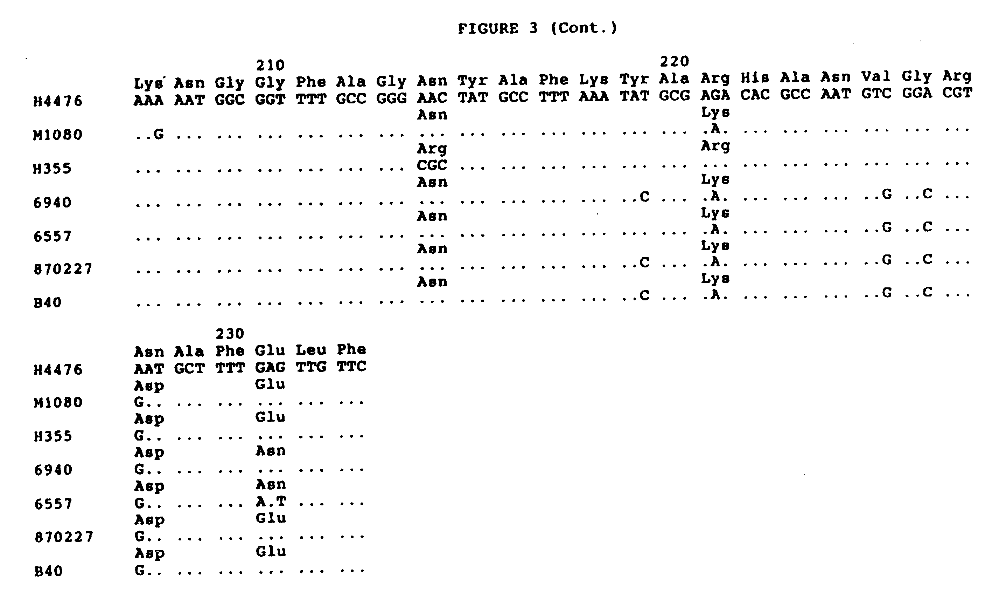

Excess single stranded template for sequencing was synthesized in an 'asymmetric PCR' amplification using 100x excess of primer carrying an 18 base extension at the 5' end corresponding to the universal fluorescent sequencing primers used with an Model 370A Automated DNA Sequencer (Applied Biosystems, Foster City, CA). Taq polymerase was used in a Standard dideoxynucleotide chain termination sequencing reaction with the PCR generated single stranded Class I gene fragments as templates. Derived sequences for gene segments of strains H44/76 (P1.7,16), M1080 (P1.1,7), H355 (P1.15), 6940 (P1.6), 6557 (P1.14), 870227 (P1.10) and B40 (P1.10) are shown in Figures 2 and 3.

### EXAMPLE 6: Confirmation of Amino Acid Sequences of Class I OMP Subtype Epitopes

From these gene sequences confirmed by direct sequencing of Class I OMP genes, it was deduced that the sequences corresponding to amino acids 24-34 and 176-187 of P1.16 are markedly variable in the four Class I OMP sequences. Three amino acid sequences N-terminal or C-terminal from these positions should also be considered for possible inclusion in these epitopes to allow for maximizing epitope stability presentation and unexpected insertions or deletions in the native protein sequence. Further the DNA and amino acid sequences of other Class I OMPs should be compared with the P1.7,16 sequence to allow for maximum allignment and epitope prediction. The first variable region epitope and second variable region epitope are called VR1 and VR2 respectively. These regions encode the subtype epitopes as was confirmed with the aid of peptide synthesis and the reaction of the peptides with P1.2; P1.7; P1.15 and P1.16 specific monoclonal antibodies.

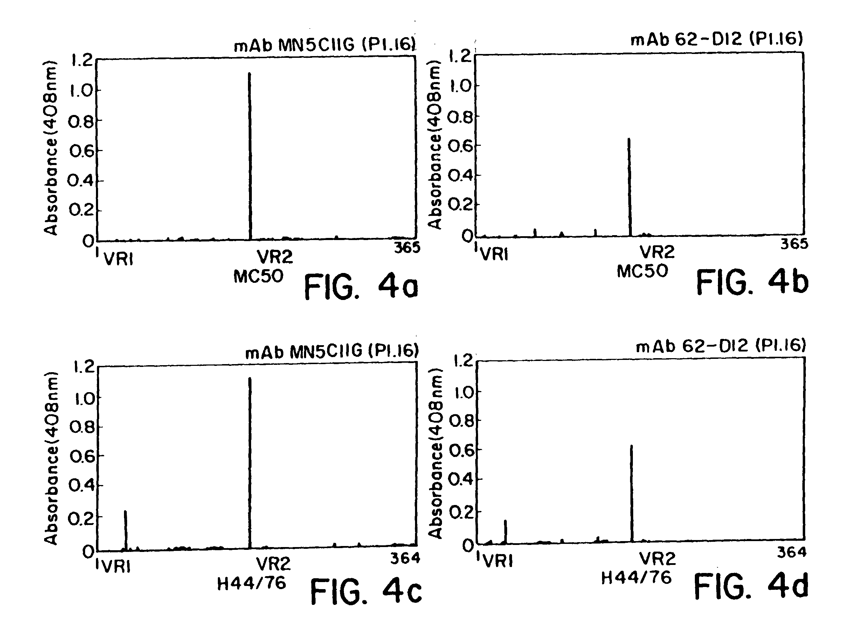

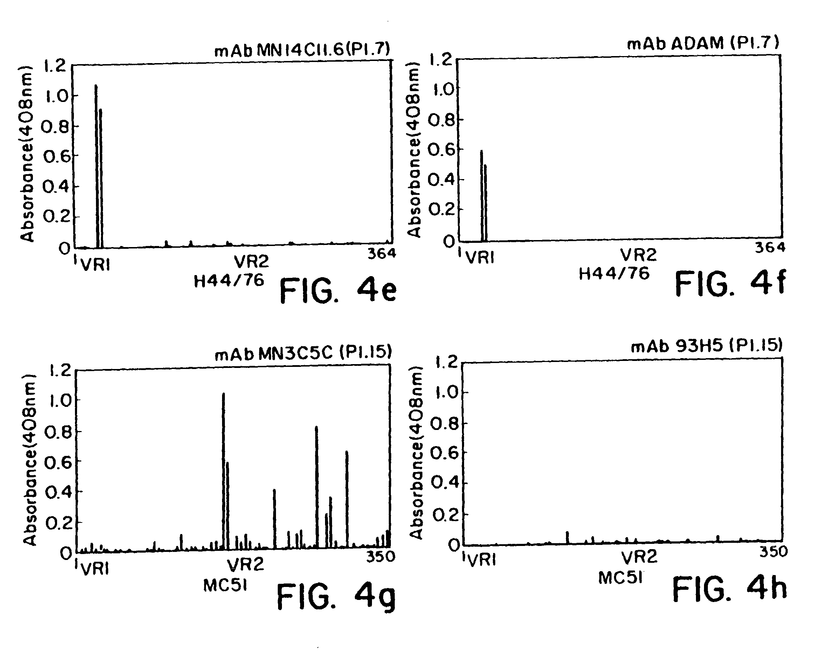

A complete set of overlapping decapeptides staggered by 5 amino acids were prepared using the P1.16 protein sequence. The anti- P1.16 monoclonal antibody reacted with the decapeptide YYTKDTNNNL from P1.16 reacted as expected and no other decapeptide. (Figure 4).

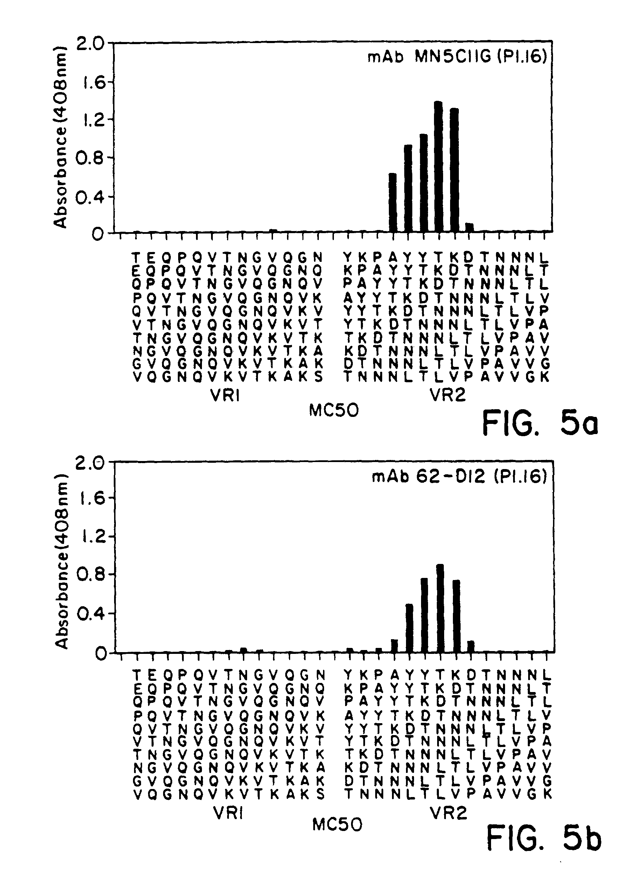

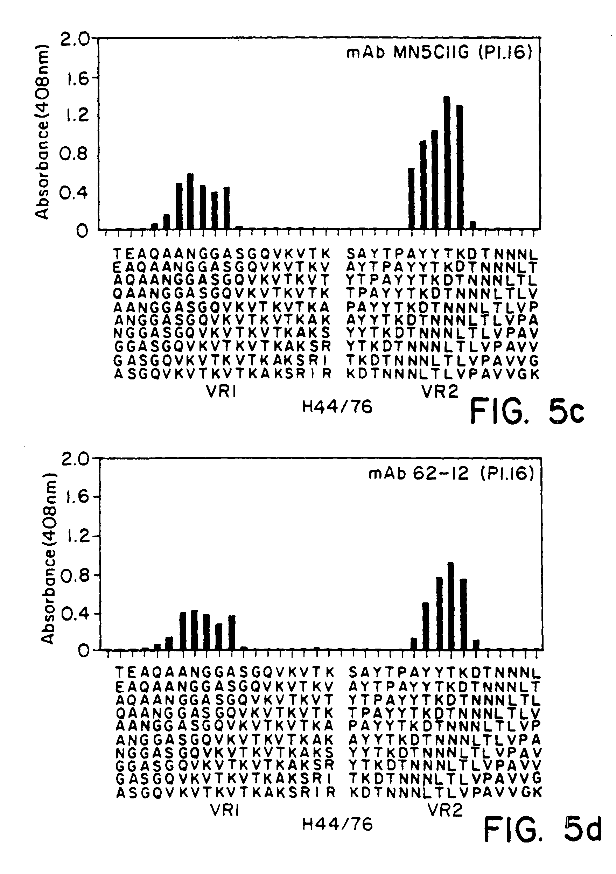

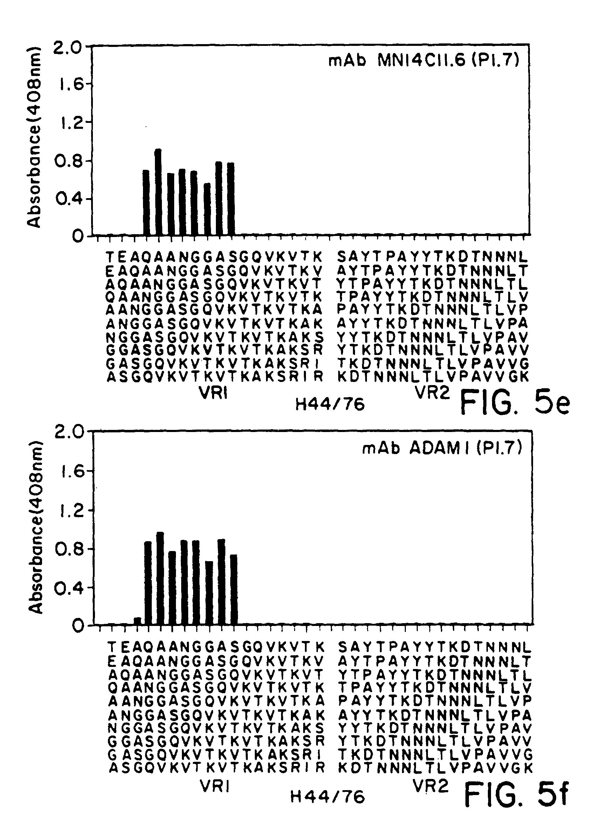

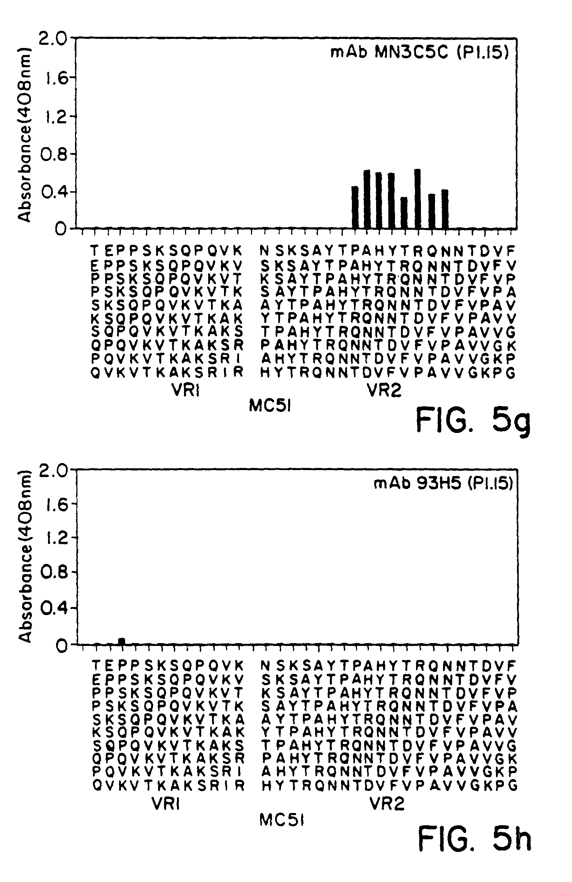

Of overlapping decapeptides provided with a one (1) amino acid sequence shift in the region 24-34 and 176-187 of the Class I OMP of strains H44/76 (P1.7,16), MC50 (P1.16) and MC51 (P1.15) more than one peptide reacted with the subtype specific monoclonal antibody. In most cases one or more of the group of these overlapping peptides reacted with the subtype specific monoclonal antibody more strongly than others (Figure 5).

These peptides are designated as the VR1 and VR2 epitopes. In the P1.7,16 strain, the sequence YYTKNTNNNL is present, the change D to N at residue 180 does have some effect on reducing antibody binding. The sequence HYTRQNNTDVF in P1.15 in the same relative position in the protein as the P1.16 epitope and is responsible for binding to the anti-P1.15 monoclonal antibody. AQAANGGASG shows some binding and peptides 1-3 amino acids downstream show far greater binding to the P1.7 monoclonal antibody. Sequence HFVQQTPQSQP of VR2 is responsible for binding to the anti-P1.2 monoclonal antibody. It is probable that the sequences QPQVTNGVQGN and PPSKSQP in the P1.16 and P1.15 proteins also represent epitopes.

### Example 6B: Class I OMP Constant Region Epitope Identification

Peptides forming surface loops were prepared and conjugated to tetanus toxoid. A Biolynx 4170 automated peptide synthesizer (Pharmacia/LKB) was used for continuous flow solid-phase synthesis with the following exception. In the last cycle of the synthesis SAMA-OPfp (o.5 mmol) (Drijfhout, J.W. (1989), Ph.D. Thesis, Leiden, The Netherlands) was coupled in the presence of 1-hydroxybenzotriazole (0.5 mmol) for 30 min., using a standard protocol wiht omission of the piperidine-treatment (i.e. the "Fmoc-deblocking step" which in this case would cause undesirable S-deacetylation). These are referred to as SAMA-peptides.

The peptides and their surface region location which were conjugated to TT are as follows:

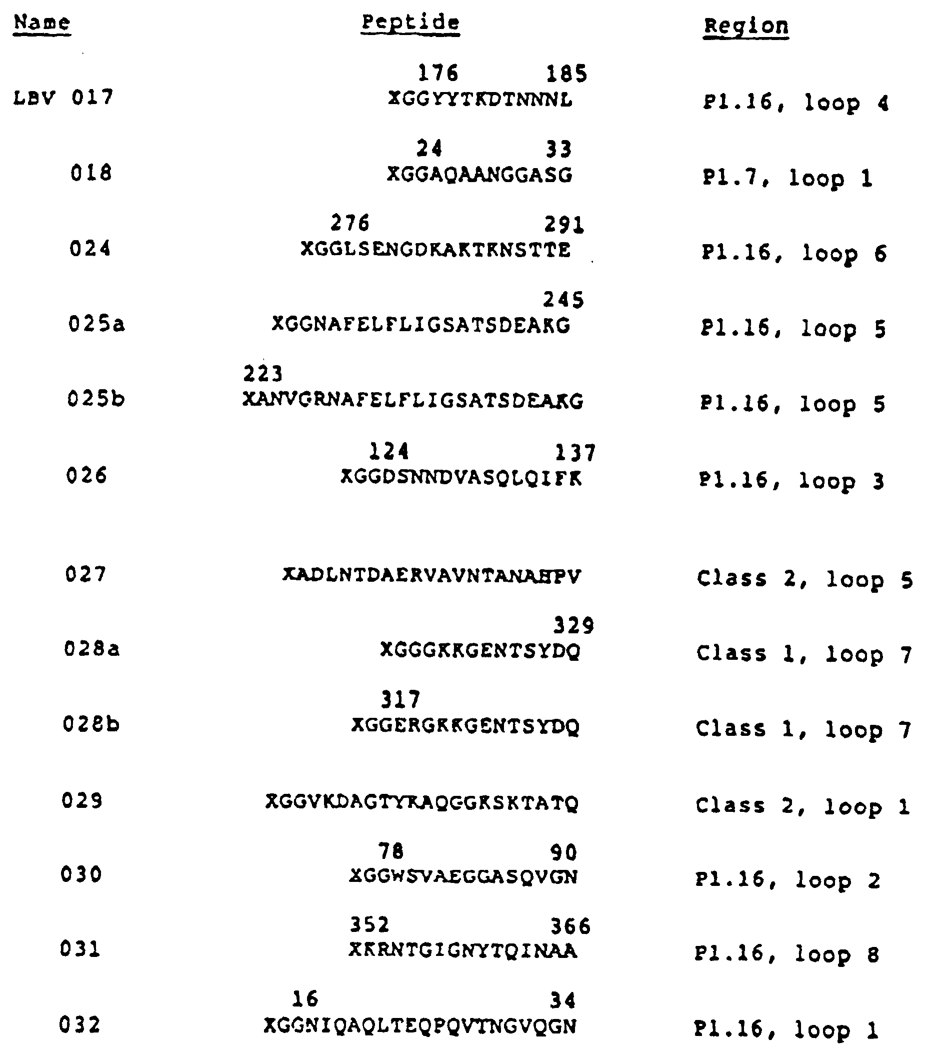

Conjugation of SAMA-peptides to tetanus toxoid was performed as follows. A solution of N-succinimidyl bromoacetate (4.7 mg, 10 µmol) in DMF (100 µl) was mixed with a solution of tetanus toxoid (TT) (20 mg) in 0.1 M. sodium phosphate buffer pH. 7.8 (3.5 ml). After 1h, 1.8 ml of the reaction mixture was subjected to gel filtration using a Sephadex PD-10 column (Pharmacia) equilibrated in 0.1 M sodium phosphate, containing 5mm EDTA (PE buffer) ph 6.1. The bromoacetylated tetanus toxoid was eluted with the same buffer and collected in 3.5 ml. The solution of bromoactylated tetanus toxoid (1.2 ml) was added to the SAMA peptide (4.5 mg. 3 µmol) and deaerated with helium. Next, 150 µl of 0.2 M hydroxylamine (in PE buffer, pH 6.1) was added. After 16 h remaining bromoacetyl groups were blocked by addition of 2-aminoethanethiol hydrochloride (4 µmol) in buffer, pH 6.1 (150 µl). After a further period of 16 h, the peptide-TT conjugate was purified by gel filtration over a PD-10 column using PE buffer, pH 6.1, as the eluant. The appropriate fractions were combined and stored at 4 ° C.

To determine the immunological activity, 25 µg (total protein) per does of a peptide-TT conjugate was injected subcutaneously at weeks 0 and 4 into 6-8 week old NIH outbred mice. (Note: Vaccine LBV 017-TT and LBV 018-TT were used at 10 µg total protein/dose.) Sera were colledted 6 weeks following the first dose and evaluated for antibody response in an ELISA assay (Beuvery, E.C. et al. (1983) Infect. and Immun. 40:3690380). The following antigens were coated into the microtiter wells: Outer membrane proteim (OMP), purified Class I OMP (Poolman, J.T. et al., (1989) Infect. and Immun. 57:1005) and the unconjugated peptides. Bactericidal activity (BC) of sera was also measured (Poolman, J.T. et al., (1985) supra.)

The results are presented in Table 2 below.

**TABLE 2**

| Vaccine | OMC | Class 1 OMP | Synth. Peptide | Bactericidal Test |
|---|---|---|---|---|
| LBV 018-TT | 1:900 (0.05)* | 1:2700 | ND | <1:64 |
| LBV 017-TT | 1:900 (1) | 1:900 | ND | <1:64 |
| LBV 024-TT | 1:100 | 1:100 | 1:900 (homol.) | <1:64 |
| LBV 025a-TT | - | 1:100 | 1:2700 (homol.) | <1:64 |
| LBV 025b-TT | 1:2700 (4) | 1:300 | 1:8100 (homol.) | <1:64 |
| LBV 026-TT | - | - | - (homol.) | <1:64 |
| LBV 027-TT | - | 1:300 | 1:300 (homol.) | <1:64 |
| LBV 028a-TT | 1:100 | - | 1:2700 (homol.) | <1:64 |
| LBV 028b-TT | 1:100 | 1:100 | 1:900 (homol.) | <1:64 |
| LBV 029-TT | - | 1:100 | 1:8100 (homol.) | <1:64 |
| LBV 030-TT | - | 1:100 | 1:2700 (homol.) | <1:64 |
| LBV 031-TT | - | 1:100 | - (homol.) | <1:64 |
| LBV 032-TT | - | 1:100 | 1:900 (homol.) | <1:64 |

| | | | | |
|---|---|---|---|---|
| * numbers in ( ) indicate O.D. level showing this titer | | | | |

These data suggest that of the constant surface loops tested of Class 1 and 2 OMPs of N. meningitidis loop 5 appears to represent at least one region that will produce antibodies which will cross-react with Class 1 and Class 2 OMP of many strains of N. meningitidis.

### EXAMPLE 7: Construction of recombinant flagellins expressing meningococcal epitopes

To create hybrid flagella containing epitopes from class I meningococcal epitopes, a series of oligonucleotides was designed based on primary protein sequence data and epitope mapping data. Two oligonucleotides based on VR1 or VR2 epitopes of outer membrane P1.7.16 were designed so that they could be cloned in single or multiple copies into a cloning region within the gene for S. muenchen flagellin. Translation termination signals were included on the non-coding strand of the oligonucleotide to facilitate screening by expression of the cloned inserts.

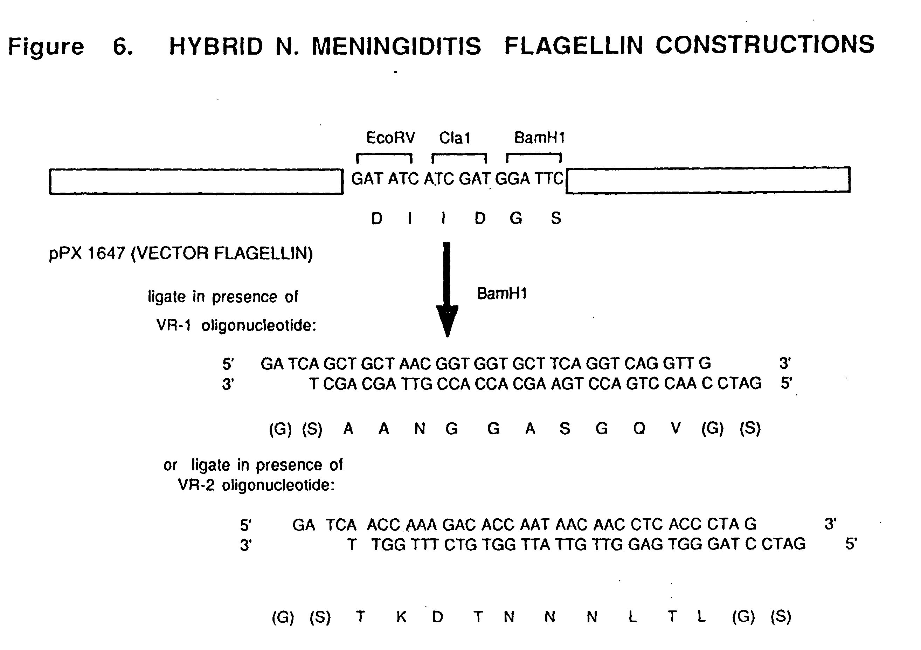

The plasmid vector pPX1650 containing the entire coding region and promoter regions for the structural gene for flagellin H1-d of Salmonella meunchen (deposited at the ATCC, accession #67685) was modified to contain several unique cloning sites suitable for the insertion of either oligonucleotides or gene fragments in each of the three reading frames of the flagellin gene (Figure 6). First, pPX1650 was digested with EcoRV, which cleaves pPX1650 twice, 48 base pairs apart, and religated to yield a plasmid, pPX1651, which has a unique EcoRV cloning site and which results in a 16 amino acid deletion in the flagellin protein. pPX1651 was identified by screening E. coli recombinants on Western blots probed with polyclonal antibody directed against H1-d flagellin. pPX1651 was identified amongst several candidates having flagellins smaller than wild type flagellin (of 1650) and was verified by sequencing. Second, pPX1651 was restricted with BamH1 and religated after filling out the overhanging ends with Klenow enzyme to remove the unique BamH1 restriction enzyme site in the polylinker region of the vector. As a final step, the resulting vector was digested with EcoRV and the following oligonucleotide linker was inserted: 
5' ATG ATC GAT GGA TTC 3'
3' TAG TAG CTA CCT AAG 5'
 Candidates were screened for the newly created BamH sites and several candidates having BamH1 sites were screened for orientation of the linker by double strand DNA sequencing methodology. One candidate having the linker in the above orientation was retained as pPX1647:

5'.... GAT ATC ATC GAT GGA TTC ATC....
EcoRV Cla1 BamH1

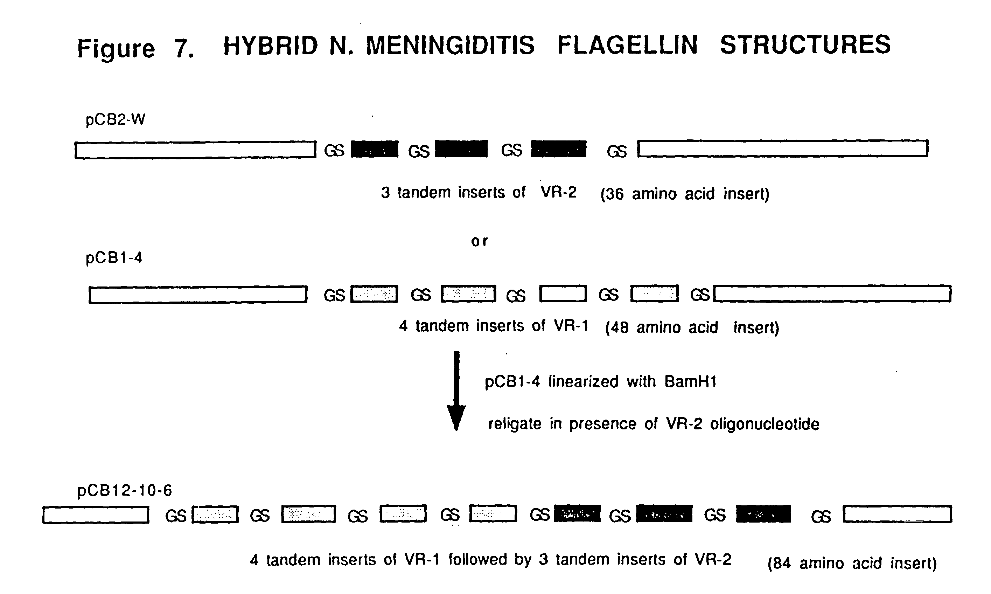

Plasmid pPX1647 (Figure 7) was digested with BamH and either oligonucleotides for VR1 or VR2 were cloned into E. coli cells. Screening for desired recombinants was accomplished by digesting plasmid minilysate DNA with appropriate diagnostic restriction enzymes and screening for expression by probing hybrid flagella for decreased mobility on SDS-PAGE gels with specific flagellar antiserum (H1-d). A number of the resultant clones showed decreased mobility on SDS-PAGE, indicating proper insertion of one or more of the oligonucleotides for VR1 or VR2. Several of each were retained for analysis by DNA sequencing. Clone CB1-2 results from tandem insertion of two copies of the VR1 oligonucleotide and clone CB1-4 results from insertion of four oligonucleotides. Likewise CB2 P contained a single insert of the VR2 oligonucleotide and CB2 W showed the expected trimeric insert, CB2 P clone contained a single base pair change which resulted in a change from Leu to Phe in the expressed VR2 fusion protein and was not retained for further study. The recombinant flagellin clones in E. coli were probed with monoclonal antibodies (Abdillahi and Poolman, Microbiol. Pathogenesis 4:27-32, 1988; RIVM, The Netherlands) known to react-with either VR1 or VR2 epitopes. Monoclonals Adam-1 (P1.7) and Mn14-C11-6 (P1.7) react with hybrid flagellin containing 2 or 4 tandem inserts of VR1, but do not react with clones containing VR2. The weaker reaction of both monoclonals with CB1-2 than with CB1-4 is likely due to epitope density. By the same token, monoclonals 62 (P1,16) and Mn5-c11-G (P1.16) react with CB2 W clone, but not with the VR1 inserts. The CB2 P clone fails to react with either VR2 antibody, probably due to the Leu to Phe change.

Each of these clones was transformed into an aroA S. dublin strain (SL5927), having a Tn10 insertion in the H1-d locus, to examine the functioning of the hybrid flagella. Each of the four clones resulted in motile bacteria; motility of the transformants was inhibited by the corresponding monoclonal antibody, including clone CB2 P, indicating affinity of the VR2 monoclonal for the epitope in intact flagella. This result indicates that epitopes are exposed at the cells surface and are accessible to antibody.

Hybrid flagellin containing both VR1 and VR2 epitopes were created by cleaving either CB1-2, CB1-4, or CB2 W with BamH1 and cloning the heterologous epitope. Clones CB12-7 and CB12-10 result from the in-frame insertion of a single copy of the VR2 oligonucleotide behind either 2 or 4 VR1 tandem inserts, respectively; clone CB21-F arose from the insertion of one copy of the VR1 epitope behind 3 tandem copies of VR2. CB12-7 and CB12-10 are recognized only by VR1 monoclonal antibody and CB21-F is recognized only by VR2 monoclonal. These results, taken together with DNA analysis revealing predicted sequences, indicate epitope density is too low in the combined hybrids. To create a hybrid flagellin with increased density of both VR1 and VR2 epitopes, CB12-10 was digested with BamH1 and VR2 encoding oligonucleotides were inserted. Clone 12-10-6 contains two further tandem inserts of the VR2 epitope, resulting in a hybrid flagellin molecule in which four tandem copies of VR1 are followed by three copies of VR2. As is shown in Figure 3a and b, three of the hybrid flagellin vaccine candidates have the expected molecular properties. The flagellin (pCB1 x 4) containing 4 copies of VR1 reacts with anti-H1-d (anti-flagellin) and anti-VR1 monoclonal antibodies, but not with anti-VR2 monoclonal antibodies; the flagellin (pCB2-W) containing 3 tandem copies of VR2 reacts with anti-H1-d and anti-VR2 antibodies, but not with anti-VR1; the combined hybrid containing copies of VR1 and 3 copies of VR2 reacts with both anti-VR1 and anti-VR2 monoclonal antibodies. The combined hybrid specified motility when introduced into a non-motile recipient S. dublin strain.

As a subunit vaccine, the goal is to obtain suitable initial vaccine candidates in high quantity and high purity. A suitable vaccine candidate can be chosen from the above type constructions based on reactivity to monoclonal antibodies and function of flagella in non-motile Salmonella host strains. A subunit flagellin vaccine may not need to retain all functional aspects of a parental flagellin, but should at least retain surface localization for purification purposes. Several subunit flagellin meningococcal vaccine were chosen from the above described hybrid molecules based on reactivity to monoclonal antibodies and implied surface localization based on restoration of bacterial motility. Three flagellin vaccine candidates contained either 4 tandem inserts of VR1, 3 tandem inserts of VR2, or 4 VR1 inserts followed by 3 VR2 inserts. Because flagellin is a major protein of Salmonella, it is possible to easily purify sufficient material for vaccination studies using techniques established for flagella purification (Logan et al., J. Bacteriol. 169: 5072-5077, 1987).

### EXAMPLE 8: Initial Purification of recombinant flagellin molecules

The three hybrid flagellin vaccine candidates and a wild type (derived from pPX1650) were inoculated into four-liter baffled Fernbach flasks containing 1 liter of LB broth. Bacterial cultures were incubated at 37 ° C with shaking (200 rpm) for 22-24 hr. Under these conditions of culturing, the bulk of the flagella were sloughed from the bacterial cell surface and were localized in the supernatant culture medium. To obtain suitable material, flagella were isolated from 6-8 liters of culture medium. To obtain purified flagellin preparations for vaccination studies, flagellar filaments were harvested from bacterial culture supernatants by the following procedure: Ammonium sulfate was added to culture supernatant so that final solution was 50% saturated; the solution was stirred gently at 4°C for several hours and the precipitated material was collected by centrifugation in a GSA rotor at 5000 rpm for 30 minutes. The collected ammonium-sulfate precipitated material was reconstituted in PBS and dialyzed against PBS at 4°C for 12-15 hrs. The dialyzed material was subjected to high speed centrifugation at 100,000 x g for 1 hour in an SW-27 rotor to pellet the flagellar filaments. The pelleted material, which consisted primarily of flagellin, was subjected to further purification by the following method.

### EXAMPLE 9:HPLC Purification of recombinant flagellins

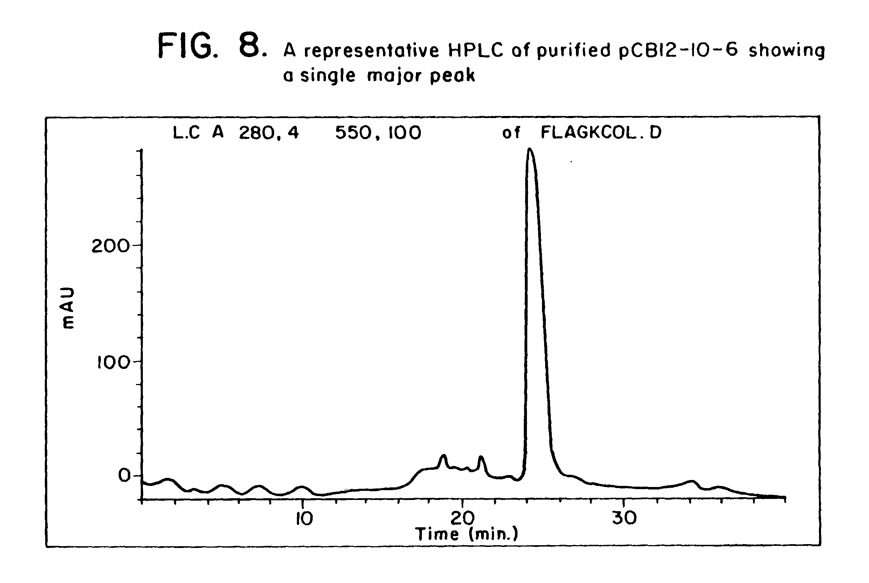

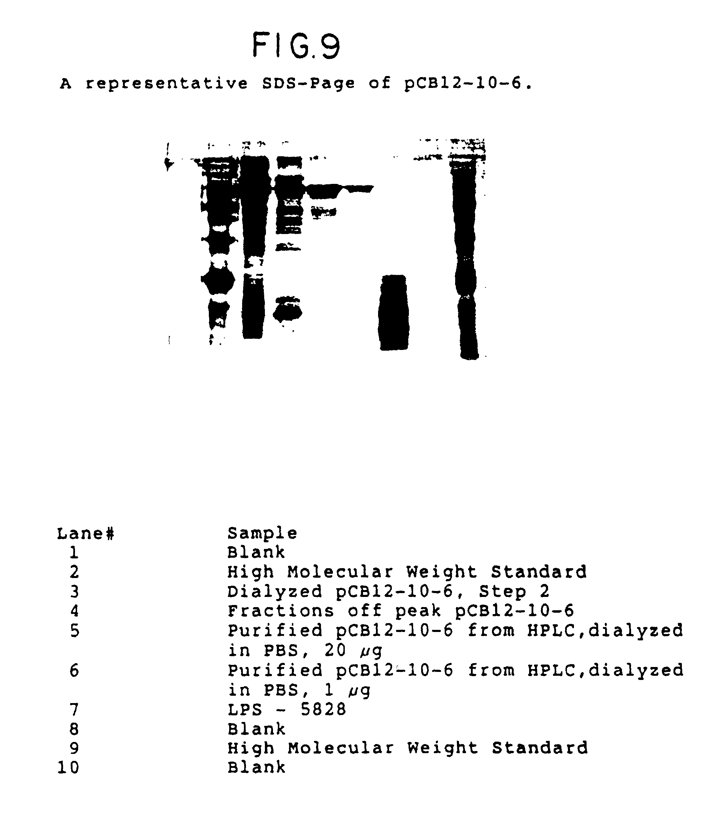

To prepare highly purified flagellins, Salmonella expressing the constructions, in particular pCB12-10-6, was grown as described above and the cells pelleted at 10,000G. The culture supernatant was then precipitated with 50% ammonium sulfate, centrifuged at 10,000G and resuspend in 30 ml PBS. The resuspended pellet was dialyzed against 10mM Tris buffer (pH=8.0) containing 6M urea, 1mM PMSF, 2mM NEM, and 5mM EDTA overnight at 4°C. Dialyzed material was then passed over two DEAE sepharose minicolumns (3.0 ml volume, 4.0 ml eluent over each). The columns were eluted (5X) with 50mM NaCl in 10mM tris (pH=8.0) containing 6M urea and then with 1M NaCl in 10mM tris (pH=8.0) containing 6M urea. The first four elution collections (20 ml) of the 50mM NaCI were pooled and dialyzed against 1.0 liter 10mM Acetate buffer (pH=4.0) in 6M urea at room temperature. The dialyzed fractions were then loaded onto a TSK SP PW cation exchange HPLC column (75mm x 300mm). The column was eluted with a mobile phase consisting of 10mM Acetate (pH=4.0) containing 6M urea. A gradient 0 - 300mM NaCL was established in 10mM acetate (pH=4.0) containing 6M urea over the 5 - 30 min interval. After 30 mins the gradient went from 300mM to 1M NaCI in 10mM acetate in 6M urea over the next 5 min. The flagellin construct was collected at approximately 24 min. which corresponds to about 200mM NaCl. The fraction was dialyzed against PBS and purity determined on the material was established by Western blots using anti-flagellin antibody. A representative HPLC analysis and SDS-PAGE are shown in figures 8 and 9 respectively.

### EXAMPLE 10: Preparation of meningococcal-flagellin glycoconjugate

Group C meningoccal capsular polysaccharide (GCM CPS: lot # 86 NM 01) was prepared essentially according to Bundle et al., J. Biol. Chem. 249: 4797-801, 1974).

Neisseria meningitidis strain C11 was obtained from the Walter Reed Army Institute (Washington, DC). The strain was precultured twice on sheep blood agar plates, then used for the inoculation of a liquid seed culture medium Neisseria chemically defined medium, NCDM) Kenney et. al., Bull. W.H.O. 37 469-73, 1967). Finally, 40 I of liquid medium (NCDM) in a fermentor was inoculated with the liquid preculture. The purity of the strain was checked at each stage. After centrifugation, the supernatant was precipitated by addition of Cetavlon to a final concentration of 0.1%, and the insoluble complex re-dissolved in cold 1 M calcium chloride (CaCl₂) (Gotschlich et al., J. Exp. Med. 129:1349-65, 1969). Ethanol (96%) was added to a final concentration of 25% (v/v). After 1 h, the suspension was centrifuged (1 h, 50,000 g), the supernatant was collected, and its ethanol concentration was increased to 80% (v/v). After 1h, centrifugation (20 min, 5,000 g) yielded a precipitate which was washed successively with absolute ethanol, acetone, and diethylether, and then dried in a vacuum dessicator over phosphorus pentoxide (P₂O₅) to constant weight. This crude CPS was stored at -20 ° C.

In order to obtain a purer preparation, the CPS was then dissolved in sodium acetate buffer (1.10 dilution of a saturated solution, pH 7.0) and extracted four times wiht hot phenol (Westphal et al., Z. Naturforsch 7b:148-55, 1952). After dialysis of the combined aqueous phases against 0.1 M CaCl₂, followed by centrifugation (3-5 h, 100,000 g), a final ethanol precipitation was performed on the clear supernatant, and the resulting precipitate washed with organic solvents and dried, as described above. The pure CPS was then stored at -20 ° C.

At each stage of the purification process, the CPS was analyzed for carbohydrate N-acetylneuraminic acid, NANA) (Svennerhold, Biochim. Biophys. Acta 24:604, 957), O-acetyl (Hestrin, J. Biol. Chem. 180:249, 1949), and protein (260 nm detection) content, and its molecular weight checked by gel filtration.

Group C meningococcal capsular polysaccharide (GCM CPS) was simultaneously depolymerized and activated via sodium periodate (NalO₄) oxidation in aqueous buffer (Anderson et al., J. Immunol. 137:1181-6, 1986; Eby et al., Pediat. Res. 20:308A, 1986, Anderson et al., J. Pediatr. 111(5):644-50, 1987; Anderson, U.S. Pat. 4,762,713; 1988). The reaction was monitored by high performance gel permeation chromatography (HPGPC) in aqueous eluent, using ultraviolet (UV) and refractive index (RI) detection. The reaction was stopped and the activated oligosaccharides (GCM OS) were desalted by low pressure gel permeation (GPC) in water, and then lyophilized. A solution was then prepared in water and subsequently frozen for temporary storage. GCM OS and flagellin pCB12-10-6 were mixed in aqueous neutral buffer and the conjugation was initiated by addition of sodium cyanoborohydride (NaBH₃CN) (Anderson, U.S. Patent 4,762,713, 1988; U.S. Patent 4,673,574, 1987; U.S. Patent 4,761,283, 1988). The reaction was carried out for 5 days, while being monitored by HPGPC. It was finally stopped by dialysis/concentration on centrifugal microconcentrators. The final preparation was stored in the cold, in the presence of thimerosal to prevent bacterial growth. The resulting glycoconjugate not only provides a mechanism to present the expressed VR1 and VR2 meningococcal epitopes to the immune system but also serves as a carrier molecule for the presentation of a meningococcal oligosaccharide.

In preparation of the-conjugate, the-following conditions were employed. Purified flagellin pCB12-10-6 was dissolved in 15% sucrose (3.5 mg/ml) and then stored at -20°C. GCM CPS (9.7 mg; final concentration: 5 mg/ml) was oxidized by 100 mM NaIO₄ in 0.05 M sodium phosphate buffer (pH 6.2 - 6.5) at RT, in the dark, with agitation. Aliquots (100 µl) were withdrawn at regular intervals, the reaction stopped by addition of ethylene glycol (10 µl), and analysis was performed by HPGPC on Waters (Milford, MA) Ultrahydrogel™ 250 + 120 (2 columns coupled; 2 x 300 mm x 7.8 mm) in 0.2 M phosphate-saline buffer (PBS; 0.2 M sodium phosphate, 0.9% NaCI, pH 7.8), at a flow rate of 0.8 ml/min, using UV (206 nm) and RI detection. After 2 h 30 min, the reaction was stopped by addition of ethylene glycol (1/10 of the reaction volume), and the GCM OS were desalted by GPC on Bio-Rad (Richmond, CA) Bio-Gel^{R} P-2 (200-400 mesh, 30 cm x 1.5 cm) in water, at about 18 ml/h. Fractions were collected (1.2 ml) and analyzed for the presence of NANA the carbohydrate N-acetylneuraminic acid (NANA) (Barry et al., J. Gen. Microbiol. 29:335-52, 1962) and aldehydes (Porro et al., Anal. Biochem 118:301-306, 1981). Positive fractions were pooled and lyophilized. Desalted GCM OS (4.7 mg) were then dissolved in water (10 mg/ml) and frozen at -20 °C.

Both GCM OS and pCB12-10-6 solutions were analyzed by HPGPC (UV at 206 and 280 nm respectively) before being frozen, and prior to the conjugation. No degradation occurred during storage, as ascertained by the exact similarity of the elution profiles.

GCM OS (2 mg; final concentration: 2.6 mg/ml) and flagellin pCB12-10-6 (2.3 mg; final concentration: 3 mg/ml) were mixed in a polypropylene tube in 0.4 M sodium phosphate buffer (pH 7.0), and NaBH₃CN was added (12 µmoles) to initiate the conjugation (Anderson, U.S. Patent 4,762,713, 1988; U.S. Patent 4,673,574; U.S. Patent 4,761,283). The reaction mixture was left one day at RT, then 4 days at 35°C, without agitation. The reaction was monitored by HPGPC (UV at 280 nm) at different stages, and finally stopped by dialysis/concentration on microconcentrators. The final preparation was analyzed for NANA (Barry et al., J. Gen. Microbiol. 29:335-353, 1962) (0.09 mg at 0.12 mg/ml) and protein (Lowry et al., J. Biol. Chem. 193:265-275, 1951) (1.12 mg; 1.45 mg/ml) content. It was then stored at 4°C in the presence of thimerosal (0.01%, w/v) to prevent bacterial growth.

The conjugate preparation was also checked by SDS-PAGE (silver nitrate stain) and Western blots analyzes. Several high molecular weight bands appeared on the gel above the pure pCB12-10-6 band and near the stacking well, the latter being an evidence that cross-linking occurred during conjugation. Western blot analyzes showed that each band was reactive with the antisera used (anti-GCM, -VR1, and -VR2), proving covalency of the conjugate bonds.

### EXAMPLE 11: Conjugation of Meningococcal peptides to CRM and bovine serum albumin

Peptides designated as M20 and M21 were produced on an ABI model-peptide synthesizer by solid phase synthesis using the tBoc chemistry were coupled to CRM₁₉₇ (prepared as described by Andersen, U.S. Patent No. 4,762,713) using a bifunctional crosslinking agent, sulfosuccinimidyl (4-iodoacetyl) amino benzoate (Sulfo SIAB; purchased from Pierce) following the modification of a published procedure (Welt-man, J.K. et al., (1983) Bio Techniques 1, 148-152). Briefly CRM₁₉₇ was activated by sulfo SIAB resulting in the formation of an amide bond between SIAB and amino groups of CRM₁₉₇. After the removal of unreacted crosslinker from the activated CRM₁₉₇ by gel filtration, peptide (M20 or M21) containing linking spacer (represented in underlined letters) with carboxy terminal cysteine residue was mixed with activated CRM and incubated at room temperature for 2-4 hours. Following the reaction, the conjugated material was dialyzed extensively against PBS at 4 C. The sequence of M20 peptide (VR2 epitope) is as follows: 
H-Tyr-Tyr-Thr-Lys-Asp-Thr-Asn-Asn-Asn-Leu-Thr-Leu-V-
al-Pro-Ala-Gly-Ala-Cys-OH
 The sequence of M21(VR1 epitope) peptide is:

H-Ala-Gln-Ala-Ala-Asn-Gly-Gly-Ala-Ser-Gly-Gln-Val-L-
ys-Ala-Gly-Ala-Cys-OH.

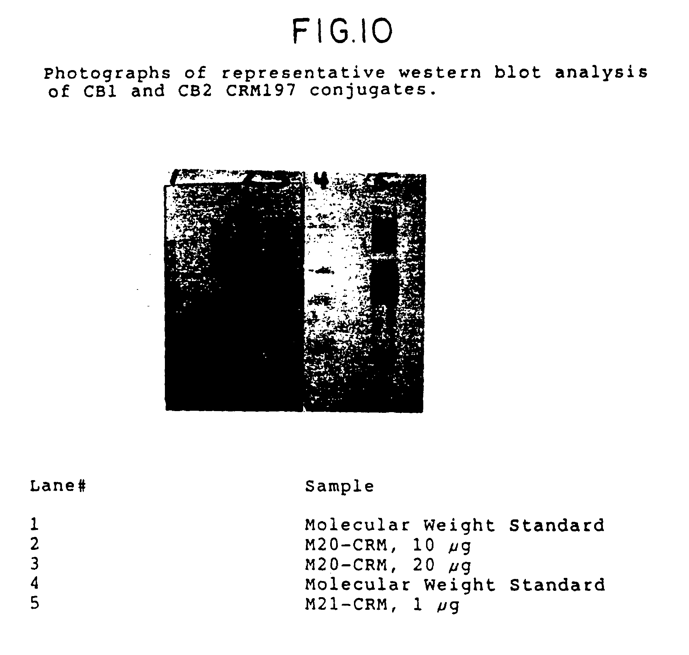

Conjugated materials were subjected to SDS PAGE, transferred to PVDF membranes (Immobilon, Millipore) and reacted with specific monoclonals which recognize VR1 and VR2 epitopes. Figure 10a and 10b show the western blot analysis of M20 and M21 CRM₁₉₇ conjugates, against a pool of VR1 and VR2 specific monoclonals (Adam I, G2-D12-8 (P1.7), MN5-C11-G (P1.16) and MN14-C11-6 (P1.7)).

In order to assay the antibody response to M20 and M21 peptide by enzyme linked immunoassay procedure, BSA conjugates were prepared by using a different bifunctional crosslinking agent, N-Succinimidyl Bromoacetate as described by Bernatowicz and Matsueda (Anal. Biochem. 155, 95-102 (1986)). Covalent coupling of peptide to the protein was confirmed by western blotting of electrophoresed samples as described for CRM₁₉₇ conjugates.

### EXAMPLE 12: Retention of T cell activity by M20 and M21-CRM₁₉₇ conjugates

To determine whether conjugation of the VR1 and VR2 epitopes to CRM₁₉₇ adversely affect the T cell recognition of the CRM₁₉₇ protein a T cell proliferative assay was performed as previously described by Bixler and Atassi (Immunol. Commun. 12:593, 1983). Briefly, SJL/j mice were immunized with 50 µg of native CRM197 emulsified in CFA. Seven days later, lymph nodes were removed, cultured in RPMI and challenged with various concentrations of proteins (0.05-100.0 µg/ml) and peptides. After 3 days incubation, cultures were pulsed with [³H]-thymidine for 16 hours and then harvested for counting.

**TABLE 3**

| T cell responses to meningococcal peptide-CRM197 conjugates. | | | |
|---|---|---|---|
| In Vitro Challenge | Maximum observed [³H] Incorporation | | |
| | µg/ml | ΔCPM | SI |
| Diphtheria toxoid | 5 | 27,510 | 57 |
| CRM197 | 50 | 108,631 | 221 |
| CRM197 - mock conjugate | 100 | 116,326 | 236 |
| M21-CRM197 | 100 | 182,499 | 370 |
| M20-CRM197 | 10 | 89,972 | 183 |
| CON A | 1 | 34,316 | 70 |
| LPS | 50 | 61,579 | 126 |
| Tetanus toxoid | 10 | 515 | 2 |
| Background (cpm) | - | 494 | 1 |

As shown in Table 3, a comparison of CRM₁₉₇ with the CRM₁₉₇-mock conjugate shows that the conjugation procedure by itself did not alter the T cell recognition of the protein. The T cell responses induced by the M20 and M21-CRM₁₉₇ conjugates were essential equivalent to or greater than the response elicited by CRM₁₉₇ itself indicating that the recognition of the T cell epitopes on the CRM₁₉₇ is not adversely affected by the peptide conjugation. The responses to the control materials Con A, LPS and Tetanus toxoid were as expected.

### EXAMPLE 13: Immunogenicity of conjugate and recombinant meningococcal b vaccines

Recombinant flagellin expressing the meningococcal VR1 and/or VR2 epitopes were prepared and purified as described in Examples 7, 8 and 9. In addition, synthetic peptides representing the meningococcal epitopes VR1 and VR2 were synthesized, covalently coupled to the carrier molecule CRM₁₉₇ and purified as in Example 12. Vaccines were formulated with each of these materials at protein concentrations of 10 or 100 µg/ml for each of the components. The vaccine compositions also included aluminum phosphate at 1 mg/ml or except as noted were compounded with Freund's complete adjuvant or without supplemental material.

To evaluate immunogenicity, outbred Swiss Webster mice were immunized intramuscularly at weeks 0 and 2 with 1 or 10 µg protein/dose. Sera were collected at two week intervals, pooled for assay, and screened for antibody activity by ELISA to outer membrane complex (OMC), purified OMP (P1.16), VR1 peptide coupled to Bovine serum albumin (M21-BSA), VR2 peptide coupled to BSA (M20-BSA), wildtype flagellin, and to CRM₁₉₇. The results of the ELISA performed on sera obtained at 6 weeks are shown in Table 4.

**TABLE 4**

| Immunogenicity of recombinant or CRM197 conjugate vaccines containing the meningococcal P1.16 OMP epitopes VR1 and VR2. | | | | | | |
|---|---|---|---|---|---|---|
| DOSE µg | ELISA TITERS 4 WEEKS AFTER SECONDARY BOOST¹ | | | | | |
| | OMC | P1.16 | M21-BSA | M20-BSA | FLAGELLIN | CRM |
| pPx1650 (control wildtype flagellin)² | | | | | | |
| 1 | <150 | <100 | 171 | 100 | 427,781 | ND |
| 10 | <150 | 100 | 154 | <100 | 468,385 | ND |
| | | | | | | |

| pCB1-4 | | | | | | |
|---|---|---|---|---|---|---|
| 1 | 532 | 4,376 | 4,525 | ND | 787,120 | ND |
| 10 | 2,034 | 12,387 | 17,565 | ND | 887,861 | ND |
| | | | | | | |

| pCB2-W | | | | | | |
|---|---|---|---|---|---|---|
| 1 | 150 | 308 | ND | 501 | 263,143 | ND |
| 10 | 1,350 | 12,190 | ND | 5,476 | 1,493,216 | ND |
| | | | | | | |

| PCB12-10-6 | | | | | | |
|---|---|---|---|---|---|---|
| 1 | 615 | 3,374 | 4,651 | 824 | 299,889 | ND |
| 10 | 1,423 | 3,666 | 3,882 | 2,253 | 497,622 | ND |
| | | | | | | |

| pCB12-10-6 without aluminum phosphate | | | | | | |
|---|---|---|---|---|---|---|
| 1 | 409 | 739 | 505 | 597 | 139,147 | ND |
| 10 | 450 | 1,533 | 817 | 1,611 | 358,033 | ND |
| | | | | | | |

| M20-CRM197 | | | | | | |
|---|---|---|---|---|---|---|
| 1 | <150 | <100 | 217 | <100 | ND | 42.27 |
| 10 | 50 | <100 | 150 | <100 | ND | 95.31 |
| | | | | | | |

| M21-CRM197 | | | | | | |
|---|---|---|---|---|---|---|
| 1 | 68 | 249 | 10,494 | 100 | ND | 17.41 |
| 10 | 110 | 311 | 26,807 | 191 | ND | 20.92 |
| | | | | | | |

| MIXTURE OF M20 AND M21 CONJUGATES | | | | | | |
|---|---|---|---|---|---|---|
| 1 | 50 | 100 | 40,000 | 187 | ND | 37.32 |
| 10 | 50 | 227 | 15,539 | 132 | ND | 184.27 |
| | | | | | | |

| OMP P1.16 | | | | | | |
|---|---|---|---|---|---|---|
| 1 | 12,630 | 17,714 | 100 | 764 | ND | ND |
| 10 | 23,178 | 67,565 | 162 | 3,276 | ND | ND |
| | | | | | | |

| pCB1-4 in CFA | | | | | | |
|---|---|---|---|---|---|---|
| 10 | 1,665 | 10,606 | 19,945 | ND | 1,841,852 | ND |
| | | | | | | |

| pCB2-W in CFA | | | | | | |
|---|---|---|---|---|---|---|
| 10 | 1,157 | 6,869 | ND | 17,749 | 1,217,063 | ND |

| | | | | | | |
|---|---|---|---|---|---|---|
| ¹ All pre-bleed values at or below the lower limit of assay of 1/100 dilution. | | | | | | |
| ² All vaccines were formulated with 1 mg/ml aluminum phosphate except as noted. | | | | | | |

Alternatively, the various vaccines were evaluated for immunogenicity in 6-8 week old NIH outbred mice. The mice were immunized with 100 µg (total protein)/dose subcutaneously on week 0 and 4 with vaccine and sera was collected on week 6. The sera were evaluated in an ELISA assay and using antigens as described in Example 6. Bactericidal activity was measured as in Example 6. The results are found in Table 5.

**TABLE 5**

| ELISA (titer > 0.5 OD) | | | | |
|---|---|---|---|---|
| Vaccine | OMC | Class 1 OMP | Synth. Peptide | Bactericidal Test |
| FLAGELLIN | | | | |
| p1650 | - | - | - | <1:64 |
| pCB12.10.6 | - | 1:900 | - | <1:64 |
| pCB2-W | - | 1:300 | 1:100 | <1:64 |
| pCB1-4 | 1:300 (.25) | 1:2700 | - | <1:64 |
| CRM197 | - | - | - | <1:64 |
| M20-CRM197 | 1:00 | 1:8100 | - | <1:64 |
| M21-CRM197 | 1:300 (.125) | 1:8100 | - | <1:64 |

The recombinant flagellins containing either a VR1, VR2 or a cassette of both VR1 and VR2 were effective in eliciting an antibody response which was cross-reactive to the purified P1.16 and to a lesser extent to OMC. Sera from animals immunized with 10 µg of either pCB1-4 or pCB2-w induced antibodies which bound to their respective peptide--BSA conjugates as well as cross reacted with the P1.16 and OMC. Similar results were obtained with the constructed pCB12-10-6 which contains both meningococcal epitopes. In addition, each construction induced significant anti-flagellin titers as well. In contrast, the control wildtype flagellin only induced an antibody response to flagellin itself. Sera collected prior to immunization showed no pre-existing response to the materials being evaluated.

The data also demonstrates the benefits of formulating the recombinant flagellins with alum or other adjuvants such as CFA. The construction pCB12-10-6 was formulated with and without the addition of aluminum phosphate. As shown in table 2, pCB12-10-6 alone was capable of inducing an antibody response which react to the peptide conjugates as well as to the purified P1.16 as well as to OMC. In comparison, the same construction when formulated with alum was able to elicit greater antibody response at an equivalent dose. Similarly, the recombinant flagellins pCB1-4 and pCB2-w were also formulated with CFA. Again, equivalent or higher antibody titers were observed in the presence of CFA.

The results of the immunogenicity studies with the meningococcal VR1 and VR2 conjugates are also shown in Table 4. Both the M20 and the M21-CRM₁₉₇ conjugates as well as a mixture containing equal amounts of both conjugates were capable of inducing an anti-CRM₁₉₇ response as well as an anti-Class I OMP response.

These preliminary data indicate a Class I OMP variable region epitopes either chemically conjugated to a carrier or genetically fused to a carrier elicit an immune response. New epitope-carrier conjugates can be made using standard techniques to enhance the immune response to the vaccine, for example, use of 1) larger epitopes, 2) peptides with multiple epitope repeats and/or 3) different carriers.

### EXAMPLE 14: Preparation of Meningococcal-human serum albumin glycoconjugate

GCM CPS was depolymerized by acid hydrolysis and GCM OS obtained were subsequently activated via NalO₄ oxidation in aqueous buffer. The reactions were monitored by HPGPC in aqueous eluent, using UV and RI detection. The reactions were each followed by GPC desalting in water. GCM OS and human albumin (HA) were mixed and conjugated essentially as described in Example 10 for the meningococcal-flagellin glycoconjugate. The final preparation was stored in the cold, in the presence of thimerosal to prevent bacterial growth.

In preparation of the conjugate, the following experimental conditions were employed. Human albumin (HA; Sigma^{R}, St. Louis, MO) was dissolved in 15% sucrose (10 mg/ml) and then stored at -20°C. GCM CPS (lot # 86 NM 01; 106 mg; final concentration: 10 mg/ml was hydrolyzed in 0.1 N HCI at 50°C with agitation. Aliquots (25 µl) were withdrawn at regular intervals, the reaction stopped by addition of sodium hydroxide (NaOH) and analysis was performed by HPGPC as described. After 3 h 40 min., the reaction was stopped by addition of NaOH, and the GCM OS were desalted by GPC. Fractions were collected (1.2 ml) and analyzed as described before. Positive fractions were pooled and lyophilized. Desalted GCM OS (89 mg) were then stored at -20°C. Activated OS were prepared by oxidation of GCM OS (11.8 mg; final concentration: 5mg/ml) with 2 mM NalO₄ in 0.05 M sodium phosphate buffer (pH 6.2-6.5) at RT, in the dark, with agitation. The reaction was stopped after 30 min by addition of ethylene glycol. HPGPC analyzes showed no degradation of the molecular weight of the OS during activation. Desalting and colorimetric anaylzes were then performed as described above. The resulting activated GCM OS (8.8 mg) were dissolved in water (10 mg/ml) and frozen at -20°C.

Both GCM OS and HA solutions were analyzed by HPGPC (UV at 206 and 280 nm respectively) before being frozen, and prior to the conjugation. No degradation occurred during storage, as ascertained by the exact similarity of the elution profiles.

GCM OS (6 mg; final concentration: 2.5 mg/ml) and HA (12 mg; final concentration: 5mg/ml) were mixed in a polypropylene tube in 0.4 M sodium phosphate buffer (pH 7.0), and NaBH₃CN was added (60 µmoles) to initiate the conjugation (Anderson, U.S. Patent 4,762,713, 1988; U.S. Patent 4,673,574, 1987; U.S. Patent 4,761,283, 1988). The reaction mixture was left one day at RT, then 4 days 15 35°C, without agitation. The reaction was monitored by HPGPC (UV at 280 nm) at different stages, and finally stopped by diaiysis/concentration on microconcentrators. The final preparation was analyzed for NANA (Barry et al., J. Gen. Microbiol. 29P335-51, 1962) (2.07 mg at 0.86 mg/ml) and protein (Lowry et al., J. Biol. Chem. 193265-75, 1951) (9.51 mg at 3.96 mg/ml) content. It was then stored at 4°C in the presence of thimerosal (0.01%, w/v) to prevent bacterial growth.

The conjugate preparation was also checked by SDS-PAGE (silver nitrate stain) and Western blot analyzes. A diffuse band appeared on the gel which covered a significantly wider molecular weight range than the pure HA. Western blot analyzes showed that this band was reactive with the antiserum used (anti-GCM), proving covalency of the conjugate bonds.

### EXAMPLE 15: Immunogenicity of meningococcal oligosaccharide-recombinant flagellin vaccines

A meningococcal oligosaccharide-recombinant flagellin vaccine was prepared as described above and formulated at 100 µg protein/ml. Vaccine compositions were also prepared which contained aluminum phosphate (1 mg/ml) or complete Freund's adjuvant in addition to the glycoconjugate.

To evaluate the immunogenicity, outbred Swiss Webster mice were immunized intramuscularly with 10 µg protein at week 0 and 2. Sera were collected at weeks 0, 2 and then weekly intervals thereafter to 6 weeks. After collection, pooled sera samples were assayed for antibody activity by ELISA to meningococcal C oligosaccharide conjugate to human serum albumin, OMC, P1.16, CB1 and CB2-BSA conjugates and flagellin.

The MenC-CB12-10-6 glycoconjugate was effective at eliciting an immune response which was reactive with both the oligosaccharide and the meningococcal B OMP epitopes expressed in the recombinant flagellin. As shown in Table 5B, as little as three weeks into the study, mice immunized with 1 µg of MenC-CB12-10-6 conjugate in complete Freund's adjuvant had detectable antibody to MenC-HSA, OMP and to both the CB1 and CB2 epitopes. Further, all of the MenC-CB12-10-6 preparations, regardless of adjuvant, elicited antibody response to MenC-HSA which were greater than the response observed following immunization with MenC-CRM197.

**Table 5B**

| Immunogenicity of Meningococcal C - recombinant flagellin vaccine one week after secondary immunization. | | | | | | | |
|---|---|---|---|---|---|---|---|
| IMMUNOGEN | | Dose | ELISA TITERS¹ | | | | |
| | | | MenC-HSA | OMP | CB1-BSA | CB2-BSA | FLAGELLIN |
| MenC-CB12-10-6 | CFA | 10 | 24,530 | 608 | 5,240 | 432 | 541,467 |
| | | 1 | 5,069 | 5,614 | 5,375 | 12,685 | 526,593 |
| | alum² | 10 | 11,845 | 253 | 835 | 673 | 472,766 |
| | | 1 | 4,415 | 136 | 242 | 244 | 214,263 |
| | None | 10 | 11,497 | 920 | 626 | 2,382 | 233,307 |
| | | 1 | 4,920 | 483 | 1,123 | 1,210 | 135,625 |
| MenC-CRM197 | alum | 10 | 4,905 | ND | ND | ND | ND |
| | | 1 | 8,505 | ND | ND | ND | ND |
| OMP (P1.16) | alum | 10 | ND | 12,907 | <100 | <100 | ND |
| | | 1 | ND | 10,405 | <100 | 3,377 | ND |

| | | | | | | | |
|---|---|---|---|---|---|---|---|
| 1 Titers for initial prebleed samples (week 0) samples were <100. | | | | | | | |
| 2 Aluminum phosphate was used as adjuvant at 1 mg/ml. | | | | | | | |

### EXAMPLE 16: T-cell epitopes of Class I OMP and their identification

An effective vaccine must contain one or more T-cell epitopes. T-cell epitopes within a protein can be predicted as described by Margalit et al., J. Immunol. 138:2213, (1987) or Rothbard and Taylor, EMBO J. 7:93, (1988). These predictive methods were applied to the amino acid sequence of the Class I OMP of N. meningitidis strains P1.7,16, P1.16 and P1.15. The segments of the sequence containing potential T cell epitopes identified by these methods are shown in Tables 6 and 7. The predicted peptides were synthesized by standard FMOC procedures, purified by standard methods and were identified as shown in Table 8.

To determine which of the predicted peptides actual contain T cell epitopes, their capacity to stimulate human peripheral blood lymphocytes (PBL) was tested by lymphocyte proliferative assay. Briefly, peripheral blood was collected from HLA typed normal volunteers or from volunteers who were previously immunized with MPC-2 (Poolman, J.T. et al., Antonie van Leeuwenhoek, 53:413-419, 1987) which contained P1.16, 15, Class 4 OMP and Group C polysaccharide. Lymphocytes were isolated from the peripheral blood by isolation on Ficoll-Hypaque (Pharmacia Fine Chemicals AB, Uppsala, Sweden) and cultured at 1 X 10⁵ cells/well in supplemented RPMI 1640 (Gibco Laboratories, Paisly, Scotland) containing 10% heat-inactivated pooled human AB serum. Cultures were challenged with various concentrations of the predicted T cell epitopes (0.05 - 10 µg/ml). After in vitro challenge, the cultures were incubated for six days and then pulsed (18 hours) with 0.5 µCi of [³H]-thymidine. Cultures were then harvested and counted by liquid scintillation. Data are expressed as stimulation indices which were calculated as a ratio of the CPM obtained in the presence of antigen to the CPM obtained in the absence of antigen.

As shown in Table 9, 10 of the 16 predicted peptides showed some capacity to stimulate T-cells. These include the peptides identified at 16-34, 47-59, 78-90, 103-121, 124-137, 151-158, 176-185, 223-245, 276-291 and 304-322. In some instance, peptides stimulated a response in both immunized as well as non-immune individuals. The response in the non-immune individuals may be attributed to a previous asymptomatic infection.

In the case of the T cell epitope identified as region 176-185, enhancement of the T cell response was observed following addition of the monoclonal antibody MN5C11G (P1.16). Briefly, PBL were challenged in vitro with a synthetic peptide containing the region 175-185 or with this peptide mixed with varying dilutions of MN5C11G. As shown in Table 10, enhancement of the T cell response was observed following addition of MN5C11G indicating that monoclonal antibody recognized a B cell epitope within the region 176-185 and facilitates the presentation of the peptide to the immune system. Thus, it was established that the T and B cell epitopes either coincide or are found on contiguous sequences within the Class I OMP.

In several cases, T cell lines and clones were established from individuals responding to various peptides. Briefly, T cell lines were obtained by culturing isolated lymphocytes in 24 well plates at 1 x 10⁶ cells/ml. The culture medium, supplemented RPMI-1640 with 10% human serum, also contained 12 U/ml recombinant IL-2 (Boehringer). In addition, 5 x 10⁴ homologous, irradiated (3,000R) antigen presenting cells (APC) were also added to each well. In some cases, APC were obtained from HLA compatible donors. From the lines, T cell clones were isolated by limiting dilution at a frequency of 0.5 cells/well. Clones were maintained by bi-weekly stimulation with antigen in the presence of irradiated APC and IL-2 (2 U/ml). Clones were tested by lymphocyte proliferation assay essentially as described above except that clones were cultured at 1 x 10⁴ cells/well in the presence of irradiated APC.

Clones obtained as described were challenged in vitro with OMP from 7 different strains of meningococci. As shown in Table 11, the clones recognized a T cell epitope or epitopes common to the seven OMPs examined. Although the reactivity of these clones to the various peptides remains to be determined, the data, nevertheless, does indicate the commonality of T cell epitopes among the various strains. Now that these clones have been established and identified their peptide reactivity will indicate T-cell epitopes for vaccine use.

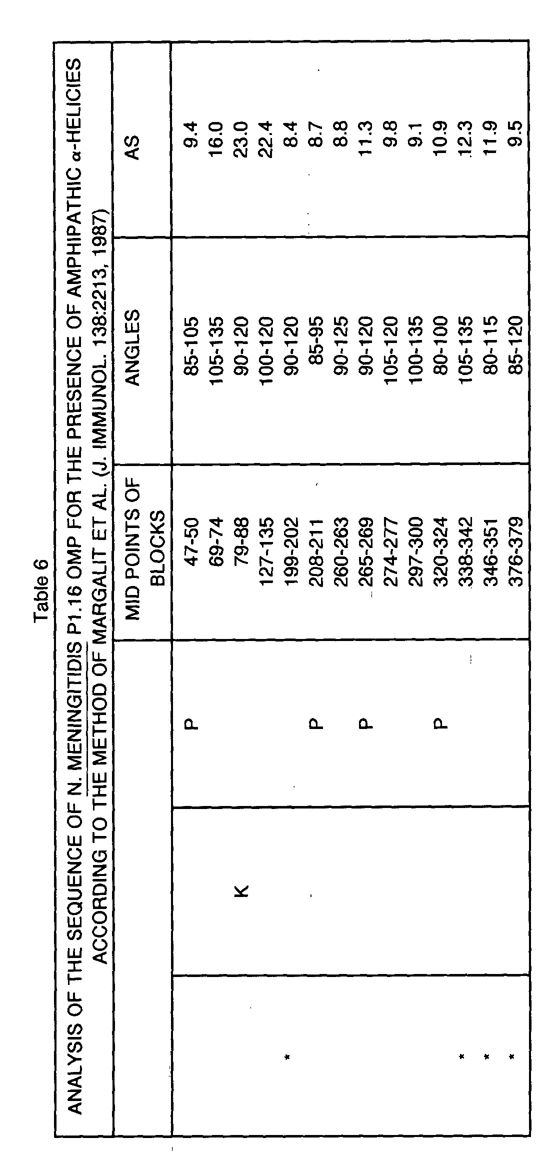

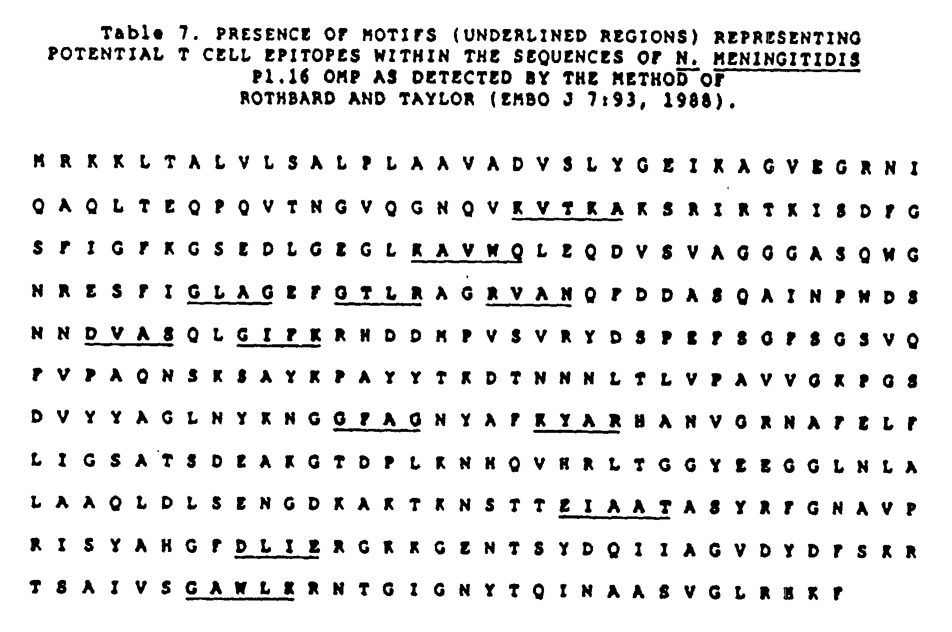

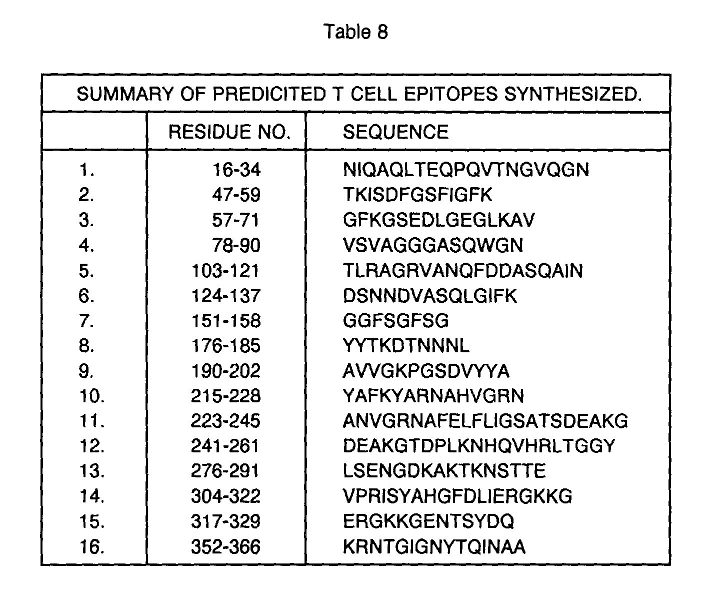

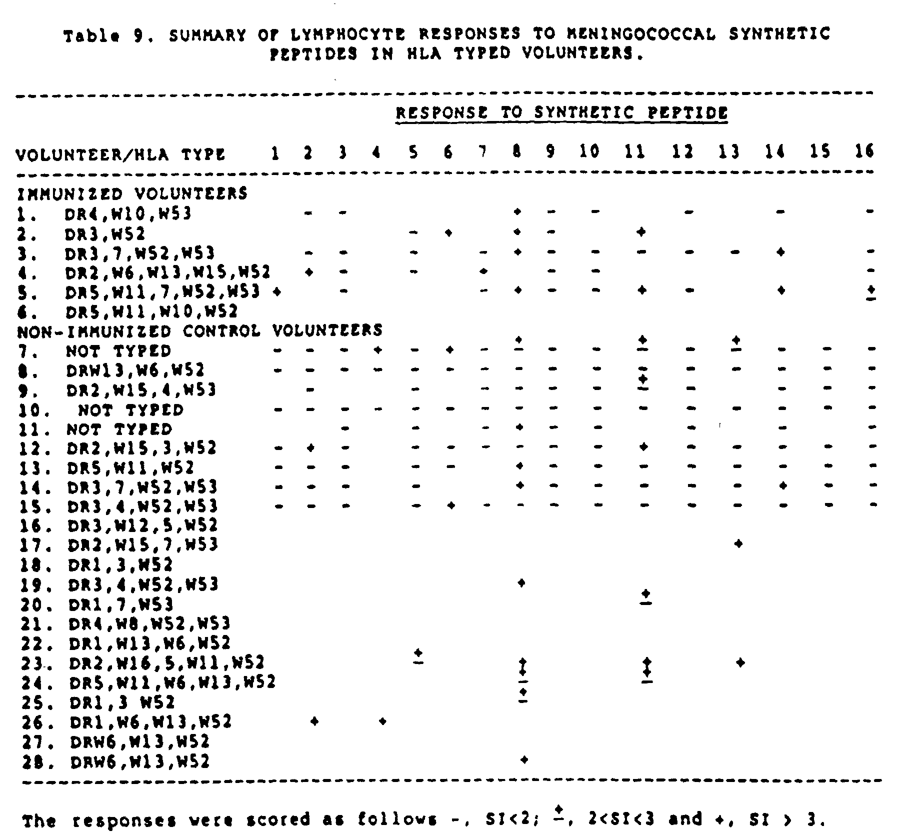

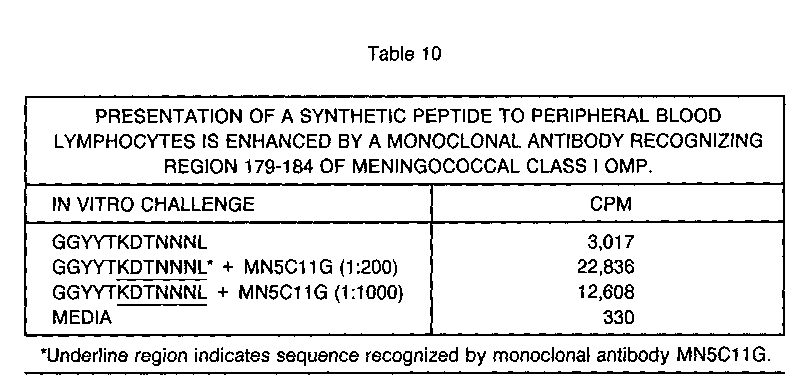

**Table 11**

| RECOGNITION OF OMP FROM DIFFERENT MENINGOCOCCAL STRAINS BY HUMAN T-CELL CLONES | | | | | | | | |
|---|---|---|---|---|---|---|---|---|
| STRAIN | SUBTYPE | RESPONSE OF HUMAN T CELL CLONES (CPM X 10⁻³) | | | | | | |
| | | 5-5 | 5-7 | 5-9 | 5-12 | 5-13 | 5-14 | 5-15 |
| H44-76 | P1.16 | 6.0 | 1.2 | 6.8 | 2.6 | 2.3 | 9.5 | 1.5 |
| SWISS 4 | P1.15 | 4.9 | 1.0 | 10.1 | 6.9 | 3.6 | 10.5 | 1.4 |
| 395 | P1.9 | 5.2 | 1.5 | 4.8 | 1.5 | 6.1 | 13.1 | 1.4 |
| 2996 | P1.2 | 5.4 | 1.0 | 3.7 | 2.3 | 3.4 | 11.8 | 1.0 |
| M990 | P1.6 | 3.6 | 0.4 | 3.5 | 2.5 | 0.9 | 4.7 | 0.6 |
| 187 | P1.1 | 4.4 | 0.7 | 4.5 | 3.1 | 1.6 | 6.2 | 1.4 |
| 6557 | P1.17 | 3.7 | 2.0 | 8.2 | 4.2 | 1.7 | 6.2 | 0.8 |
| MEDIA | -- | <0.1 | <0.1 | <0.1 | <0.1 | <0.1 | <0.1 | <0.1 |

### EXAMPLE 17: Construction of Protein Model for Membrane Topology of Class I OMP and Comparison to Other Pathogenic Gram Negative Porin Proteins for Vaccine Development.

A model was constructed using the principles recognized for the structure of several Escherichia coli outer membrane Proteins (Vogel, H. et al. (1986) supra Ferenci, T. et al. (1988) supra; and Tommassen, J. (1988) supra). The central assumption is that protein segments spanning the outer membrane form beta-sheets. Specifically, in the case of Class 1 protein, the division in exposed and transmembrane segments was arrived at in the following way:
1. A comparison of the amino acid sequence of Class 1 protein (subtype P1.16) with those of the gonococcal PIA and PIB proteins (Carbonetti, N.H. et al. (1987) PNAS 84:9084; Carbonetti, N.H. et al. - (1988) PNAS 85:6841; and Gotschlich, E.C. et al. (1987) PNAS 84:8135) reveals 34% identity. In the model, the variable sequences form the surface-exposed parts, whereas the conserved regions are placed mostly in the outer membrane and periplasm. Thus, the latter two areas consist for 58% of residues that are conserved among all proteins,
2. The hydrophilic maxima observed in a hydropathy profile (Kyte, J. et al. (1982) J. Mol. Biol. 157:105) to correspond to exposed regions.
3. The transmembrane segments should preferentially be able to form amphipathic beta-stands of 9-12 residues, with at least one side consisting entirely of hydrophobic residues. These conditions are met in 12 of the 16 membrane-spanning segments.
4. The number of residues at the periplasmic side is minimized.

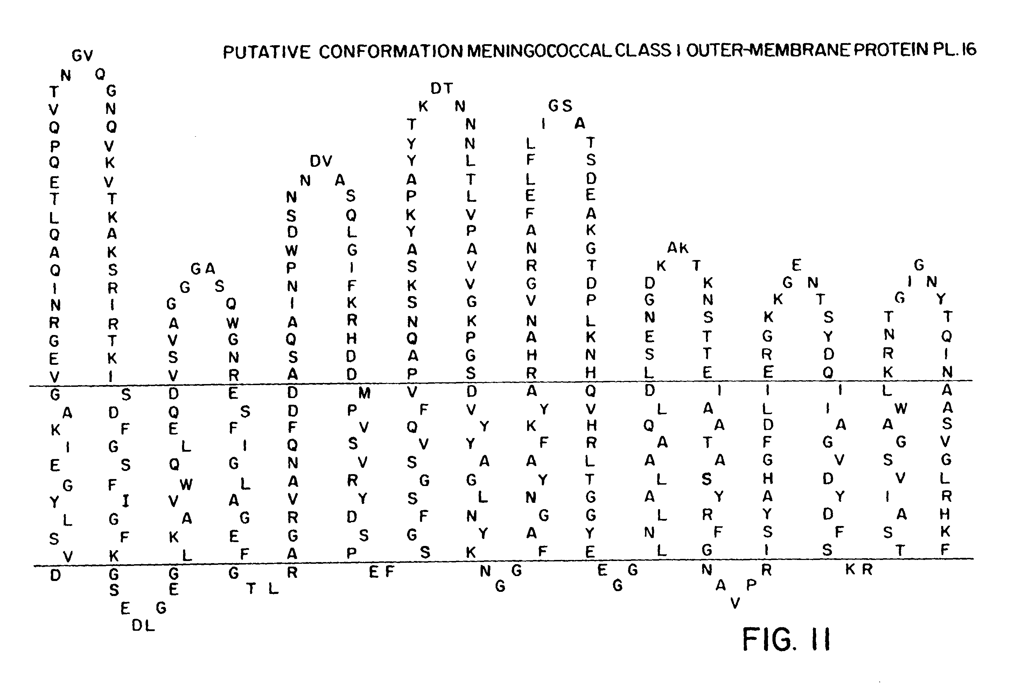

Figure 11 shows the model for the folding of Calss 1 protein in the outer membrane. The sequence shown is for subtype P1.16. The top part of the figure shows the surface-exposed regions, whereas the central part indicates the presumed transmembrane segments, whose length is set at ten. Amino acid are shown alternating where they can form an amphipathic beta-strand. This model contains eight surface loops, whereby the first and the fourth loop contain the type-specific and protective variable region epitopes. These epitopes, as has been shown when formulated into a vaccine, can elicit a protective immune response. Loop 5 is constant and has been shown to elicit cross-reactive antibodies to other OMPs and is useful for vaccine formulation.

The one or two variable epitope regions of the individual proteins are located on so called surface loops of these membrane proteins. Such porin outer-membrane proteins contain more than two surface loops. This implicates that there are surface loops which have near identical amino acid sequence in the different Class 1 outer-membrane proteins as well. This opens the way to use of common peptides of the Class 1 outer-membrane protein for vaccine objectives as well. More especially a schematic two-dimensional model of the meningococci Class 1 outer-membrane protein P1,16 is illustrated in Figure 11. This model contains eight surface loops, whereby the first and the fourth loop contain the type specific epitopes as shown on the basis of strain subtyping results. The fifth surface loop represents the constant region described above. Antibody to the constant region of loop 5 appears to react with N. meningitidis OMP complex. The amino sequence of Class 1 OMP, as derived, was compared to the Class OMP of N. meningitidis (Murakami, K. et al., (1989), Infect. Immun. 57:2318) and the porin PIA and PIB proteins of N. gonorrhoeae. With similar principle as used for the Class I OMP modeling, the sequences were aligned as follows:

LOOP 1

Class I     DVSLYGEIKAG | VEGRNIQAQLTEQPQVTNGVQGN
            ** *** **** | *
Class IB    DVTLYGAIKAG | V-------------QTYRSVEHT
            ** *** **** | *
Class IA    DVTLYGTIKAG | VEGRNIQAQLTEQPETSRSVAHH
            ** *** **** | *

Class II    DVTLYGTIKAGV EGRNIQAQLTEQPEVSRVKDAG
            ** *** *****

QVKVTKAKSRIRTKI | SDFGSTIGFKGSEDLGEGL
              * |   * ** ***** **** **
DGKVSKVET--GSEI | ADFGSKIGFKGQEDLGNGL
              * |   ** ***** ** * **
GAQADRVKT--ATEI | ADLGSKIGFKGQEDLGNGL
              * |   * ** ***** ** * **

TYKAQGGKSKTATQ IADFGSKIGFKGQEDLGNGL
               * * ** ***** ** * **

LOOP 2

KAVWQLEQD | VSVAGGGASQWGN | RESFIGLAGEFG
** ***** |     *     *** | ** ** * **
KAVWQLEQG | ASVAGTNTG-WGN | KQSFVGLKGGFG
** ***** |     *     *** | ** ** * **
KAIWQLEQK | AYVSGTDTG-WGN | RQSFIGLKGGFG
** ***** |     *     *** |  ** ** * **

KAIWQLEQ KASIAGTNSG-WGNRQSFIGLKGGFG
** *****      *     ***  ** ** * **

LOOP 3

TLRAGRVANQFDD | ASQAINPWDSNNDVASQLQI-
 * *
TIRAGSLNSPLKN | TGANVNAWESGKFTGNVLEIS
 * *
KVRVGRLNSYLKD | TGGFNPWEGKSYYLPLSNIAQ
 * *

TVRAGNLNTVLKDSGDNVNAWESGSNTEDVLGLG
 * *

LOOP 4

-FKRHDD MPV SVRYDSPEFSGFSGSVQFV PAQNS
*******    **     *    *  **
GMAQREH RYL SVRYDSPEFAGFSGSVQYA PKDNS
*******    **     *    *  **
PEERHV-- -- SVRYDSPEFAGFRAV-QYV PNDNS
*******    **     *    *  **

TIGRVESREI SVRYDSPVFAGFSGSVQ YVPRDNA
*******    **     *    *  **

KSAYKPAYYTKDTNNNLTLVPAVVGKPGS DVYYA
*
GS---------------------------NG ESYHV
*
GK---------------------------NHS ESYHA
*

ND----------------VDKYKHTKSSR ESYHA
*

LOOP 5

GLNYKNGGFAGNYAFKYA RHANVGRNAFELFLIG
* ** * * *   **
GLNYQNSGFFAQYAGLFQ RYGEGTKK------IE
* ** * * *   **
GFNYKNSGFFVQYAGFYK RHSYTTEKHFELFL--
* ** * * *   **

GLKYENAGFFGQYA GSFAKYADLNTD------AE
* ** * * *   **

N
SATSDEAKGTDPLKH QVHRLTGGYEEGGLNLALA
***** ***     *   *
DDQTYSIPSLFVEKL QVHRLVGGYDNNALYVSVA
***** ***     *   *
--------------L QVHRLVGGYDHDALYASVA
***** ***     *   *

RVAVNTANAHPVKDY QVHRVVAGYDANDLYVSVA
***** ***     *   *

LOOP 6
AQLD LSE--NGDKAKTKNSTTE IAATASYRFGNA
*                    *   **** *****
AQQQ DAKLYGAMSGNSHNSQTE VAATAAYRFGNV
*                    *   **** *****
VQQQ DAKLTWRND-NSHNSQTE VAATAAYRFGNV
*                 *   *  **** ****
GQ YEAAKNNEVGSTKGKKHQTQ VAATAAYRFGNV
*                    E *   **** *****
LOOP 7
VPRISYAHGFDLI ERGKKGENTSYDQ IIAGVDYD
** ******                        **
TPRVSYAHGFKGT VDSANHDNT-YDQ VVVGAEYD
** ******          *  *  *       **
TPRVSYAHGFKGS VYDADNDNT-YDQ VVVGAEYD
** ******          *  *  *       **
TPRVSYAHGF KAKVNGVKDANYQQDQ VIVGADYD
** ******              *  *    *  **
LOOP 8
FSKRTSAIVSGAWL KRNTGIGNYTQIN AASVGLRHKF
******* **  **    *              * *****
FSKRTSALVSAGWL QGGKGADKIV-ST ASAVVLRHKF
******* **  **    *              * *****
FSKRTSALVSAGWL QRGKGTEKFV-AT VGGVGLRHKF
******* **  **    *             * *****
FSKRTSALVSAGWL KQGKGTGKVEQ-TASM VGLRHKF
******* **  **    *                * *****

Structural similarities are indicated with transmembrane and surface loop regions. With the information now available for Class I OMP and information based on surface loop size, location, intraspecies amino acid homology or heterology of the loop regions of the particular porin protein, predictions of epitopes for incorporation into vaccines for other pathogenic gram negative bacteria including N. gonorrhoeae are possible. Using the same methods employed for Class I OMP, these epitopes can be evaluated for vaccine purposes.

### Deposit of Microorganisms

The N. meningitidis strain H4476 (B:15:P1.7,16) was deposited on December 11, 1989 in the Centraal Bureau voor Schimmelculturen (CBS), Baarn, The Netherlands and has deposit number CBS 635.89. The N. meningitidis Class 2/3 OMP deficient mutant HIII-5 was deposited on December 11, 1989 in the CBS, Baarn, The Netherlands and has deposit number CBS 636.89.

### Equivalents

Those skilled in the art will recognize, or be able to ascertain using no more than routine experimentation, many equivalents to the specific embodiments of the invention described herein. Such equivalents are intended to be encompassed by the following claims.

## Claims (Claims for the following Contracting State(s): AT, BE, CH, LI, DE, FR, GB, IT, LU, NL, SE)

1. Vaccine effective against meningococcal disease, comprising an effective amount of an outer-membrane vesicle isolated from genetically engineered or mutant meningococcal strain, the vesicle comprising: at least one meningococcal Class 1 outer-membrane protein, fragment thereof or oligopeptide containing an epitope thereof, wherein said protein, fragment thereof or oligopeptide is capable of eliciting a bactericidal immune response in a host; and not comprising a meningococcal Class 2/3 outer-membrane protein; the meningococcal Class 1 outer-membrane protein originating from a mutant meningococcal strain which is negative for Class 2/3 outer-membrane protein.

2. A vaccine of claim 1, wherein the meningococcal Class 1 outer-membrane protein is derived from a group A, B, C, W-135 or Y meningococcus.

3. A vaccine of claim 1, wherein the fragment of Class 1 meningococcal outer-membrane protein is obtained by a) cyanogen bromide treatment of a Class 1 outer-membrane protein, or b) proteolysis with an enzyme selected from the group consisting of endoLys-C, endo-Arg-C, endoGlu-C and Staphylococcus V8-protease.

4. A vaccine of claim 1, wherein the oligopeptide comprises a) at least one bactericidal antibody binding epitope of meningococcal Class 1 outer-membrane proteins, or b) at least an amino acid sequence selected from the group consisting of QPQVTNGVQGN, PPSKSQP, QAANGGASG, YYTKDTNNNLTL, YYTKNTNNNLTL, YYTKDTNNNL, YYTKNTNNNL, HFVQQTPQSQP and HYTRQNNTDVF.

5. A vaccine of claim 1, wherein the epitope is located in surface loops of meningococcal Class 1 outer-membrane proteins in the area of amino acids 24-34 and 176-187.

6. Substantially purified fragment of Class 1 outer-membrane protein of Neisseria meningitidis, the fragment having a molecular weight of about 25 kD or less and containing continuous or discontinuous epitopes reactive with bactericidal antibodies against N. meningitidis, wherein the epitopes are located in surface loops of meningococcal Class 1 outer-membrane proteins in the area of amino acids 24-34 and 176-187.

7. A fragment of claim 6, wherein the Class 1 outer-membrane protein is of the subtype P1.7.16.

8. A substantially purified oligopeptide of claim 6, containing a B cell epitope of a Class 1 outer-membrane protein which varies in amino acid sequence among different meningococcal porin proteins.

9. Oligopeptide of claim 8, containing at least one of the amino acid sequences selected from the group consisting of QPQVTNGVQGN, PPSKSQP, QAANGGASG, YYTKDTNNNLTL, YYTKNTNNNLTL, YYTKDTNNNL, YYTKNTNNNL, HFVQQTPQSQP and HYTRQNNTDVF.

10. A substantially purified oligopeptide of claim 6 containing a B cell or T cell epitope of a Class 1 outer-membrane protein which is conserved in amino acid sequence among different meningococcal porin proteins.

11. A substantially purified oligopeptide of claim 6 containing a T cell epitope of a meningococcal Class 1 outer-membrane protein.

12. Isolated nucleic acid encoding full length Class 1 OMP protein having one of the sequences shown below:

DVSLYGEIKAGVEGRNFQLQLTEPPSKSQPQVKVTKAKSRIRTKISDFGSFIGFKGSEDLG
EGLKAVWQLEQDVSVAGGGATQWGNRESFVGLAGEFGTLRAGRVANQFDDASQAIDP
WDSNNDVASQLGIFKRHDDMPVSVRYDSPDFSGFSGSVQFVPIQNSKSAYTPAHYTRQN
NTDVFVPAVVGKPGSDVYYAGLNYKNGGFAGSYAFKYARHANVGRDAFELFLLGSTSD
EAKGTDPLKNHQVHRLTGGYEEGGLNLALAAQLDLSENGDKAKTKNSTTEIAATASYR
FGNAVPRISYAHGFDLIERGKKGENTSYDQIIAGVDYDFSKRTSAIVSGAWLKRNTGIGN
YTQINAASVGLRHKF; or
DVSLYGEIKAGVEGRNYQLQLTEAQAANGGASGQVKVTKVTKAKSRIRTKISDFGSFIGF
KGSEDLGDGLKAVWQLEQDVSVAGGGATQWGNRESFIGLAGEFGTLRAGRVANQFDD

ASQAIDPWDSNNDVASQLGIFKRHDDMPVSVRYDSPEFSGFSGSVQFVPIQNSKSAYTPA
YYTKNTNNNLTLVPAVVGKPGSDVYYAGLNYKNGGFAGNYAFKYARHANVGRNAFEL
FLIGSGSDQAKGTDPLKNHQVHRLTGGYEEGGLNLALAAQLDLSENGDKTKNSTTEIAA
TASYRFGNAVPRISYAHGFDFIERGKKGENTSYDQIIAGVDYDFSKRTSAIVSGAWLKRN
TGIGNYTQINAASVGLRHKF.

13. Isolated oligonucleotide encoding an epitope located in surface loops of meningococcal Class 1 outer-membrane proteins in the area of amino acids 24-34 and 176-187.

14. A composition for eliciting a protective immune response against Neisseria meningitidis, comprising a) a liposome comprising one or more meningococcal Class 1 outer-membrane proteins, but not comprising a meningococcal Class 2/3 outer-membrane protein or fragment thereof; and, optionally, an adjuvant in a pharmaceutically acceptable vehicle, or b) a liposome comprising at least one oligopeptide containing a continuous or discontinuous epitope of a Class 1 outer-membrane protein reactive with bactericidal antibodies against N. meningitidis but not comprising an epitope of a Class 2/3 outer-membrane protein; and optionally, an adjuvant in a pharmaceutically acceptable vehicle; said Class 1 outer-membrane protein(s) having one of the two sequences shown below:

DVSLYGEIKAGVEGRNFQLQLTEPPSKSQPQVKVTKAKSRIRTKISDFGSFIGFKGSEDLG
EGLKAVWQLEQDVSVAGGGATQWGNRESFVGLAGEFGTLRAGRVANQFDDASQAIDP
WDSNNDVASQLGIFKRHDDMPVSVRYDSPDFSGFSGSVQFVPIQNSKSAYTPAHYTRQN
NTDVFVPAVVGKPGSDVYYAGLNYKNGGFAGSYAFKYARHANVGRDAFELFLLGSTSD
EAKGTDPLKNHQVHRLTGGYEEGGLNLALAAQLDLSENGDKAKTKNSTTEIAATASYR
FGNAVPRISYAHGFDLIERGKKGENTSYDQIIAGVDYDFSKRTSAIVSGAWLKRNTGIGN
YTQINAASVGLRHKF; or
DVSLYGEIKAGVEGRNYQLQLTEAQAANGGASGQVKVTKVTKAKSRIRTKISDFGSFIGF
KGSEDLGDGLKAVWQLEQDVSVAGGGATQWGNRESFIGLAGEFGTLRAGRVANQFDD
ASQAIDPWDSNNDVASQLGIFKRHDDMPVSVRYDSPEFSGFSGSVQFVPIQNSKSAYTPA
YYTKNTNNNLTLVPAVVGKPGSDVYYAGLNYKNGGFAGNYAFKYARHANVGRNAFEL
FLIGSGSDQAKGTDPLKNHQVHRLTGGYEEGGLNLALAAQLDLSENGDKTKNSTTEIAA
TASYRFGNAVPRISYAHGFDFIERGKKGENTSYDQIIAGVDYDFSKRTSAIVSGAWLKRN
TGIGNYTQINAASVGLRHKF.

15. An antigenic conjugate, comprising a carrier protein or an epitope thereof, to which is conjugated a fragment of a meningococcal Class 1 outer-membrane protein having a molecular weight of 25kd or less containing an epitope selected from the group consisting of: QPQVTNGVQGN, PPSKSQP, QAANGGASG, YYTKDTNNNLTL, YYTKNTNNNLTL, YYTKDTNNNL, YYTKNTNNNL, HFVQQTPQSQP and HYTRQNNTDVF, provided that the carrier protein is not β-galactosidase.

16. An antigenic conjugate of claim 15, wherein the antigen carrier protein is a bacterial toxin, CRM or epitope thereof.

17. A genetic fusion peptide or protein, comprising a carrier protein, peptide or epitope thereof to which is fused a fragment of a meningococcal Class 1 outer-membrane protein having a molecular weight of 25kd or less containing an epitope selected from the group consisting of: QPQVTNGVQGN, PPSKSQP, QAANGGASG, YYTKDTNNNLTL, YYTKNTNNNLTL, YYTKDTNNNL, YYTKNTNNNL, HFVQQTPQSQP and HYTRQNNTDVF, provided that the carrier protein is not β-galactosidase.

18. A fusion protein comprising a flagellin protein having an amino acid sequence for an epitope of a meningococcal Class 1 outer-membrane protein inserted within it.

19. The fusion protein of claim 18, wherein the amino acid sequence is inserted a) within the flagellin protein at a region which is non-essential to function of the flagellin protein, or b) is inserted into the hyper-variable region of the flagellin protein.

20. A fusion protein of claim 18, further comprising meningococcal capsular oligo- or polysaccharide conjugated thereto.

21. A recombinant gene encoding a fusion protein of claim 17.

22. Recombinant microorganism capable of expressing a fragment of a Class 1 outer-membrane protein of Neisseria meningitidis having a molecular weight of 25 kD or less which contains a continuous or discontinuous epitope reactive with Class 1 OMP specific antibodies against N. meningitidis.

23. A microorganism of claim 22, wherein the epitope is selected from the group consisting of QPQVTNGVQGN, PPSKSQP, QAANGGASG, YYTKDTNNNLTL, YYTKNTNNNLTL, YYTKDTNNNL, YYTKNTNNNL, HFVQQTPQSQP and HYTRQNNTDVF.

24. A microorganism of claim 22, which is a vaccinia virus, adenovirus, cytomegalovirus or a bacteria of the genus Salmonella.

25. A microorganism of claim 22, wherein the epitope of the meningococcal Class 1 outer-membrane protein is expressed as a recombinant flagellin.

26. A mutant meningococcal strain incapable of producing Class 2 and 3 outer-membrane protein.

27. The mutant meningococcal strain HIII5, CBS 636.89 which is incapable of producing Class 2 and Class 3 outer-membrane protein.

28. A genetically engineered Neisseria strain which is negative for Class 2/3 outer-membrane proteins, and which is capable of expressing an intact Class 1 outer-membrane protein of Neisseria meningitidis, genetic fusion protein thereof or a continuous or discontinuous epitope reactive with Class 1 OMP specific antibodies against N. meningitidis.

29. A genetically engineered microorganism consisting of a Neisseria strain capable of expressing more than one Class 1 outer-membrane protein of Neisseria meningitidis, each protein individually comprising at least one continuous or discontinuous epitope reactive with Class 1 OMP specific antibodies against N. meningitidis.

30. A vaccine according to claim 1 further comprising a plurality of Class 1 outer-membrane proteins.

31. A vaccine according to claim 1, further comprising a plurality of meningococcal Class 1 outer-membrane protein fragments or oligopeptides containing an epitope thereof.

32. A method of expressing at least one meningococcal Class 1 outer-membrane protein that is capable of eliciting a bactericidal immune response in a host, comprising inserting at least one gene encoding meningococcal Class 1 outer-membrane protein into a recombinant microorganism selected from a) a mutant meningococcal strain which is negative for Class 2/3 outer-membrane protein or b) a group A, B, C, W-135 or Y meningococcus; wherein the microorganism is capable of expressing said gene as intact protein that is capable of eliciting a bactericidal immune response in a host.

33. A method of claim 32 wherein the microorganism is a Neisseria strain.

34. A method of claim 33, wherein the meningococcal strain expresses more than one meningococcal Class 1 outer-membrane protein.

35. A genetically engineered Neisseria strain capable of expressing a non-native, intact Class 1 outer-membrane protein of Neisseria meningitidis, genetic fusion protein thereof or a continuous or discontinuous epitope reactive with Class 1 OMP specific antibodies against N. meningitidis.

## Claims (Claims for the following Contracting State(s): ES)

1. A method of expressing at least one meningococcal Class 1 outer-membrane protein that is capable of eliciting a bactericidal immune response in a host, comprising inserting at least one gene encoding meningococcal Class 1 outer-membrane protein into a recombinant microorganism selected from a) a mutant meningococcal strain which is negative for Class 2/3 outer-membrane protein or b) such a strain which is a group A, B, C, W-135 or Y meningococcus; wherein the microorganism is capable of expressing said gene as intact protein that is capable of eliciting a bacterial immune response in a host.

2. A method according to claim 1, wherein the microorganism is a Neisseria Strain.

3. A method according to claim 1, wherein the mutant meningococcal strain which is incapable of producing Class 2 and 3 outer-membrane protein is the mutant meningococcal strain HIII5, CBS 636.89.

4. A method according to claim 1, wherein the gene inserted encodes full length Class I outer-membrane protein.

5. A method according to claim 1, wherein the meningococcal strain expresses more than one meningococcal Class I outer-membrane protein.

6. A method for producing a fragment of a meningococcal Class I outer-membrane protein that is capable of eliciting a bactericidal immune response in a host, comprising
a) treating a Class I outer membrane protein with cyanogen bromide, or
b) proteolytically digesting a Class I outer membrane protein with an enzyme selected from the group consisting of endoLys-C, endo-Arg-C, endoGlu-C and Staphylococcus V8-protease, or
c) obtaining a DNA sequence which encodes the desired fragment and introducing same into an appropriate vector/host expression system.

7. A method according to claim 6, wherein the fragment obtained is a substantially purified fragment of a meningococcal Class I outer-membrane protein of Neisseria meningitidis, said fragment having a molecular weight of about 25 kD or less and containing continuous or discontinuous epitopes reactive with bactericidal antibodies against N. meningitidis, wherein the epitopes are located in surface loops of meningococcal Class I outer-membrane proteins in the area of amino acids 24-34 and 176-187.

8. A method according to claim 6, wherein the meningococcal Class I outer-membrane protein is of the subtype P1.7.16.

9. A method according to claim 6, wherein the fragment obtained contains at least one of the amino acid sequences selected from the group consisting of QPQVTNGVQGN, PPSKSQP, QAANGGASG, YYTKDTNNNLTL, YYTKNTNNNLTL, YYTKDTNNNL, YYTKNTNNNL, HFVQQTPQSQP and HYTRQNNTDVF.

10. A method according to claim 6, wherein the recombinant microorganism capable of expressing said fragment is a vaccinia virus, adenovirus, cytomegalovirus or a bacteria of the genus Salmonella.

11. A method according to claim 6, wherein the fragment obtained further contains a B cell epitope of a Class I outer-membrane protein which varies in amino acid sequence among different meningococcal porin proteins.

12. A method according to claim 6, wherein the fragment obtained further contains a B cell or a T cell epitope of a Class I outer-membrane protein which is conserved in the amino acid sequence among different meningococcal porin proteins.

13. A method according to claim 6, wherein the fragment obtained further contains a T cell epitope of a meningococcal Class I outer-membrane protein.

14. A method for producing an oligopeptide containing an epitope of a meningococcal Class I outer-membrane protein that is capable of eliciting a bactericidal immune response in a host, which comprises
a) proteolytically digesting
(i) a Class I outer membrane protein, or
(ii) a fragment deriving from the cleavage of a Class I outer membrane protein with cyanogen bromide, with an enzyme selected from the group consisting of endoLys-C, endo-Arg-C, endoGlu-C and Staphylococcus V8-protease; or alternatively,
b) its chemical synthesis by standard solid peptide synthesis, pepscan synthesis or standard liquid peptide synthesis; or alternatively,
c) obtaining a DNA sequence which encodes the desired oligopeptide sequence and introducing same into an appropriate vector/host expression system.

15. A method according to claim 14, wherein the oligopeptide comprises a) at least one bactericidal antibody binding epitope of meningococcal Class I outer-membrane proteins, or b) at least an amino acid sequence selected from the group consisting of QPQVTNGVQGN, PPSKSQP, QAANGGASG, YYTKDTNNNLTL, YYTKNTNNNLTL, YYTKDTNNNL, YYTKNTNNNL, HFVQQTPQSQP and HYTRQNNTDVF.

16. A method according to claim 14 wherein the epitope is located in surface loops of meningococcal Class I outer-membrane proteins in the area of amino acids 24-34 and 176-187.

17. A method according to any of claims 1, 6 or 14, wherein the Class I outer-membrane protein, the fragment thereof or the oligopeptide obtained is further mixed, conjugated or fused to carrier peptides or proteins, to other antigens of N. meningitidis or to antigens of other microorganisms by chemical or genetic coupling techniques.

18. A method according to claim 17, wherein the Class I outer-membrane protein, the fragment thereof or the oligopeptide obtained is conjugated to a carrier protein or epitope thereof, provided that the carrier protein is not β-galactosidase.

19. A method according to claim 18, wherein the carrier protein is a bacterial toxin, CRM or an epitope thereof.

20. A method according to claim 17, wherein the Class I outer-membrane protein, the fragment thereof or the oligopeptide obtained is (a) conjugated to a T cell epitope via chemical coupling or (b) contains a meningococcal A, B, C, W-135 and Y polysaccharide in conjugate form with the protein product.

21. A method according to claim 17, wherein the Class I outer-membrane protein, the fragment thereof or the oligopeptide obtained is fused to a carrier protein, peptide or epitope thereof, provided that the carrier protein is not β-galactosidase.

22. A method for producing a fusion protein comprising a flagellin protein having an amino acid sequence for an epitope of a meningococcal Class I outer-membrane protein inserted within it, which comprises expressing a recombinant gene contaning a properly inserted DNA sequence encoding an epitope of a meningococcal Class I outer-membrane protein within the gene coding for flagellin.

23. A method according to claim 22, wherein the fusion protein obtained contains the amino acid sequence for an epitope of a meningococcal Class I outer-membrane protein within the flagellin protein (a) at a region which is non-essential to function of the flagellin protein, or (b) into the hypervariable region of the flagellin protein.

24. A method according to claim 22, wherein the fusion protein obtained further contains a meningococcal capsular oligo- or polysaccharide conjugated thereto.

25. A process for producing a vaccine effective against meningococcal disease, which comprises the mixture of an effective amount of an outer-membrane vesicle isolated from genetically engineered or mutant meningococcal strain, the vesicle comprising: at least one meningococcal Class 1 outer-membrane protein, a fragment thereof or an oligopeptide containing an epitope thereof, wherein said protein, fragment thereof or oligopeptide is capable of eliciting a bactericidal immune response in a host, and not comprising a meningococcal Class 2/3 outer-membrane protein, with a suitable adjuvant; the meningococcal Class I outer-membrane protein originating from a mutant meningococcal strain which is negative for Class 2/3 outer-membrane protein.

26. A process according to claim 25 wherein the vaccine obtained comprises a plurality of Class I outer-membrane proteins.

27. A process according to claim 25 wherein the vaccine obtained comprises a plurality of meningococcal Class I outer-membrane proteins fragments or oligopeptides containing an epitope thereof.

## Patentansprüche (Patentansprüche für folgende(n) Vertragsstaat(en): AT, BE, CH, LI, DE, FR, GB, IT, LU, NL, SE)

1. Impfstoff gegen eine Erkrankung an Meningokokken, umfassend eine wirksame Menge einer Außenmembranvesikel, isoliert aus einem gentechnisch hergestellten oder mutanten Meningokokkenstamm, wobei die Vesikel umfaßt: mindestens 1 Klasse 1 Außenmembranprotein, Fragment davon oder Oligopeptid, das ein Epitop davon enthält, von Meningokokken, wobei das Protein, Fragment davon oder Oligopeptid in der Lage ist, eine bakterizide Immunantwort in einem Wirt auszulösen und kein Klasse 2/3 Außenmembranprotein von Meningokokken umfaßt, und wobei das Außenmembranprotein der Meningokokken der Klasse I von einem mutanten Meningokokkenstamm herrührt, der sich hinsichtlich einem Außenmembranprotein der Klasse 2/3 negativ verhält.

2. Impfstoff nach Anspruch 1, wobei das Klasse I Außenmembranprotein der Meningokokken abgeleitet ist von einem Meningococcus der Gruppe A, B, C, W-135 oder Y.

3. Impfstoff nach Anspruch 1, wobei das von Meningokokken stammende Klasse I Außenmembranproteinfragment erhalten wird durch a) Bromcyanbehandlung eines Außenmembranproteins der Klasse I oder b) Proteolyse mit einem Enzym, ausgewählt aus der Gruppe, bestehend aus endoLys-C, endo-Arg-C, endoGlu-C und Staphylococcus-V8-Protease.

4. Impfstoff nach Anspruch 1, wobei das Oligopeptid umfaßt a) mindestens einen bakteriziden Antikörper, der an ein Epitop von Klasse I Außenmembranproteinen von Meningokokken bindet oder b) mindestens eine Aminosäuresequenz, ausgewählt aus der Gruppe, bestehend aus QPQVTNGVQGN, PPSKSQP, QAANGGASG, YYTKDTNNNLTL, YYTKNTNNNLTL, YYTKDTNNNL, YYTKNTNNNL, HFVQQTPQSQP und HYTRQNNTDVF.

5. Impfstoff nach Anspruch 1, wobei das Epitop in den Oberflächenschleifen von Klasse I Außenmembranproteinen von Meningokokken im Bereich der Aminosäuren 24 bis 34 und 176 bis 187 angeordnet ist.

6. Im wesentlichen gereinigtes Fragment von Klasse I Außenmembranprotein von Neisseria meningitidis, wobei das Fragment ein Molekulargewicht von etwa 25 kDa oder weniger aufweist und kontinuierliche oder diskontinuierliche Epitope enthält, die mit bakteriziden Antikörpern gegen N. meningitidis reaktiv sind, wobei die Epitope in den Oberflächenschleifen von Klasse I Außenmembranproteinen von Meningokokken im Bereich der Aminosäuren 24 bis 34 und 176 bis 187 angeordnet sind.

7. Fragment nach Anspruch 6, wobei das Außenmembranprotein der Klasse I vom Subtyp P1.7.16. ist.

8. Im wesentlichen gereinigtes Oligopeptid nach Anspruch 6, enthaltend ein B-Zellenepitop eines Außenmembranproteins der Klasse I, das in der Aminosäuresequenz unter verschiedenen Porinproteinen von Meningokokken variiert.

9. Oligopeptid nach Anspruch 8, enthaltend mindestens eine der Aminosäuresequenzen, ausgewählt aus der Gruppe, bestehend aus QPQVTNGVQGN, PPSKSQP, QAANGGASG, YYTKDTNNNLTL, YYTKNTNNNLTL, YYTKDTNNNL, YYTKNTNNNL, HFVQQTPQSQP und HYTRQNNTDVF.

10. Im wesentlichen gereinigtes Oligopeptid nach Anspruch 6, enthaltend ein B-Zellen- oder T-Zellenepitop eines Außenmembranproteins der Klasse I, das in der Aminosäuresequenz unter verschiedenen Porinproteinen von Meningokokken konserviert ist.

11. Im wesentlichen gereinigtes Oligopeptid nach Anspruch 6, enthaltend ein T-Zellenepitop eines Klasse I Außenmembranproteins von Meningokokken.

12. Isolierte Nucleinsäure, die für ein OMP-Protein der Klasse I in voller Länge kodiert, wie im folgenden gezeigt:

DVSLYGEIKAGVEGRNFQLQLTEPPSKSQPQVKVTKAKSRIRTKISDFGSFIGFKGSEDLG
EGLKAVWQLEQDVSVAGGGATQWGNRESFVGLAGEFGTLRAGRVANQFDDASQAIDP
WDSNNDVASQLGIFKRHDDMPVSVRYDSPDFSGFSGSVQFVPIQNSKSAYTPAHYTRQN
NTDVFVPAVVGKPGSDVYYAGLNYKNGGFAGSYAFKYARHANVGRDAFELFLLGSTSD
EAKGTDPLKNHQVHRLTGGYEEGGLNLALAAQLDLSENGDKAKTKNSTTEIAATASYR
FGNAVPRISYAHGFDLIERGKKGENTSYDQIIAGVDYDFSKRTSAIVSGAWLKRNTGIGN
YTQINAASVGLRHKF;    oder
DVSLYGEIKAGVEGRNYQLQLTEAQAANGGASGQVKVTKVTKAKSRIRTKISDFGSFIGF
KGSEDLGDGLKAVWQLEQDVSVAGGGATQWGNRESFIGLAGEFGTLRAGRVANQFDD
ASQAIDPWDSNNDVASQLGIFKRHDDMPVSVRYDSPEFSGFSGSVQFVPIQNSKSAYTPA
YYTKNTNNNLTLVPAVVGKPGSDVYYAGLNYKNGGFAGNYAFKYARHANVGRNAFEL
FLIGSGSDQAKGTDPLKNHQVHRLTGGYEEGGLNLALAAQLDLSENGDKTKNSTTEIAA
TASYRFGNAVPRISYAHGFDFIERGKKGENTSYDQIIAGVDYDFSKRTSAIVSGAWLKRN
TGIGNYTQINAASVGLRHKF.

13. Isoliertes Oligonucleotid, das für ein Epitop, angeordnet in den Oberflächenschleifen von Klasse I Außenmembranproteinen von Meningokokken in den Bereichen der Aminosäuren 24 bis 34 und 176 bis 187, kodiert.

14. Mittel zum Hervorrufen einer Schutzimmunantwort gegen Neisseria meningitidis, umfassend a) ein Liposom, umfassend eines oder mehrere Klasse I Außenmembranproteine oder Fragmente davon, die von Meningokokken stammen, jedoch nicht umfassend ein Klasse 2/3 Außenmembranprotein oder Fragment davon, die von Meningokokken stammen, und gegebenenfalls ein Adjuvans in einem pharmazeutisch verträglichen Träger oder b) ein Liposom, umfassend mindestens ein Oligopeptid, das ein kontinuierliches oder diskontinuierliches Epitop eines mit bakteriziden Antikörpern gegen N. meningitidis reaktiven Außenmembranproteins der Klasse 1 enthält, jedoch kein Epitop eines Außenmembranproteins der Klasse 2/3 umfaßt, und gegebenenfalls ein Adjuvans in einem pharmazeutisch verträglichen Träger, wobei das Klasse 1 Außenmembranprotein eine der zwei unten gezeigten Sequenzen hat:

DVSLYGEIKAGVEGRNFQLQLTEPPSKSQPQVKVTKAKSRIRTKISDFGSFIGFKGSEDLG
EGLKAVWQLEQDVSVAGGGATQWGNRESFVGLAGEFGTLRAGRVANQFDDASQAIDP
WDSNNDVASQLGIFKRHDDMPVSVRYDSPDFSGFSGSVQFVPIQNSKSAYTPAHYTRQN
NTDVFVPAVVGKPGSDVYYAGLNYKNGGFAGSYAFKYARHANVGRDAFELFLLGSTSD

EAKGTDPLKNHQVHRLTGGYEEGGLNLALAAQLDLSENGDKAKTKNSTTEIAATASYR
FGNAVPRISYAHGFDLIERGKKGENTSYDQIIAGVDYDFSKRTSAIVSGAWLKRNTGIGN
YTQINAASVGLRHKF; oder
DVSLYGEIKAGVEGRNYQLQLTEAQAANGGASGQVKVTKVTKAKSRIRTKISDFGSFIGF
KGSEDLGDGLKAVWQLEQDVSVAGGGATQWGNRESFIGLAGEFGTLRAGRVANQFDD
ASQAIDPWDSNNDVASQLGIFKRHDDMPVSVRYDSPEFSGFSGSVQFVPIQNSKSAYTPA
YYTKNTNNNLTLVPAVVGKPGSDVYYAGLNYKNGGFAGNYAFKYARHANVGRNAFEL ,
FLIGSGSDQAKGTDPLKNHQVHRLTGGYEEGGLNLALAAQLDLSENGDKTKNSTTEIAA
TASYRFGNAVPRISYAHGFDFIERGKKGENTSYDQIIAGVDYDFSKRTSAIVSGAWLKRN
TGIGNYTQINAASVGLRHKF.

15. Antigenes Konjugat, umfassend ein Trägerprotein oder Epitop davon, woran ein Fragment eines Klasse I Außenmembranproteins von Meningokokken mit einem Molekulargewicht von 25 kd oder weniger, enthaltend ein Epitop ausgewählt ist aus der Gruppe, bestehend aus QPQVTNGVQGN, PPSKSQP, QAANGGASG, YYTKDTNNNLTL, YYTKNTNNNLTL, YYTKDTNNNL, YYTKNTNNNL, HFVQQTPQSQP und HYTRQNNTDVF, konjugiert ist, mit der Maßgabe, daß das Trägerprotein keine β-Galactosidase ist.

16. Antigenes Konjugat nach Anspruch 15, wobei das Antigenträgerprotein ein bakterielles Toxin, CRM oder ein Epitop davon ist.

17. Genfusionspeptid oder -protein, umfassend ein Trägerprotein, Peptid oder Epitop davon, an das ein Fragment eines Klasse I Außenmembranproteins von Meningokokken mit einem Molekulargewicht von 25 kd oder weniger, enthaltend ein Epitop ausgewählt ist aus der Gruppe, bestehend aus QPQVTNGVQGN, PPSKSQP, QAANGGASG, YYTKDTNNNLTL, YYTKNTNNNLTL, YYTKDTNNNL, YYTKNTNNNL, HFVQQTPQSQP und HYTRQNNTDVF, fusioniert ist, mit der Maßgabe, daß das Trägerprotein keine β-Galactosidase ist.

18. Fusionsprotein, umfassend ein Flagellinprotein mit einer Aminosäuresequenz für ein Epitop eines Klasse I Außenmembranproteins von Meningokokken darin inseriert.

19. Fusionsprotein nach Anspruch 18, wobei die Aminosäuresequenz a) in das Flagellinprotein bei einem Bereich, der nicht wesentlich ist für die Funktion des Flagellinproteins, inseriert ist oder b) in den hypervariablen Bereich des Flagellinproteins inseriert ist.

20. Fusionsprotein nach Anspruch 18, zusätzlich umfassend ein Meningokokken-Kapseloligo- oder -polysaccharid daran konjugiert.

21. Rekombinantes Gen, für ein Fusionsprotein nach Anspruch 17 kodierend.

22. Rekombinanter Mikroorganismus, befähigt ein Fragment eines Außenmembranproteins der Klasse I von Neisseria meningitidis zu exprimieren, das ein Molekulargewicht von 25 kDa oder weniger aufweist und das ein kontinuierliches oder diskontinuierliches Epitop enthält, reaktiv mit OMP-spezifischen Antikörpern der Klasse I gegen N. meningitidis.

23. Mikroorganismus nach Anspruch 22, wobei das Epitop ausgegwählt ist aus der Gruppe, bestehend aus QPQVTNGVQGN, PPSKSQP, QAANGGASG, YYTKDTNNNLTL, YYTKNTNNNLTL, YYTKDTNNNL, YYTKNTNNNL, HFVQQTPQSQP und HYTRQNNTDVF.

24. Mikroorganismus nach Anspruch 22, der ein Pockenvirus, Adenovirus, Cytomegalovirus oder ein Bakterium der Gattung Salmonella ist.

25. Mikroorganismus nach Anspruch 22, wobei das Epitop des Klasse I Außenmembranproteins von Meningokokken als ein rekombinantes Flagellin exprimiert wird.

26. Mutanter Meningokokkenstamm, unfähig Außenmembranprotein der Klasse 2 und 3 zu erzeugen.

27. Mutanter Meningokokkenstamm HIII5, CBS 636.89, unfähig Außenmembranprotein der Klasse 2 und Klasse 3 zu erzeugen.

28. Gentechnisch hergestellter Neisseria-Stamm, negativ für Außenmembranproteine der Klasse 2/3 und befähigt, ein intaktes Außenmembranprotein der Klasse 1 von Neisseria meningitidis, ein genetisches Fusionsprotein davon oder ein kontinuierliches oder diskontinuierliches Epitop, das mit Klasse 1 OMP-spezifischen Antikörpern gegen N. meningitidis reaktiv ist, zu exprimieren.

29. Gentechnisch hergestellter Mikroorganismus, bestehend aus einem Neisseria-Stamm, befähigt zur Expression mehr als eines Außenmembranproteins der Klasse 1 von Neisseria meningitidis, wobei jedes Protein einzeln mindestens ein kontinuierliches oder diskontinuierliches Epitop, reaktiv mit OMP-spezifischen Antikörpern der Klasse I gegen N. meningitidis, umfaßt.

30. Impfstoff nach Anspruch 1, zusätzlich eine Mehrzahl von Außenmembranproteinen nach Anspruch 1 umfassend.

31. Impfstoff nach Anspruch 1, zusätzlich eine Mehrzahl von Klasse 1 Außenmembranproteinfragmenten oder Oligopeptiden, die ein Epitop davon enthalten, von Meningokokken enthaltend.

32. Verfahren zur Expression mindestens eines Klasse I Außenmembranproteins von Meningokokken, befähigt zum Auslösen einer bakteriziden Immunantwort in einem Wirt, umfassend Insertieren mindestens eines Gens, das für ein Klasse I Außenmembranprotein von Meningokokken kodiert, in einen rekombinanten Mikroorganismus, ausgewählt aus a) einem mutanten Meningokokkenstamm, der für Außenmembranprotein der Klasse 2/3 negativ ist, oder b) einen Meningococcus der Gruppe A, B, C, W-135 oder Y, wobei der Mikroorganismus befähigt ist zur Expression des Gens als intaktes Protein, das befähigt ist, eine bakterizide Immunantwort in einem Wirt auszulösen.

33. Verfahren nach Anspruch 32, wobei der Mikroorganismus ein Neisseria-Stamm ist.

34. Verfahren nach Anspruch 33, wobei der Meningokokkenstamm mehr als ein Klasse I Außenmembranprotein von Meningokokken exprimiert.

35. Gentechnisch hergestellter Neisseria-Stamm, befähigt zur Expression eines nichtnativen, intakten Außenmembranproteins der Klasse I von Neisseria meningitidis, eines genetischen Fusionsproteins davon oder eines kontinuierlichen oder diskontinuierlichen Epitops, das mit OMP-spezifischen Klasse I Antikörpern gegen N. meningitidis reaktiv ist.

## Patentansprüche (Patentansprüche für folgende(n) Vertragsstaat(en): ES)

1. Verfahren zur Expression mindestens eines Klasse I Außenmembranproteins von Meningokokken, befähigt zum Auslösen einer bakteriziden Immunantwort in einem Wirt, umfassend Insertieren mindestens eines Gens, das für ein Klasse I Außenmembranprotein von Meningokokken kodiert, in einen rekombinanten Mikroorganismus, ausgewählt aus a) einem mutanten Meningokokkenstamm, der für Außenmembranprotein der Klasse 2/3 negativ ist, oder b) einem Stamm, der ein Meningococcus der Gruppe A, B, C, W-135 oder Y ist, wobei der Mikroorganismus befähigt ist zur Expression des Gens als intaktes Protein; das befähigt ist, eine bakterizide Immunantwort in einem Wirt auszulösen.

2. Verfahren nach Anspruch 1, wobei der Mikroorganismus ein Neisseria-Stamm ist.

3. Verfahren nach Anspruch 1, wobei der mutante Meningokokkenstamm, der unfähig ist zur Herstellung von Außenmembranprotein der Klasse 2 und 3, ein mutariter Meningokokkenstamm HIII5, CBS 636.89 ist.

4. Verfahren nach Anspruch 1, wobei das inserierte Gen für ein Außenmembranprotein der Klasse I in voller Länge kodiert.

5. Verfahren nach Anspruch 1, wobei der Meningokokkenstamm mehr als ein Klasse I Außenmembranprotein von Meningokokken exprimiert.

6. Verfahren zur Herstellung eines Fragments eines Klasse I Außenmembranproteins von Meningokokken, befähigt zum Auslösen einer bakteriziden Immunantwort in einem Wirt, umfassend a) Behandeln eines Außenmembranproteins der Klasse I mit Bromcyan oder b) proteolytisches Abdauen eines Außenmembranproteins der Klasse I mit einem Enzym, ausgewählt aus der Gruppe, bestehend aus endoLys-C, endo-Arg-C, endoGlu-C und Staphylococcus-V8-Protease, oder c) Gewinnen einer DNA-Sequenz, die für das gewünschte Fragment kodiert, und Einführen derselben in ein geeignetes Vektor-/Wirts-Expressionssystem.

7. Verfahren nach Anspruch 6, wobei das erhaltene Fragment ein im wesentlichen gereinigtes Fragment eines Klasse I Außenmembranproteins von Meningokokken von Neisseria meningitidis ist, wobei das Fragment ein Molekulargewicht von etwa 25 kD oder weniger aufweist und kontinuierliche oder diskontinuierliche Epitope enthält, die mit bakteriziden Antikörpern gegen N. meningitidis reaktiv sind, wobei die Epitope in den Oberflächenschleifen von Klasse I Außenmembranproteinen von Meningokokken in dem Bereich der Aminosäuren 24 bis 34 und 176 bis 187 angeordnet sind.

8. Verfahren nach Anspruch 6, wobei das Klasse I Außenmembranprotein der Meningokokken vom Subtyp P1.7.16 ist.

9. Verfahren nach Anspruch 6, wobei das erhaltene Fragment mindestens eine der Aminosäuresequenzen, ausgewählt aus der Gruppe, bestehend aus QPQVTNGVQGN, PPSKSQP, QAANGGASG, YYTKDTNNNLTL, YYTKNTNNNLTL, YYTKDTNNNL, YYTKNTNNNL, HFVQQTPQSQP und HYTRQNNTDVF, enthält.

10. Verfahren nach Anspruch 6, wobei der rekombinante Mikroorganismus, der zur Expression des Fragmentes in der Lage ist, ein Pockenvirus, Adenovirus, Cytomegalovirus oder ein Bakterium der Gattung Salmonella ist.

11. Verfahren nach Anspruch 6, wobei das erhaltene Fragment zusätzlich ein B-Zellenepitop eines Außenmembranproteins der Klasse I enthält, das in der Aminosäuresequenz unter verschiedenen Meningokokken Porinproteinen variiert.

12. Verfahren nach Anspruch 6, wobei das erhaltene Fragment zusätzlich ein B-Zellen- oder T-Zellenepitop eines Außenmembranproteins der Klasse I enthält, das in der Aminosäuresequenz unter verschiedenen Meningokokken-Porinproteinen konserviert ist.

13. Verfahren nach Anspruch 6, wobei das erhaltene Fragment zusätzlich ein T-Zellenepitop eines Klasse I Außenmembranproteins von Meningokokken enthält.

14. Verfahren zur Herstellung eines Oligopeptids, enthaltend ein Epitop eines Klasse I Außenmembranproteins von Meningokokken, das befähigt ist zum Auslösen einer bakteriziden Immunantwort in einem Wirt, umfassend
a) proteolytisches Abdauen
i) eines Membranproteins der Klasse I oder
ii) eines Fragments, abgeleitet von der Aufspaltung eines Außenmembranproteins der Klasse I mit Bromcyan,
mit einem Enzym, ausgewählt aus der Gruppe, bestehend aus endoLys-C, endo-Arg-C; endoGlu-C und Staphylococcus V8 Protease; oder alternativ,
b) seine chemische Synthese durch übliche Festphasen-Peptidsynthese, Pepscansynthese oder Standard-Flüssigpeptidsynthese; oder alternativ
c) Gewinnen einer DNA-Sequenz die die gewünschte Oligopeptidsequenz kodiert, und Einführen derselben in ein geeignetes Vektor-/Wirtsexpressionssystem.

15. Verfahren nach Anspruch 14, wobei das Oligopeptid a) mindestens ein Epitop, das einen bakteriziden Antikörper bindet, von Klasse I Außenmembranproteinen von Meningokokken oder b) mindestens eine Aminosäuresequenz umfaßt, ausgewählt aus der Gruppe, bestehend aus QPQVTNGVQGN, PPSKSQP, QAANGGASG, YYTKDTNNNLTL, YYTKNTNNNLTL, YYTKDTNNNL, YYTKNTNNNL, HFVQQTPQSQP und HYTRQNNTDVF.

16. Verfahren nach Anspruch 14, wobei das Epitop in den Oberflächenschleifen von Klasse I Außenmembranproteinen von Meningokokken in den Bereichen der Aminosäuren 24 bis 34 und 176 bis 187 angeordnet ist.

17. Verfahren nach einem der Ansprüche 1, 6 oder 14, wobei das Außenmembranprotein der Klasse I, das Fragment davon oder das erhaltene Oligopeptid, außerdem gemischt, konjugiert oder fusioniert ist an Trägerpeptide oder Proteine, an andere Antigene von N. meningitidis oder Antigene von anderen Mikroorganismen durch chemische oder genetische Kupplungstechniken.

18. Verfahren nach Anspruch 17, wobei das Außenmembranprotein der Klasse I, das Fragment davon oder das erhaltene Oligopeptid an ein Trägerprotein oder Epitop davon konjugiert ist, mit der Maßgabe, daß das Trägerprotein nicht β-Galactosidase ist.

19. Verfahren nach Anspruch 18, wobei das Trägerprotein ein bakterielles Toxin, CRM oder ein Epitop davon ist.

20. Verfahren nach Anspruch 17, wobei das Außenmembranprotein der Klasse I, das Fragment davon oder das erhaltene Oligopeptid a) über chemische Kupplung an ein T-Zellenepitop konjugiert ist oder b) ein Polysaccharid von Meningokokken A, B, C, W-135 und Y enthält, in Konjugatform mit dem Proteinprodukt.

21. Verfahren nach Anspruch 17, wobei das Außenmembranprotein der Klasse I, das Fragment davon oder das erhaltene Oligopeptid an ein Trägerprotein, Peptid oder Epitop davon fusioniert ist, mit der Maßgabe, daß das Trägerprotein nicht β-Galactosidase ist.

22. Verfahren zur Herstellung eines Fusionsproteins, umfassend ein Flagellinprotein mit einer Aminosäuresequenz für ein Epitop eines Klasse I Außenmembranproteins von Meningokokken darin inseriert, umfassend Expression eines rekombinanten Gens, das eine geeignet inserierte DNA-Sequenz enthält, die für ein Epitop eines Klasse I Außenmembranproteins von Meningokokken innerhalb des Gens, das für Flagellin kodiert.

23. Verfahren nach Anspruch 22, wobei das erhaltene Fusionsprotein die Aminosäuresequenz für ein Epitop eines Klasse I Außenmembranproteins von Meningokokken innerhalb des Flagellinproteins enthält (a) bei einem Bereich, der nicht wesentlich ist für die Funktion des Flagellinproteins, oder (b) in einem hypervariablen Bereich des Flagellinproteins.

24. Verfahren nach Anspruch 22, wobei das erhaltene Fusionsprotein zusätzlich ein Kapseloligo- oder -polysaccharid von Meningokokken, daran konjugiert, enthält.

25. Verfahren zur Herstellung eines Impfstoffes gegen Meningokokken-Erkrankungen, umfassend Vermischen einer wirksamen Menge einer Außenmembranvesikel, isoliert aus gentechnisch hergestelltem oder mutantem Meningokokkenstamm, wobei die Vesikel umfaßt: mindestens ein Klasse I Außenmembranprotein von Meningokokken, ein Fragment davon oder ein Oligopeptid, das ein Epitop davon enthält, wobei das Protein, Fragment davon oder Oligonucleotid in der Lage ist, eine bakterizide Immunantwort in einem Wirt auszulösen, und kein Klasse 2/3 Außenmembranprotein von Meningokokken umfaßt, mit einem geeigneten Adjuvans; wobei das Klasse I Außenmembranprotein von Meningokokken von einem mutanten Meningokokkenstamm stammt, der für Außenmembranprotein der Klasse 2/3 negativ ist.

26. Verfahren nach Anspruch 25, wobei der Impfstoff eine Vielzahl von Außenmembranproteinen der Klasse I umfaßt.

27. Verfahren nach Anspruch 25, wobei der erhaltene Impfstoff eine Vielzahl von Klasse I Außenmembranproteinen, Fragmenten oder Oligopeptiden, die ein Epitop davon enthalten, von Meningokokken umfaßt.

## Revendications (Revendications pour l'(les) Etat(s) contractant(s) suivant(s): AT, BE, CH, LI, DE, FR, GB, IT, LU, NL, SE)

1. Vaccin efficace contre la méningite à méningocoque, comprenant une quantité efficace d'une vésicule de la membrane externe isolée à partir de souches de méningocoques obtenues par mutation ou par génie génétique, laquelle vésicule comprend au moins une protéine de classe 1 de la membrane externe de méningocoque, un fragment de celle-ci ou un oligopeptide contenant un épitope de cette protéine, dans lequel ladite protéine, le fragment de celle-ci ou l'oligopeptide est capable d'induire une réponse immunitaire bactéricide chez un hôte, et ne comprend pas de protéine de classe 2/3 de la membrane externe de méningocoque, la protéine de classe 1 de la membrane externe de méningocoque provenant d'une souche de méningocoque mutant n'exprimant pas la protéine de classe 2/3 de la membrane externe.

2. Vaccin selon la revendication 1, dans lequel la protéine de classe 1 de la membrane externe de méningocoque est obtenue à partir d'un méningocoque du groupe A, B, C, W-135 ou Y.

3. Vaccin selon la revendication 1, dans lequel le fragment de protéine de classe 1 de la membrane externe de méningocoque est obtenu a) par un traitement au bromure de cyanogène de la protéine de classe 1 de la membrane externe de méningocoque, ou b) par protéolyse par une enzyme choisie dans le groupe formé par endoLys-C, endo-Arg-C, endoGlu-C et la protéase V8 de *Staphylococcus*.

4. Vaccin selon la revendication 1, dans lequel l'oligopeptide comprend a) au moins un épitope de protéine de classe 1 de la membrane externe de méningocoque qui se lie à un anticorps bactéricide, ou b) au moins une séquence d'acides aminés choisie dans le groupe formé par les séquences QPQVTNGVQGN, PPSKSQP, QAANGGASG, YYTKDTNNNLTL, YYTKNTNNNLTL, YYTKDTNNNL, YYTKNTNNNL, HFVQQTPQSQP et HYTRQNNTDVF.

5. Vaccin selon la revendication 1, dans lequel l'épitope est situé dans les boucles superficielles de protéines de classe 1 de la membrane externe de méningocoque dans la région des acides aminés 24-34 et 176-187.

6. Fragment pratiquement pure de la protéine de classe 1 de la membrane externe de *Neisseria meningitidis*, le fragment ayant une masse moléculaire d'environ 25 kD ou moins et contenant des épitopes continus ou discontinus capables de réagir avec des anticorps bactéricides anti-*N.meningitidis*, les épitopes étant situés dans des boucles superficielles de protéines de classe 1 de la membrane externe de méningocoque dans la région des acides aminés 24 - 34 et 176 - 187.

7. Fragment selon la revendication 6, dans lequel la protéine de classe 1 de la membrane externe de méningocoque est du sous-type P1.7.16.

8. Oligopeptide pratiquement pur selon la revendication 6, contenant un épitope, reconnu par les lymphocytes B, d'une protéine de classe 1 de membrane externe dont la séquence d'acides aminés varie parmi différentes porines de méningocoques.

9. Oligopeptide selon la revendication 8, contenant au moins une séquence d'acides aminés choisie dans le groupe formé par QPQVTNGVQVN, PPSKSQP, QAANGGASG, YYTKDTNNNLTL, YYTKNTNNNLTL, YYTKDTNNNL, YYTKNTNNNL, HFVQQTPQSQP et HYTRQNNTDVF.

10. Oligopeptide pratiquement pur selon la revendication 6, contenant un épitope, reconnu par les lymphocytes B ou T, d'une protéine de classe 1 de la membrane externe dont la séquence d'acides aminés est conservée parmi différentes porines de méningocoques.

11. Oligopeptide pratiquement pur selon la revendication 6, contenant un épitope, reconnu par les lymphocytes T, d'une protéine de classe 1 de la membrane externe de méningocoque.

12. Acide nucléique isolé codant la protéine complète de classe 1 de la membrane externe, ayant la séquence ci-dessous :

DVSLYGEIKAGVEGRNFQLQLTEPPSKSQPQVKVTKAKSRIRTKISD
FGSFIGFKGSEDLGEGLKAVWQLEQDVSVAGGGATQWGNRESFVG
LAGEFGTLRAGRVANQFDDASQAIDPWDSNNDVASQLGIFKRHDD
MPVSVRYDSPDFSGFSGSVQFVPIQNSKSAYTPAHYTRQNNTDVFV
PAVVGKPGSDVYYAGLNYKNGGFAGSYAFKYARHANVGRDAFEL
FLLGSTSDEAKGTDPLKNHQVHRLTGGYEEGGLNLALAAQLDLSE
NGDKAKTKNSTTEIAATASYRFGNAVPRISYAHGFDLIERGKKGEN

TSYDQIIAGVDYDFSKRTSAIVSGAWLKRNTGIGNYTQINAASVGL
RHKF, ou
DVSLYGEIKAGVEGRNYQLQLTEAQAANGGASGQVKVTKVTKAKS
RIRTKISDFGSFIGFKGSEDLGDGLKAVWQLEQDVSVAGGGATQWG
NRESFIGLAGEFGTLRAGRVANQFDDASQAIDPWDSNNDVASQLGI
FKRHDDMPVSVRYDSPEFSGFSGSVQFVPIQNSKSAYTPAYYTKNT
NNNLTLVPAVVGKPGSDVYYAGLNYKNGGFAGNYAFKYARHANV
GRNAFELFLIGSGSDQAKGTDPLKNHQVHRLTGGYEEGGLNLALA
AQLDLSENGDKTKNSTTEIAATASYRFGNAVPRISYAHGFDFIERGK
KGENTSYDQIIAGVDYDFSKRTSAIVSGAWLKRNTGIGNYTQINAA
SVGLRHKF.

13. Oligonucléotide isolé codant un épitope situé dans les boucles superficielles de protéines de classe 1 de la membrane externe de méningocoque dans la région des acides aminés 24 - 34 et 176 - 187.

14. Composition induisant une réponse immunitaire protectrice contre *Neisseria meningitidis*, comprenant a) un liposome comprenant une ou plusieurs protéines de classe 1 de la membrane externe de méningocoque, mais ne comprenant pas de protéine de classe 2/3 de la membrane externe de méningocoque ou de fragment d'une telle protéine, et éventuellement un adjuvant dans un véhicule pharmaceutiquement acceptable, ou b) un liposome comprenant au moins un oligopeptide contenant un épitope continu ou discontinu d'une protéine de classe 1 de la membrane externe capable de réagir avec des anticorps bactéricides anti-*N.meningitidis*, mais ne comprenant pas d'épitope d'une protéine de classe 2/3 de la membrane externe, et éventuellemnet un adjuvant dans un véhicule pharmaceutiquement acceptable, ladite ou lesdites protéines de classe 1 de la membrane externe ayant une des deux séquences ci-dessous :

DVSLYGEIKAGVEGRNFQLQLTEPPSKSQPQVKVTKAKSRIRTKISD
FGSFIGFKGSEDLGEGLKAVWQLEQDVSVAGGGATQWGNRESFVG
LAGEFGTLRAGRVANQFDDASQAIDPWDSNNDVASQLGIFKRHDD
MPVSVRYDSPDFSGFSGSVQFVPIQNSKSAYTPAHYTRQNNTDVFV
PAVVGKPGSDVYYAGLNYKNGGFAGSYAFKYARHANVGRDAFEL
FLLGSTSDEAKGTDPLKNHQVHRLTGGYEEGGLNLALAAQLDLSE
NGDKAKTKNSTTEIAATASYRFGNAVPRISYAHGFDLIERGKKGEN
TSYDQIIAGVDYDFSKRTSAIVSGAWLKRNTGIGNYTQINAASVGL

RHKF, ou
DVSLYGEIKAGVEGRNYQLQLTEAQAANGGASGQVKVTKVTKAKS
RIRTKISDFGSFIGFKGSEDLGDGLKAVWQLEQDVSVAGGGATQWG
NRESFIGLAGEFGTLRAGRVANQFDDASQAIDPWDSNNDVASQLGI
FKRHDDMPVSVRYDSPEFSGFSGSVQFVPIQNSKSAYTPAYYTKNT
NNNLTLVPAVVGKPGSDVYYAGLNYKNGGFAGNYAFKYARHANV
GRNAFELFLIGSGSDQAKGTDPLKNHQVHRLTGGYEEGGLNLALA
AQLDLSENGDKTKNSTTEIAATASYRFGNAVPRISYAHGFDFIERGK
KGENTSYDQIIAGVDYDFSKRTSAIVSGAWLKRNTGIGNYTQINAA
SVGLRHKF.

15. Conjugué antigénique, comprenant une protéine porteuse ou un épitope de celle-ci, à laquelle est lié un fragment d'une protéine de classe 1 de la membrane externe de méningocoque ayant une masse moléculaire de 25 kD ou moins contenant un épitope choisi dans le groupe formé par QPQVTNGVQVN, PPSKSQP, QAANGGASG, YYTKDTNNNLTL, YYTKNTNNNLTL, YYTKDTNNNL, YYTKNTNNNL, HFVQQTPQSQP et HYTRQNNTDVF, la protéine porteuse n'étant pas la β-galactosidase.

16. Conjugué antigénique selon la revendication 15, dans lequel la protéine portant l'antigène est une toxine bactérienne, une CRM ou un épitope de celles-ci.

17. Protéine ou peptide de fusion génétique, comprenant une protéine porteuse ou un peptide porteur ou un épitope de ceux-ci, auquel est fusionné un fragment d'une protéine de classe 1 de la membrane externe de méningocoque ayant une masse moléculaire de 25 kD ou moins, contenant un épitope choisi dans le groupe formé par QPQVTNGVQVN, PPSKSQP, QAANGGASG, YYTKDTNNNLTL, YYTKNTNNNLTL, YYTKDTNNNL, YYTKNTNNNL, HFVQQTPQSQP et HYTRQNNTDVF, avec la réserve que la protéine porteuse ne soit pas la β-galactosidase.

18. Protéine de fusion comprenant une flagelline dans laquelle est insérée une séquence d'acides aminés pour un épitope d'une protéine de classe 1 de la membrane externe de méningocoque.

19. Protéine de fusion selon la revendication 18, dans laquelle la séquencé d'acides aminés est insérée a) à l'intérieur de la flagelline dans une région non essentielle à la fonction de la flagelline, ou b) dans la région hypervariable de la flagelline.

20. Protéine de fusion selon la revendication 18, comprenant en outre un oligo- ou polysaccharide de la capsule d'un méningocoque, lié à la protéine.

21. Gène recombiné codant une protéine de fusion selon la revendication 17.

22. Microorganisme génétiquement modifié capable d'exprimer un fragment de la protéine de classe 1 de la membrane externe de *Neisseria meningitidis* ayant une masse moléculaire de 25 kD ou moins contenant un épitope continu ou discontinu capable de réagir avec des anticorps spécifiques des protéines de classe 1 de la membrane externe, dirigés contre *N. meningitidis*.

23. Microorganisme selon la revendication 22 dans lequel l'épitope est choisi dans le groupe formé par QPQVTNGVQVN, PPSKSQP, QAANGGASG, YYTKDTNNNLTL, YYTKNTNNNLTL, YYTKDTNNNL, YYTKNTNNNL, HFVQQTPQSQP et HYTRQNNTDVF.

24. Microorganisme selon la revendication 22, qui est un virus de la vaccine, un adénovirus, un cytomégalovirus ou une bactérie du genre *Salmonella*.

25. Microorganisme selon la revendication 22, dans lequel l'épitope de la protéine de classe 1 de la membrane externe de méningocoque est exprimée en tant que flagelline recombinée.

26. Souche mutante de méningocoque incapable de produire les protéines de classe 2 et 3 de la membrane externe.

27. Souche mutante de méningocoque HIII5, CBS 636.89 incapable de produire les protéines de classe 2 et 3 de la membrane externe.

28. Souche génétiquement modifiée de *Neisseria* qui ne produit pas les protéines de classe 2/3 de la membrane externe mais qui est capable d'exprimer une protéine de classe 1 de la membrane externe de *Neisseria meningitidis* intacte, une protéine correspondante obtenue par fusion génétique ou un épitope continu ou discontinu capable de réagir avec des anticorps spécifiques des protéines de classe 1 de la membrane externe, dirigés contre *Neisseria meningitidis*.

29. Microorganisme génétiquement modifié qui est une souche de *Neisseria* capable d'exprimer plusieurs protéines de classe 1 de la membrane externe de *Neisseria meningitidis*, chaque protéine contenant au moins un épitope continu ou discontinu capable de réagir avec des anticorps spécifiques des protéines de classe 1 de la membrane externe, dirigés contre *Neisseria meningitidis*.

30. Vaccin selon la revendication 1, qui contient en outre plusieurs protéines de classe 1 de la membrane externe.

31. Vaccin selon la revendication 1, comprenant en outre plusieurs fragments de protéines de classe 1 de la membrane externe de méningocoque ou des oligopeptides contenant un épitope de ceux-ci.

32. Procédé d'expression d'au moins une protéine de classe 1 de la membrane externe de méningocoque capable d'induire une réponse immunitaire bactéricide chez un hôte, comprenant l'insertion d'au moins un gène codant une protéine de classe 1 de la membrane externe de méningocoque dans un microorganisme recombiné choisi parmi a) une souche mutante de méningocoque qui ne produit pas les protéines de classe 2 et 3 de la membrane externe ou b) un méningocoque du groupe A, B, C, W-135 ou Y, dans lequel le microorganisme est capable d'exprimer ledit gène sous forme de protéine intacte capable d'induire une réponse immunitaire bactéricide chez un hôte.

33. Procédé selon la revendication 32, dans lequel le microorganisme est une souche de *Neisseria*.

34. Procédé selon la revendication 33, dans lequel la souche de méningocoque exprime plus d'une protéine de classe 1 de la membrane externe de méningocoque.

35. Souche génétiquement modifiée de *Neisseria* capable d'exprimer une protéine intacte non-native de classe 1 de la membrane externe de *Neisseria meningitidis*, une protéine correspondante obtenue par fusion génétique ou un épitope continu ou discontinu capable de réagir avec des anticorps spécifiques des protéines de classe 1 de la membrane externe, dirigés contre *Neisseria meningitidis*.

## Revendications (Revendications pour l'(les) Etat(s) contractant(s) suivant(s): ES)

1. Procédé d'expression d'au moins une protéine de classe 1 de la membrane externe de méningocoque capable d'induire une réponse immunitaire bactéricide chez un hôte, comprenant l'insertion d'au moins un gène codant une protéine de classe 1 de la membrane externe de méningocoque dans un microorganisme recombiné choisi parmi a) une souche mutante de méningocoque qui ne produit pas de protéines de classe 2 et 3 de la membrane externe ou b) une telle souche qui est un méningocoque de groupe A, B, C, W-135 ou Y, dans lequel le microorganisme est capable d'exprimer ledit gène sous forme de protéine intacte capable d'induire une réponse immunitaire bactéricide chez un hôte.

2. Procédé selon la revendication 1, dans lequel le microorganisme est une souche de *Neisseria*.

3. Procédé selon la revendication 1, dans lequel la souche mutante de méningocoque qui ne produit pas de protéines de classe 2 et 3 de la membrane externe est la souche de méningocoque mutante HIII5, CBS 636.89.

4. Procédé selon la revendication 1, dans lequel le gène inséré code la protéine complète de classe 1 de la membrane externe.

5. Procédé selon la revendication 1, dans lequel la souche de méningocoque exprime plus d'une protéine de classe 1 de la membrane externe.

6. Procédé de préparation d'un fragment d'une protéine de classe 1 de la membrane externe capable d'induire une réponse immunitaire bactéricide chez un hôte, comprenant
a) le traitement d'une protéine de classe 1 de la membrane externe avec du bromure de cyanogène, ou
b) la digestion protéolytique de la protéine de classe 1 de la membrane externe avec une enzyme choisie dans le groupe formé par endoLys-C, endo-Arg-C, endoGlu-C et la protéase V8 de *Staphylococcus*, ou
c) l'obtention d'une séquence d'ADN codant le fragment désiré et l'introduction de cette séquence dans un système d'expression vecteur/hôte approprié.

7. Procédé selon la revendication 6, dans lequel le fragment obtenu est un fragment partiellement pur d'une protéine de classe 1 de la membrane externe de *Neisseria meningitidis*, ledit fragment ayant une masse moléculaire d'environ 25 kD ou moins et contenant des épitopes continus ou discontinus capables de réagir avec les anticorps bactéricides dirigés contre *Neisseria meningitidis*, dans lequel les épitopes sont situés dans les boucles superficielles de la protéine de classe 1 de la membrane externe de méningocoque dans la région des acides aminés 24 - 34 et 176 - 187.

8. Procédé selon la revendication 6, dans lequel la protéine de classe 1 de la membrane externe de méningocoque est une protéine du sous-type P1.7.16.

9. Procédé selon la revendication 6, dans lequel le fragment obtenu contient au moins une séquence d'acides aminés choisie dans le groupe formé par les séquences QPQVTNGVQGN, PPSKSQP, QAANGGASG, YYTKDTNNNLTL, YYTKNTNNNLTL, YYTKDTNNNL, YYTKNTNNNL, HFVQQTPQSQP et HYTRQNNTDVF.

10. Procédé selon la revendication 6, dans lequel le microorganisme recombiné capable d'exprimer ledit fragment est le virus de la vaccine, un adériovirus, un cytomégalovirus ou une bactéries du genre *Salmonella*.

11. Procédé selon la revendication 6, dans lequel le fragment obtenu contient en outre un épitope, reconnu par les lymphocytes B, d'une protéine de classe 1 de la membrane externe dont la séquence d'acides aminés varie parmi différentes porines de méningocoques.

12. Procédé selon la revendication 6, dans lequel le fragment obtenu contient en outre un épitope, reconnu par les lymphocytes B ou par les lymphocytes T, d'une protéine de classe 1 de la membrane externe dont la séquence d'acides amines est conservée parmi différentes porines de méningocoques.

13. Procédé selon la revendication 6, dans lequel le fragment obtenu contient en outre un épitope, reconnu par les lymphocytes T, d'une protéine de classe 1 de la membrane externe de méningocoque.

14. Procédé de préparation d'un oligopeptide contenant un épitope d'une protéine de classe 1 de la membrane externe de méningocoque capable d'induire une réponse immunitaire bactéricide chez un hôte, comprenant
a) la digestion protéolytique
(i) d'une protéine de classe 1 de la membrane externe, ou
(ii) d'un fragment issu du clivage d'une protéine de classe 1 de la membrane externe par du bromure de cyanogène,
avec une enzyme choisie dans le groupe formé par endoLys-C, endo-Arg-C, endoGlu-C et la protéase V8 de *Staphylococcus*, ou bien
b) sa synthèse chimique par synthèse peptidique sur support solide, par synthèse sur pepscan ou par synthèse peptidique en phase liquide, ou
c) l'obtention d'une séquence d'ADN codant l'oligopeptide désiré et l'introduction de cette séquence dans un système d'expression vecteur/hôte approprié.

15. Procédé selon la revendication 14, dans lequel l'oligopeptide comprend a) au moins un épitope de la protéine de classe 1 de la membrane externe de méningocoque qui se lie à un anticorps bactéricide, ou b) au moins une séquence d'acides aminés choisie dans le groupe formé par les séquences QPQVTNGVQGN, PPSKSQP, QAANGGASG, YYTKDTNNNLTL, YYTKNTNNNLTL, YYTKDTNNNL, YYTKNTNNNL, HFVQQTPQSQP et HYTRQNNTDVF.

16. Procédé selon la revendication 14, dans lequel l'épitope est situé dans les boucles superficielles d'une protéine de classe 1 de la membrane externe de méningocoque dans la région des acides aminés 24 - 34 et 176 - 187.

17. Procédé selon l'une quelconque des revendications 1, 6 ou 14, dans lequel la protéine de classe 1 de la membrane externe, le fragment de celle-ci ou l'oligopeptide obtenu est en outre mélangé, conjugué ou fusionné avec des peptides porteurs ou protéines porteuses, à d'autres antigènes de *N. meningitidis* ou à des antigènes d'autres micro-organismes par des techniques de couplage chimique ou génétique.

18. Procédé selon la revendication 17, dans lequel la protéine de classe 1 de la membrane externe, le fragment de celle-ci ou l'oligopeptide obtenu est conjugué à une protéine porteuse ou un épitope de celle-ci, avec la réserve que la protéine porteuse ne soit pas la β-galactosidase.

19. Procédé selon la revendication 18, dans lequel la protéine porteuse est une toxine bactérienne, une CRM ou un épitope de celles-ci.

20. Procédé selon la revendication 17, dans lequel la protéine de classe 1 de la membrane externe, le fragment ou l'oligopeptide a) sont conjugués à un épitope, reconnu par les lymphocytes T, par couplage chimique ou b) contient un polysaccharide de méningocoque A, B, C, W-135 ou Y, lié au produit protéique.

21. Procédé selon la revendication 17, dans lequel la protéine de classe 1 de la membrane externe, le fragment de celle-ci ou l'oligopeptide obtenu sont fusionnés avec une protéine porteuse, un peptide porteur ou un épitope de ceux-ci, avec la réserve que la protéine porteuse ne soit pas la β-galactosidase.

22. Procédé de préparation d'une protéine de fusion comprenant une flagelline dans laquelle est insérée une séquence d'acides aminés d'un épitope d'une protéine de classe 1 de la membrane externe de méningocoque, comprenant un gène recombiné contenant une séquence d'ADN codant un épitope d'une protéine de classe 1 de la membrane externe de méningocoque, insérée de manière appropriée dans le gène codant la flagelline.

23. Procédé selon la revendication 22, dans lequel la protéine de fusion obtenue contient la séquence d'acides aminés d'un épitope d'une protéine de classe 1 de la membrane externe de méningocoque au sein de la flagellin (a) dans une région qui n'est pas essentielle pour la fonction de la flagelline, ou (b) dans une région hypervariable de la flagelline.

24. Procédé selon la revendication 22, dans lequel la protéine de fusion obtenue contient en outre un oligo- ou un polysaccharide capsulaire de méningocoque, lié à celle-ci.

25. Procédé de préparation d'un vaccin efficace contre la méningite à méningocoque, comprenant le mélange d'une quantité efficace d'une vésicule de membrane externe isolée à partir de souches de méningocoques obtenues par mutation ou par génie génétique, laquelle vésicule comprend au moins une protéine de classe 1 de la membrane externe de méningocoque, un fragment de celle-ci ou un oligopeptide contenant un épitope de cette protéine, dans lequel ladite protéine, le fragment de celle-ci ou l'oligopeptide est capable d'induire une réponse immunitaire bactéricide chez un hôte, et ne comprendt pas de protéine de classe 2/3 de la membrane externe de méningocoque, avec un adjuvant approprié, la protéine de classe 1 de la membrane externe de méningocoque étant issue d'une souche mutante de méningocoque n'exprimant pas de protéines de classe 2/3 de la membrane externe.

26. Procédé selon la revendication 25, dans lequel le vaccin obtenu contient plusieurs protéines de classe 1 de la membrane externe.

27. Procédé selon la revendication 25, dans lequel le vaccin obtenu contient plusieurs fragments de protéines de classe 1 de la membrane externe de méningocoque ou plusieurs oligopeptides contenant un épitope de ces protéines.
